(19) Europäisches Patentamt / European Patent Office / Office européen des brevets

(11) **EP 3 434 784 A1**

(12) **EUROPEAN PATENT APPLICATION**

(43) Date of publication:
**30.01.2019 Bulletin 2019/05**

(51) Int Cl.:
**C12Q 1/68** *(2018.01)*      **C12Q 1/6876** *(2018.01)*
**C12P 19/34** *(2006.01)*      **C12Q 1/6816** *(2018.01)*

(21) Application number: **18176103.2**

(22) Date of filing: **12.06.2014**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.06.2013   US 201361834926 P**

(62) Document number(s) of the earlier application(s) in
accordance with Art. 76 EPC:
**14739298.9 / 3 008 209**

(71) Applicant: **Nanostring Technologies, Inc
Seattle, WA 98109 (US)**

(72) Inventor: **WEBSTER, Philippa, J.
Seattle, WA 98103 (US)**

(74) Representative: **Cooley (UK) LLP
Dashwood
69 Old Broad Street
London EC2M 1QS (GB)**

Remarks:
•This application was filed on 05.06.2018 as a
divisional application to the application mentioned
under INID code 62.
•Claims filed after the date of receipt of the divisional
application (Rule 68(4) EPC).

(54) **MULTIPLEXABLE TAG-BASED REPORTER SYSTEM**

(57) The present invention relates to compositions and methods for the detection and quantification of individual target molecules in biomolecular samples. In particular, the invention relates to coded, labeled compositions comprising at least two probes hybridized to each other that are capable of binding to and identifying target molecules based on the probes' label codes. Methods of making and using such compositions are also provided. The compositions can be used in diagnostic, prognostic, quality control and screening applications.

## Figure 3

Reporter Probe          Labeled Capture Probe

tag          tag

Oligo A          Oligo B

Nucleic acid target

**Description**

CROSS-REFERENCE TO RELATED APPLICATIONS

This application claims priority to, and the benefit of, U.S. Provisional Application No. 61/834,926, filed June 14, 2013, the contents of which are incorporated herein in its entirety.

FIELD OF THE INVENTION

This disclosure relates generally to compositions and methods for detection and quantification of individual target molecules in biomolecular samples. In particular, the invention relates to a multiplexable tag-based reporter system for labeling a plurality of target molecules with a unique reporter code utilizing compositions comprising a capture probe and a labeled reporter probe, wherein the capture probe and/or reporter probe are associated indirectly to a specific target molecule through hybridization to intermediate oligonucleotide molecules.

BACKGROUND OF THE INVENTION

Although all cells in the human body contain the same genetic material, the same genes are not active in all of those cells. Alterations in gene expression patterns can have profound effects on biological functions. These variations in gene expression are at the core of altered physiologic and pathologic processes. Therefore, identifying and quantifying the expression of genes in normal cells compared to diseased cells can aid the discovery of new drug and diagnostic targets.

Nucleic acids can be detected and quantified based on their specific polynucleotide sequences. The basic principle underlying existing methods of detection and quantification is the hybridization of a labeled complementary probe sequence to a target sequence of interest in a sample. The formation of a duplex indicates the presence of the target sequence in the sample. The recent development of DNA microarrays has enabled the detection of the presence or absence of thousands of genes in a biological sample in a single experiment.

Despite significant advances, many drawbacks still exist in molecular hybridization and microarray techniques. Microarray methods still require significant amounts of biological sample, which can be a critical limitation for drug and diagnostic assays that rely upon biological samples with limited supply, such as biopsies of diseased tissues or samples of a discrete cell type. In addition, the kinetics of hybridization on the surface of a microarray is less efficient than hybridization in small amounts of aqueous solution. Moreover, while methods exist to estimate the amount of nucleic acid present in a sample based on microarray hybridization result, microarray technology thus far does not allow for detection of target molecules on an individual level, nor are there microarray-based methods for directly quantifying the amount of target molecule in a given sample.

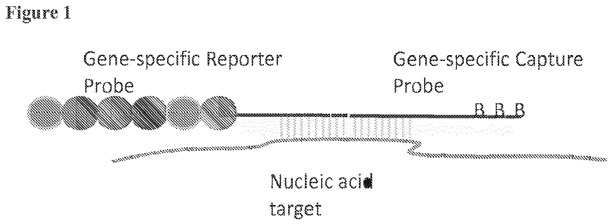

An existing nanoreporter system (US 2010/0015607 and US 2010/0047924), herein referred to as the "standard nanoreporter system" provides a sensitive, multiplexed method for detecting target nucleic acid molecules in biological samples. The assay is based on the direct molecular barcoding and digital detection of target molecules through the use of a color-coded probe pair. The probe pair consists of 1) a reporter probe which carries an ordered series of fluorescent signals which can be read as a colored barcode, as well as an affinity moiety in the form of DNA sequence repeats; and 2) a capture probe which carries affinity moieties both in the form of a second set of DNA sequence repeats and in the form of biotin molecules. Each target molecule is assigned a unique color code by attaching a target-specific probe to a designated reporter. A large diversity of color-coded reporter probes can be mixed together for direct, multiplexed hybridization to a mixture of target molecules, and then the codes can be individually resolved and identified during data collection. The structure of the molecular complex formed by the target, the reporter probe and the capture probe is shown in Figure 1.

In brief, the standard nanoreporter assay is carried out as follows: reporter and capture probe pairs are introduced in large excess into target mixtures. Following hybridization, excess probes are washed away in a two-step bead-based purification. First, the hybridization mixture is bound to beads derivatized with short nucleic acid sequences that are complementary to the affinity repeats on the capture probe, and non-hybridized reporter probes and non-target molecules are washed away. The remaining molecules are then bound to beads derivatized with short nucleic acid sequences that are complementary to the affinity repeats on the reporter probe, and non-hybridized capture probes are washed away. The final complexes are then immobilized on the surface of a cartridge for data collection in the form of imaging and counting of each barcode.

The data from the standard nanoreporter system is precise and uses very small quantities of biological target material. However, due to the complex and highly customized nature of the nanoreporter reagents, the standard nanoreporter assay is relatively inflexible and expensive. Each batch of reporter and capture probes can only be used to assay a pre-selected set of targets, and new reagents must be created for each additional target. The reagents are labor-intensive and time-consuming to manufacture. Furthermore, the complexity in the manufacturing processes adds to variability in the reagents and increases the cost of their manufacture and quality control (QC).

Thus, there exists a need for accurate and sensitive detection, identification and quantification of target molecules in complex mixtures. In addition, there exists a need for detection reagents that can be produced with high efficiency and consistency, and allow flexibility in experimental design and target selection. It is important for the productivity and effectiveness of scientific research to be able to rapidly and inexpensively accommodate changes to the selected list of target molecules from one experiment to the next.

SUMMARY OF THE INVENTION

The present invention relates to a tag-based nanoreporter system for the detection, identification, and direct quantification of a wide variety of target molecules that utilizes compositions comprising four probes, wherein two of the probes comprise a first region that binds or hybridizes directly to the target molecule and a second non-overlapping region that binds or hybridizes to the other two probes, which comprise a label attachment region and/or an affinity moiety for detection. The present invention is advantageous in that it allows increased efficiency and consistency in the manufacture of the nanoreporters, increased flexibility in the content of the assay, decreased errors in code-target association (i.e., decreased false positives) and lower background signal. The present invention also provides methods for generation and use of such a tag-based nanoreporter system.

The present invention provides a composition including a first probe and a second probe, the first probe includes a first region that has a first target-specific sequence; and a second region that does not overlap with the first region and does not bind to the target molecule; the second probe includes a first region that binds to the second region of said first probe; a first label attachment region which is hybridized to a first RNA molecule, wherein the first RNA molecule is attached to one or more label monomers that emit light constituting a first signal; and a second label attachment region, which is non-overlapping to the first label attachment region, and which is hybridized to a second RNA molecule, wherein the second RNA molecule is attached to one or more label monomers that emit light constituting a second signal, and wherein the label attachment regions do not overlap with the first region of the second probe. In one embodiment, the second region of the first probe or the first region of the second probe is any one of SEQ ID NOs 1-1345 or a complement thereof.

The present invention provides a composition including a first probe and a second probe, the first probe includes a first region comprising a first target-specific sequence; and a second region that does not overlap with the first region and does not bind to the target molecule; the second probe includes a first region that binds to the second region of said first probe; and a second region that does not overlap with the first region and comprises at least one affinity moiety. In one embodiment, the second probe also includes at least a first label attachment region which is hybridized to a first RNA molecule, wherein the first RNA molecule is attached to one or more label monomers that emit light constituting a first signal. In one embodiment, the second region of the first probe or the first region of the second probe is any one of SEQ ID NOs 1-1345 or a complement thereof.

The present invention provides a composition pair comprising a first composition and a second composition. The first composition includes a first probe and a second probe: the first probe includes a first region comprising a first target-specific sequence; and a second region that does not overlap with the first region and does not bind to the target molecule; and the second probe includes a first region that binds to the second region of said first probe; and a second region comprising at least one affinity moiety, wherein the first region does not overlap with the second region. The second composition includes a third probe and a fourth probe: the third probe includes a first region comprising a second target-specific sequence; and a second region that does not bind to the target molecule, wherein the first region and the second region do not overlap; the fourth probe includes a first region that binds to the second region of said third probe; a first label attachment region which is hybridized to a first RNA molecule, wherein the first RNA molecule is attached to one or more label monomers that emit light constituting a first signal; and a second label attachment region, which is non-overlapping to the first label attachment region, and which is hybridized to a second RNA molecule, wherein the second RNA molecule is attached to one or more label monomers that emit light constituting a second signal. The first target-specific sequence and the second target-specific sequence bind to different regions of the same target molecule. The first and second probes of the first composition cannot bind to the third or fourth probe of the second composition. When the composition pair is bound to its target molecule, the identity of the first and second signals and their locations relative to each other constitute at least part of a code that identifies the target molecule. When the composition is bound to its target molecule, the code comprises the identity of the first and second signals and their locations relative to each other. The code comprises the identity of the first and second signals, and the size of the spot resulting from at least one of said signals. The first, second, third and fourth probes are nucleic acid molecules.

In some embodiments, the second probe further comprises at least a first label attachment region which is hybridized to a first RNA molecule, wherein the first RNA molecule is attached to one or more label monomers that emit light constituting a first signal.

In some embodiments, the second region of the first probe or the first region of the second probe includes any one of SEQ ID NOs: 1-1345, or a complement thereof. The second region of the third probe or the first region of the fourth probe includes any one of SEQ ID NOs: 1-1345, or a complement thereof. The second region of the first probe and the second region of the third probe are not the same sequence.

In any of the foregoing compositions, a plurality of first RNA molecules are hybridized to the first label attachment region, wherein the first RNA molecules are attached to said one or more label monomers the emit light constituting said first signal; and wherein a plurality of second RNA molecules are hybridized to the second label attachment region, wherein the second RNA molecules are attached to one or more label monomers that emit light constituting a second signal.

In any of the foregoing compositions, the first signal and the second signal are spatially or spectrally distinguishable.

In any of the foregoing compositions, the first and second label attachment regions are predetermined nucleotide sequences. The first and second probes are nucleic acid molecules.

The present invention provides a composition including: a first region containing a target-specific sequence; and a second non-overlapping region containing any one of SEQ ID NOs: 1-1345, or a complement thereof.

The present invention also provides a method of detecting a target molecule in a biomolecular sample by: contacting the sample with the composition pair of the present invention under conditions that allow (i) binding of the first target-specific sequence and the second target-specific sequence to the target molecule, (ii) binding of the first probe to the second probe; and (iii) binding of the third probe to the fourth probe; and detecting the code that identifies the target molecule. In some embodiments, the method further includes quantitating the amount of said target molecule in said biomolecular sample.

The present invention also provides a method of detecting a plurality of target molecules in a biomolecular sample by: contacting said sample with a population of composition pairs of the present invention under conditions that allow (i) binding of the first target-specific sequence and the second target-specific sequence of each composition to their respective target molecule, wherein each composition in said population when bound to its respective target molecule is associated with a distinguishable code; (ii) binding of the first probe to the second probe; and (iii) binding of the third probe to the fourth probe; and detecting the codes that identify the plurality of target molecules. In some embodiments, the method further includes quantitating the amount of each of said plurality of target molecules in said biomolecular sample. In some embodiments, the fourth probe is different for each target molecule in said biomolecular sample. In some embodiments, the second probe is the same for all target molecules in said biomolecular sample.

The present invention also provides a method of manufacturing the second probe of the compositions of the present invention, by introducing the sequence of the first region adjacent to the sequence of the second region in an expression plasmid, and transcribing the first and second region to produce the second probe.

The present invention also provides an expression plasmid for the manufacture of the second probe of the compositions of the present invention, in which the sequence of the first region includes any one of SEQ ID NOs: 1-1345, or a complement thereof.

While the disclosure has been described in conjunction with the detailed description thereof, the foregoing description is intended to illustrate and not limit the scope of the disclosure, which is defined by the scope of the appended claims. Other aspects, advantages, and modifications are within the scope of the following claims.

The patent and scientific literature referred to herein establishes the knowledge that is available to those with skill in the art. All United States patents and published or unpublished United States patent applications cited herein are incorporated by reference. All published foreign patents and patent applications cited herein are hereby incorporated by reference. All other published references, documents, manuscripts and scientific literature cited herein are hereby incorporated by reference.

While this disclosure has been particularly shown and described with references to preferred embodiments thereof, it will be understood by those skilled in the art that various changes in form and details may be made therein without departing from the scope of the disclosure encompassed by the appended claims.

BRIEF DESCRIPTION OF THE DRAWINGS

Figure 1 is an illustration of a non-tag-based nanoreporter system utilizing a reporter and capture probe. Both reporter and capture probes contain unique target-specific sequences for direct binding to different regions of the target nucleic acid. In this example the reporter probe also contains a 6-position fluorescently-labeled reporter code which uniquely "barcodes" the target sequence. The capture probe also contains an affinity moiety, biotin (B).

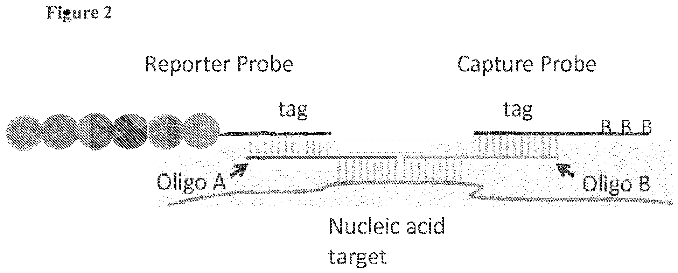

Figure 2 is an illustration of a tag-based nanoreporter system utilizing a reporter oligo (Oligo A) and a capture oligo (Oligo B) as intermediate probes that bind to the reporter or capture probe. Both Oligo A and Oligo B contain unique target-specific sequences that bind to different regions of the target nucleic acid, and distinct tag sequences that bind to either the capture probe or the reporter probe. In this embodiment, the tag sequence in Oligo B can be a universal tag associated with all target-specific capture sequences. However, the target-specific sequence in Oligo A is associated with a unique tag sequence. Each species of Oligo A carries a unique tag sequence which is associated with a specific reporter code. Under the appropriate hybridization conditions, Oligo A and Oligo B will bind to the target nucleic acid. The universal capture probe will bind to Oligo B, and a specific reporter probe will bind to its associated Oligo A, uniquely barcoding the target sequence.

**Figure 3** is an illustration of a tag-based nanoreporter system in which the capture probe as well as the reporter probe is fluorescently labeled. In this embodiment, the tag sequence in the capture oligo (Oligo B) is a tag associated with all target-specific capture sequences which have been assigned the additional color(s) contributed by the capture probe.

**Figure 4** is a graph that compares the average background counts of the internal negative control reporters generated from the standard nanoreporter system to the tag-based nanoreporter system.

**Figure 5** is a scatterplot demonstrating the flexibility of assay content in the tag-based nanoreporter system.

**Figure 6** is a scatterplot showing the log2 fold-change correlations between the measurements made by the standard nanoreporter system and the tag-based nanoreporter system of the present invention.

**Figure 7** is a scatterplot showing the reproducibility of tag-based nanoreporter system of the present invention.

**Figure 8** is a scatterplot showing the assay performance of tag-based nanoreporter system of the present invention on FFPE samples.

**Figure 9** is a scatterplot showing the assay performance of tag-based nanoreporter system of the present invention on cell lysate.

**Figure 10** is a scatterplot showing the assay performance of tag-based nanoreporter system of the present invention on amplified samples.

## DETAILED DESCRIPTION OF THE INVENTION

The present invention pertains to a multiplexable tag-based reporter system and methods for production and use. The present invention is based upon the discovery that nanoreporters comprising a reporter and capture probe and probes that bind to the reporter or capture probe and a target molecule can be utilized for the accurate and efficient detection and quantification of a plurality of target molecules in complex mixtures. Moreover, these nanoreporters allow more reliable and reproducible methods for manufacturing nanoreporters and reduce both the variability and the false positives of previous nanoreporter systems. The tag-based nanoreporter system allows economical and rapid flexibility in the assay design, as the gene-specific components of the assay are now separated from the nanoreporter and capture reagents and are enabled by inexpensive and widely available DNA oligonucleotides. A single set of nanoreporters can be used to probe for an infinite variety of genes in different experiments simply by replacing the gene-specific oligonucleotide portion of the assay. In the non-tag-based reporter system (**Figure 1**), the reporter and capture reagents (*e.g.*, the label attachment regions and attached labels, and affinity moieties) are covalently attached to the target-specific regions, and are complicated and costly to modify.

The present invention provides probes comprising two regions, a first region comprising a target-specific sequence and a second region that does not bind to the target. The first region and the second region do not overlap. The second region contains a tag sequence which can bind or hybridize to a region of a reporter probe or a capture probe. A probe that binds or hybridizes to a reporter probe is referred to herein as a reporter oligo (*e.g.*, Oligo A in **Figure 2**). A probe that binds or hybridizes to a capture probe is referred to herein as a capture oligo (*e.g.*, Oligo B in **Figure 2**).

The present invention provides compositions (also referred to herein as reporter oligos or capture oligos) comprising two regions, a first region containing a target-specific sequence, and a second non-overlapping region comprising any one of SEQ ID NOs: 1-1345, or a complement thereof.

The present invention provides compositions comprising a first and a second probe. In one embodiment, the first probe, contains a first region comprising a target-specific sequence and a second non-overlapping region that does not bind to the target. The second non-overlapping region contains a tag sequence that binds or hybridizes to a second probe. In some embodiments, the second probe may comprise at least one label attachment region that is hybridized to at least one RNA molecule, wherein the RNA molecule is labeled or attached to one or more label monomers that emit a signal that contributes to the nanoreporter code. In some embodiments, the second probe may comprise at least one affinity moiety. The second probe also comprises a region that does not overlap with the label attachment region(s) and/or the affinity moiety. In some embodiments, this region is complementary to and binds the tag sequence of the first probe. In one embodiment, the second region of the first probe (e.g., reporter oligo or capture oligo), or the first region of the second probe (e.g., reporter probe or capture probe) comprises any one of SEQ ID NOs 1-1345 or a complement thereof.

In some embodiments, the second probe may comprise two label attachment regions, wherein the first and second label attachment regions are hybridized to a first and second RNA molecule respectively, and each first and second RNA molecule is attached to one or more label monomers that emit light that constitute a first and second signal, respectively. In some embodiments, the second probe may comprise three or more label attachment regions, wherein at each label attachment region, an RNA molecule attached to one or more label monomers that emit light that constitute a signal can bind. The signals emitted from the labeled monomers contribute to the nanoreporter code that identifies the specific target molecule.

The compositions of the present invention can be labeled with any of a variety of label monomers, such as a fluorochrome, dye, enzyme, nanoparticle, chemiluminescent marker, biotin, or other monomer known in the art that can be detected directly (e.g., by light emission) or indirectly (e.g., by binding of a fluorescently-labeled antibody). Generally, one or more of the label attachments regions in the reporter or capture probe is labeled with one or more label monomers, and the signals emitted by the label monomers attached to the label attachment regions of a reporter probe or capture probe constitute a code that identifies the target. In certain embodiments, the lack of a given signal from the label attachment region (i.e., a "dark" spot) can also constitute part of the nanoreporter code.

In certain preferred embodiments, the label monomers are fluorophores or quantum dots.

In some embodiments, the first probe is a reporter oligo and the second probe is a reporter probe. A reporter oligo of the present invention contains two regions, a first region containing a target-specific sequence and a second region that does not overlap with the first region and contains a tag sequence that can bind or hybridize to a reporter probe. A reporter probe of the present invention contains a first region containing a complementary sequence that binds to the tag sequence of the reporter oligo, and a second non-overlapping region containing at least one label attachment region. In some aspects, the second region may comprise one, two or three label attachment regions, wherein at each label attachment region, an RNA molecule attached to one or more label monomers that emit light that constitute a signal can bind. The signals emitted from the labeled monomers contribute to the nanoreporter code that identifies the specific target molecule.

In some embodiments, the reporter probe does not bind or hybridize directly to the target molecule. This reporter probe binds to the reporter oligo and is used to detect target molecules in a composition further comprising a capture probe bound to a target-specific capture oligo.

In some embodiments, the first probe is a capture oligo and the second probe is a capture probe. A capture oligo of the present invention contains two regions, a first region containing a target-specific sequence, and a second region that does not overlap with the first region and contains a tag sequence that can bind or hybridize to a capture probe. A capture probe of the present invention contains a first region containing a complementary sequence that binds to the tag sequence of the capture oligo, and a second non-overlapping region containing one or more affinity moieties for purification and/or for immobilization. The affinity moieties may be attached to the capture probe by covalent or non-covalent means. Various affinity moieties appropriate for purification and/or for immobilization are known in the art. Preferably, the affinity moiety is biotin. In some embodiments, the capture probe may contain a second affinity moiety, such as repeat sequences, which are used for affinity purification through hybridization to an oligo column, in which the column contains oligos that are complementary to the repeat sequences of the capture probe.

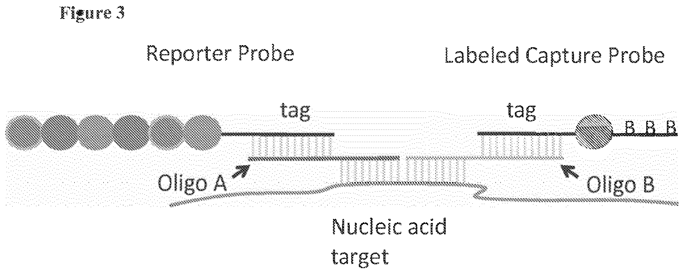

In some embodiments, the capture probe is not labeled. Alternatively, in some embodiments, the capture probe comprises at least one label attachment region that is hybridized to at least one RNA molecule, wherein the RNA molecule is labeled or attached to one or more label monomers that emit a signal that contributes to the nanoreporter code (**Figure 3**). In some embodiments, the capture probe comprises two or more label attachment regions, wherein at each label attachment region, an RNA molecule attached to one or more label monomers that emit light that constitute a signal can bind. The signals emitted from the labeled monomers contribute to the nanoreporter code that identifies the specific target molecule.

In some embodiments, the capture probe does not bind or hybridize directly to the target molecule. This capture probe binds to the capture oligo and is used to detect target molecules in a composition further comprising a reporter probe bound to a target-specific reporter oligo.

The present invention provides a tag-based nanoreporter system comprising a first composition and a second composition, wherein the first composition comprises a first probe and a second probe, said first probe comprising a first region comprising a first target-specific sequence; and a second region that does not overlap with the first region and does not bind to the target molecule; said second probe comprising a first region that binds to the second region of said first; a first label attachment region which is hybridized to a first RNA molecule, wherein the first RNA molecule is attached to one or more label monomers that emit light constituting a first signal; and a second label attachment region, which is non-overlapping to the first label attachment region, and which is hybridized to a second RNA molecule, wherein the second RNA molecule is attached to one or more label monomers that emit light constituting a second signal; and wherein the second composition comprises a third probe and a fourth probe, said third probe comprising a first region comprising a second target-specific sequence; and a second region that does not bind to the target molecule, wherein the first region and the second region do not overlap; said fourth probe comprising a first region that binds to the second region of said third probe; and a second region comprising an affinity moiety, wherein the first region does not overlap with the second region; wherein the first target-specific sequence and the second target-specific sequence bind to different regions of the same target molecule, and wherein the first and second probes of the first composition cannot bind to the third or fourth probe of the second composition, and wherein when said composition pair is bound to its target molecule, the identity of the first and second signals and their locations relative to each other constitute at least part of a code that identifies the target molecule. In some embodiments, the second probe further comprises at least a first label attachment region which is hybridized to a first RNA molecule, wherein the first RNA molecule is attached to one or more label monomers that emit light constituting a first signal. For example, the tag-based nanoreporter system comprises a reporter probe that binds to or hybridizes to a reporter oligo via a tag sequence, wherein the reporter oligo also specifically binds to the target molecule, and a capture probe that binds or hybridizes to a capture oligo via a tag sequence, wherein the capture oligo also specifically binds to the target molecule. The reporter oligo and the capture oligo bind to different regions of the target molecule. Once the composition pairs (reporter probe/oligo and capture probe/oligo) are bound to the target molecule, the identity of the signals from the reporter probe and, optionally, the capture probe, constitutes part of the signal that identifies the target molecule. In addition, the reporter oligo does not hybridize to the capture probe or capture oligo, or any other probes or oligos that detect different target molecules, or any other biological sequences present in the biomolecular sample. Similarly, the capture oligo does not hybridize to the reporter probe or reporter oligo, or any other probes or oligos that detect different target molecules, or any other biological sequences present in the biomolecular sample.

In some embodiments, the second region of the first probe, or the first region of the second probe comprises any one of SEQ ID NOs: 1-1345, or a complement thereof, and the second region of the third probe, or the first region of the fourth probe comprises any one of SEQ ID NOs: 1-1345, or a complement thereof. In some embodiments, the second region of the first probe and the second region of the third probe are not the same sequence. Therefore, the first and second probe cannot hybridize to the third or fourth probe, and the third and fourth probe cannot hybridize to the first or second probe.

The tag-based nanoreporter systems of the present invention can be oriented such that the reporter probe/reporter oligo hybridizes upstream of the capture probe/capture oligo on the target molecule. Alternatively, the tag-based nanoreporter systems of the present invention can be oriented such that the capture probe/capture oligo hybridizes upstream of the reporter probe/reporter oligo on the target molecule.

Schematics illustrating the compositions of a tag-based nanoreporter system are depicted in **Figures 2 and 3**.

**Figure 3** shows a tag-based reporter system that utilizes a capture probe that contains at least one label attachment region, attached to a label monomer for detection. In this system, each capture probe with a unique label and each corresponding capture oligo contains a specific tag and its complement, respectively. For example, in a system where each capture probe contains a single fluorescent spot, each color of capture probe will be assigned a specific tag sequence, and each capture oligo will contain a complementary sequence to the tag sequence of capture probe with a specific color. The final code assigned to the specific gene will contain both the spot colors from the reporter probe and the additional spot color from the capture probe.

The present invention also provides methods of detecting or quantifying an individual or plurality of target molecules in a biomolecular sample, using the compositions and systems described herein.

In certain embodiments, the methods of detection are performed in multiplex assays, whereby a plurality of target molecules are detected in the same assay (a single reaction mixture). In a preferred embodiment, the assay is a hybridization assay in which the plurality of target molecules are detected simultaneously. In certain embodiments, the plurality of target molecules detected in the same assay is at least 5 different target molecules, at least 10 different target molecules, at least 20 different target molecules, at least 50 different target molecules, at least 75 different target molecules, at least 100 different target molecules, at least 200 different target molecules, at least 500 different target molecules, or at least 750 different target molecules, or at least 1000 different target molecules. In other embodiments, the plurality of target molecules detected in the same assay is up to 50 different target molecules, up to 100 different target molecules, up to 150 different target molecules, up to 200 different target molecules, up to 300 different target molecules, up to 500 different target molecules, up to 750 different target molecules, up to 1000 different target molecules, up to 2000 different target molecules, or up to 5000 different target molecules. In yet other embodiments, the plurality of target molecules detected is any range in between the foregoing numbers of different target molecules, such as, but not limited to, from 20 to 50 different target molecules, from 50 to 200 different target molecules, from 100 to 1000 different target molecules, from 500 to 5000 different target molecules, and so on and so forth.

Preferably, the target molecule is DNA (including cDNA) or RNA (including mRNA and cRNA).

The present invention may be particularly useful for multiplex assays to detect a plurality of target molecules in a sample, for example, a set of genes. Each target molecule, or gene of interest, in a multiplex assay is assigned a unique or distinct tag sequence in the reporter oligo, thereby associating each gene with a unique reporter code as designated by the unique linear arrangement of label monomers associated with the reporter probe. A single non-labeled capture probe can be utilized for all genes wherein the region of each target-specific capture oligo that binds to the capture probe is the same for all target genes. In such a system, the specificity for each individual target gene is directed by the target-specific sequence of each capture oligo; therefore, each target gene has a unique capture oligo comprising a unique target-specific sequence plus a universal capture tag sequence.

As will be appreciated from the description and examples provided below, the present invention provides numerous advantages over the previous nanoreporter systems, such as those described in U.S. Patent Publications US2010/0015607 and US2010/0047924. In those previous "standard" nanoreporter systems, the reporter probe and the capture probe are custom-made in small batches for each gene target of interest, which is highly inefficient. The gene-specific portion of the probes is covalently attached to the reporter code region in the case of the reporter probe, or the affinity-moiety regions in the case of the capture probe. Thus, there are an almost infinite number of possible variants of these reagents, which increases the cost of manufacture and precludes efficient production of the probes as generic stock items.

In addition, the standard approach of enzymatic or chemical ligation for generating unique target-specific reporter and capture probes has limitations in consistency, as well as efficiency. For example, the manufacture of gene-specific reporter probes through the ligation of a target specific sequence to a selected reporter code sequence has at least two inherent drawbacks: 1) the reaction may not go to completion, leading to reporters which do not carry the target-specific sequence and therefore cannot detect the target of interest, thereby lowering the sensitivity of the assay for that particular target, and 2) any level of cross contamination of the gene-specific oligos or of the code-specific reporters during high-throughput manufacturing can give rise to gene-specific probes that are associated with the wrong reporter code, and generate false-positive read-outs.

While the generation of unique target-specific capture probes by ligation is not subject to cross contamination due to the generic nature of the affinity sequences, it is impacted by the necessity for each gene-specific capture probe to be above a certain concentration in the final pooled reagent, similar to the ligated reporter probe, in order to maintain the sensitivity of the assay. Due to the enzymatic or chemical nature of the ligation process, each gene-specific capture probe and reporter probe ligation must be monitored separately for completion of the reaction, involving significant labor and cost.

The tag-based system described herein provides a solution for high cost and inefficient manufacturing process of standard nanoreporter system components, as well as improving the quality of the reagents by eliminating issues of cross-contamination and variability. Specifically, ligation is completely eliminated from the production of the components of the tag-based system. Reporter probes for use with the tag-based system can be designed with stock tag regions that bind to the tags of the reporter oligos, where the tag regions are predetermined and predesigned for each reporter code, and therefore can be generated in mass batches. These tag regions are cloned into the specific plasmids used to generate each individual reporter, adjacent to the label attachment region(s) that form the unique reporter code. In this manner, the regions that bind to tags are an intrinsic and consistent part of the code-specific reporter molecule. Therefore, the inadvertent production of probe-less reporters is eliminated, thereby improving the manufacturing efficiency and lowering variability in target-to-target assay sensitivity. In addition, no reporters are produced from erroneous association of a probe with the wrong color-code through contamination of either gene-specific probe oligonucleotides or code-specific reporters, therefore eliminating the potential for such false-positives and improving the quality of data. Therefore, the present invention improves the quality of production and the ability to quantitate the effective activity of the reporter probes, leading to improved data from the assay through more consistent sensitivity and reduced false positives.

Similarly, capture probes can be fully synthesized as synthetic molecules without ligation, utilizing a single, or limited number of, complementary tag sequences for all hybridization reactions or target molecules in a multiplex assay. Thus, the capture probe(s) can be manufactured in large batches as individual biotinylated oligonucleotides in a highly cost-effective manner. The reagent can also be easily quantitated. Because the tag-based capture probe(s) are either identical or limited in number for each hybridization reaction in a multiplexed experiment, the probe-to-probe variability inherent to quantitation and pooling of the products of multiple ligation reactions to generate a unique capture probe for each specific target of standard nanoreporter assay systems is no longer a factor. The target-specific capture oligos can also be efficiently synthesized as synthetic DNA oligonucleotides, thereby substantially improving the efficiency and quality of assay reagent production. The specificity of the hybridization reaction, and therefore, the accuracy and reproducibility of the multiplex assay data, are improved as the quality of the reagents improves.

One additional advantage of the tag-based system is the ability to formulate the reagent with both the reporter and the capture probes in the same stock tube. This relates to the lowered background - in the standard system, the pre-mixing and storage of gene-specific reporter and capture probes together leads to significant elevation in assay background due to spuriousinteractions of some of the target-specific probe sequences over time. Not all sequence interactions can be predicted and eliminated during probe design; as there are almost infinite possible target-specific probes, it is not possible to pre-screen empirically for such interactions before manufacturing the reagents. In the tag-based system, the fixed set of capture oligos has been pre-screened empirically to have no interaction with the fixed set of reporter oligos, and thus the background is stable in a pre-mixed formulation. A pre-mixed formulation simplifies the reagent manufacturing and contributes to the assay consistency, and lowers the potential for spurious, unpredicted background.

The present invention exhibits lower background signal when compared to standard non-tag-based nanoreporter systems. Background in standard nanoreporter systems is most commonly due to the unpredicted, direct interaction of a capture probe with a reporter probe through the gene-specific sequences they carry. This direct interaction allows a capture and reporter molecule to form a complex which can undergo the dual purification without needing to be joined by a target molecule; the biotinylation of the capture further allows this complex to be captured and counted in the imaging cartridge. Such interactions lead to background reporter counts even in the absence of a target molecule. The higher the multiplex, the higher the likelihood of such interactions, as the complexity of the sequences in the reaction increases. However, in the instance where tagged intermediates are used, the gene-specific sequences are carried on non-biotinylated oligonucleotides; the spurious interaction of gene-specific sequences with each other or with a reporter does not lead to a viable biotinylated complex. In addition, the fixed capture probe tags can be pre-screened against the fixed pool of reporter tags and empirically adjusted or replaced to eliminate non-specific interactions, and those with minimal background can be pre-selected for use.

The present invention also provides improved methods of manufacturing reporter probes and capture probes. The methods described herein for manufacturing a reporter probe includes introducing the sequence of the first region containing the tag sequence or complement thereof, adjacent to the sequence of the second region containing the at least one label attachment region in an expression plasmid, and transcribing the first and second region to produce a single molecule. The methods described herein for manufacturing a capture probe includes introducing the sequence of the first region containing the tag sequence or complement thereof, adjacent to the sequence of the second region containing at least one affinity moiety and, optionally, at least one label attachment region in an expression plasmid, and transcribing the first and second region to produce a single molecule. Furthermore, the present invention provides expression plasmids encoding reporter or capture probes, wherein the first region of the reporter or capture probe comprises any one of SEQ ID NOs: 1-1345, or a complement thereof.

Nanoreporter Nomenclature

STANDARD NANOREPORTER SYSTEM: The term "standard nanoreporter system" refers to the existing non-tag-based nanoreporter systems that utilize capture and reporter probes that contain target-specific sequences for binding directly to the target molecule. This system allows for efficient detection and quantification of a plurality of target molecules, however has some drawbacks.

TAG-BASED NANOREPORTER SYSTEM: The term "tag-based nanoreporter system" refers to reporter systems utilizing four probes for each target molecule. Two of the probes (*e.g.*, reporter and capture oligo) bind specifically to the target molecule, and each also binds to another probe containing either a detection label (*e.g.*, reporter probe) or an affinity moiety (*e.g.*, capture probe) via a tag sequence. This system allows for more reliable and reproducible methods for manufacturing the probes and reduces the variability and false positives of previous non-tag-based nanoreporter systems.

NANOREPORTER: The term "nanoreporter", when not referring to the standard nanoreporter system, refers to tag-based nanoreporters, or compositions that include a first probe that has a target specific region and a region that hybridizes to a second probe, wherein the second probe comprises an affinity moiety, and a third probe that has a target specific region and a region that hybridizes to a fourth probe, and where the fourth probe has a label attachment region or an affinity moiety. For example, the nanoreporters of the present invention refer to a composition that includes a reporter oligo and reporter probe pair, and a capture oligo and capture probe pair, wherein the target-specific sequences of the reporter oligo and capture oligo recognize or bind to different sequences of the same target molecule. Nanoreporters are preferably synthetic, i.e., non-naturally-occurring, nucleic acid molecules.

PROBE: This refers to a molecule that can specifically hybridize to another molecule through a sequence-specific interaction. In some embodiments, the probes may contain target-specific sequences. In some embodiments, the probes may contain sequences that can hybridize to other probes. In some embodiments, the probes may contain label attachment regions and attached label monomers suitable for detection. In some embodiments, the probes may contain at least one affinity moiety, such as biotin or repeat nucleotide sequences.

REPORTER PROBE: A molecule that is labeled with at least one label monomer that emits a signal that contributes to the nanoreporter code and may or may not also contain a target-specific sequence. A reporter probe without a target-specific sequence contains instead a tag-specific sequence, which is complementary to a tag sequence present on a second probe (referred to herein as "reporter oligo"). The reporter probe binds or hybridizes to the second probe, which binds to the target molecule through a target-specific sequence.

CAPTURE PROBE: A molecule that has at least one affinity tag for purification and immobilization, and may or may not also contain a target specific sequence. Preferably the affinity moiety is biotin. The capture probe may also contain additional affinity moieties, such as repeat nucleotide sequences, for affinity purification. In some embodiments, the capture probe contains at least one label monomer that emits a signal that contributes to the nanoreporter code. A capture probe without a target-specific sequence contains instead a tag-specific sequence which is complementary to a tag sequence present on a second probe (referred to herein as "capture oligo"). The capture probe binds or hybridizes to the second probe, which binds to the target molecule through a target-specific sequence.

REPORTER OLIGO: The reporter oligo is a probe that comprises a target-specific sequence in a first region, and a second region that does not overlap with the first region and does not bind to the target molecule. The second region binds to a reporter probe.

CAPTURE OLIGO: The capture oligo is a probe that comprises a target-specific sequence in a first region, and a second region that does not overlap with the first region and does not bind to the target molecule. The second region binds to a capture probe.

TAG: The region of the reporter or capture oligo that binds to the reporter probe or capture probe, and/or its complementary sequence that is present in the reporter or capture probe that binds to the reporter oligo or the capture oligo. This sequence preferably consists of "alien" sequences which have no significant similarity to known biological genomes or sequences derived from these genomes. These tags are also typically selected due to structural properties, such as melting temperature and secondary structure.

TARGET-SPECIFIC SEQUENCE: The term "target-specific sequence" refers to a molecular entity that is capable of binding a target molecule. In the context of the tag-based nanoreporter system of the present invention, the target-specific sequence is covalently attached to a tag sequence in a reporter or capture probe. The target molecule is preferably (but not necessarily) a naturally occurring or synthetic DNA or RNA molecule or a cDNA of a naturally occurring RNA molecule or the complement of said cDNA.

SEGMENT: The term "segment" refers to a molecular entity attached to the label attachment region of the nanoreporter, generally for the purpose of labeling the nanoreporter. The segment can have one or more label monomers either directly (covalently or noncovalently) or indirectly attached to it.

NANOREPORTER CODE: The order and nature (e.g., primary emission wavelength(s), optionally also size) of spots of light from a nanoreporter serve as a nanoreporter code that identifies the target molecule capable of being bound by the nanoreporter through the nanoreporter's target-specific sequence. When the nanoreporter is bound to a target molecule, the nanoreporter code also identifies the target molecule. Optionally, the size of a spot can be a component of the nanoreporter code. Nanoreporter codes are also known as reporter codes, barcodes, or codes.

SPOT: A spot, in the context of nanoreporter detection, is the aggregate signal detected from the label monomers attached to a single label attachment site on a nanoreporter, and which, depending on the size of the label attachment region and the nature (e.g. primary emission wavelength) of the label monomer, may appear as a single point source of light when visualized under a microscope. Spots from a nanoreporter may be overlapping or non-overlapping. The nanoreporter code that identifies that target molecule can comprise any permutation of the size of a spot, its position relative to other spots, and/or the nature (e.g., primary emission wavelength(s)) of its signal. Generally, for each probe, probe pair, composition or composition pair of the invention, adjacent label attachment regions are non-overlapping, and/or the spots from adjacent label attachment regions are spatially and/or spectrally distinguishable.

Tags

The "tags" referred to herein are the sequences of the reporter or capture oligos that hybridize to a reporter probe or a capture probe, and their complementary sequences, or complements, present in the reporter or capture probe that hybridize to the corresponding reporter or capture oligo. In a reporter or capture oligo, the tag sequence is adjacent to, but not overlapping with, the target-specific sequence. In a multiplexed reaction, a given tag must only hybridize to its complement to form a reporter probe and reporter oligo pair, or a capture probe and capture oligo pair. This given tag must not cross hybridize to any other tag of a different oligo, reporter probe or capture probe, or any other biological or synthetic nucleic acid sequence present in the reaction under the conditions of hybridization at which the experiment is performed.

Preferably, a set of tags are "alien" sequences which have no significant similarity to known biological genomes or sequences derived from these genomes. In some embodiments, the tags may be 10 bases, 15 bases, 20 bases, 25 bases, 30 bases, 35 bases, 40 bases, 45 bases, 50 bases, 55 bases, or 60 bases long. Preferably, the tags are 25 or 35 bases long. The tags must also have similar melting temperatures (Tm's) under the same hybridization conditions so that the hybridization of each tag retains its specificity when mixed in the same reaction. In addition, the tags should be substantially free of any secondary structure which could impact the kinetics of hybridization to the complementary target. The sample tags in Table 1 are "alien tags" which have been matched for Tm and screened for minimal secondary structure and cross-hybridization with known biological sequences. Each tag can be synthesized adjoined to a target-specific sequence and can be utilized as the tag region of a reporter oligo or a capture oligo in a multiplex reaction.

**Table 1. Tag sequences**

| TAG SEQUENCE | SEQ ID NO: |
|---|---|
| AAAATGAGCACACTTTTTCCCATCTACCGTTACAG | 1 |
| AAAATGGCAAAATCAAAGGAAGAAATTCCAGAAGG | 2 |
| AAACCACATTTTTGACATTCGTGGATAGCTTTAAG | 3 |
| AAAGACAAACTCGTTGGCAATACATAAACTCCAGG | 4 |
| AAAGATATGTTTTCATCGGGAGACAGGATAACAAG | 5 |
| AAAGTCGGGTTCTACAGGGTATATGATGTTGCTCG | 6 |
| AAATAGGTCTGGCTGATATCTTTCTCTCCAATAGG | 7 |
| AAATCCCGGGGTTGTGTTTCCTAGAGCTAATTAGG | 8 |
| AACAAAATCCGGCTGATGAAAACCATTTATCATAG | 9 |
| AACGTCTGTCCAAATGGAGCTATAGTTAAGAGGGG | 10 |
| AAGCTCGCAATTTCATGCCCACTGGCAAGAGTAAG | 11 |
| AAGGTGATTCACTAACCAGCTCTTACTCCTCGTTG | 12 |
| AAGTACGCTCACATTACTTCACATGGTTGCGAATG | 13 |
| AAGTCTTTGTTCTGCGAACTCGTAAAGTCGTAATG | 14 |
| AAGTTGAATTGCTGAAAGGCAAAACCAATTTTATG | 15 |
| AATAGGTTACCTATGTGCGGTAAGACGTATCTCGG | 16 |
| AATAGTGGTTTTTGAGCAATAATTGAGACAGCTGG | 17 |
| AATGGCCCGTTCAGTTTGTCCGTTATGAAGATCGG | 18 |
| AATGTATTGGAAGAAAGAATCGACCCTTCTGGTAG | 19 |
| AATTGAGAACATCTCTGGGGTGACAACAAGTAAAG | 20 |
| AATTGGCGTATTCATATGGAACGGAAGGAAAGTGG | 21 |
| AATTTTGCTGTTTCAGCAATAGCCATAACAGCTAG | 22 |
| ACAAACAACCTTTTTTGGTAAAAGCTCCCTTGCTG | 23 |
| ACAATCCCAGTTCCCTCGCCTCAATTGGCATATTG | 24 |
| ACAGAAACAGAGTTAGACGAACACATAATAAAGCG | 25 |
| ACAGGGCGTTAACTATACTTTACATTGGTATGAGG | 26 |
| ACCATTCACCATAATCTAGTGCCCGGGGGTTACGAG | 27 |
| ACCTCAGCGACCTGTCCGTTACATTAATGAAACAG | 28 |
| ACGCGCTTATAACTTTGATATTGCAGGTCTGCTGG | 29 |
| ACGGACATACAGAGTGACGACAGTATTGCTTCGGG | 30 |
| ACGTAATCGGTCGCTCCTATCCAAGGTTCGACATG | 31 |
| ACGTCTGTGGAAGTCATGAGCACACGATCTGTAAG | 32 |
| ACTCCGCATAATCGAGGGGAGTAAAACCAAATTGG | 33 |
| ACTTTTCCGTCTGCTGTTTTCGTCAGAGATGCTAG | 34 |
| AGAACGTCTTTAGCGGCCTGTCATATTAACAACCG | 35 |
| AGACAGCTGTAGCTATAGGGACAGAACCAAGCTCG | 36 |
| AGACTAATTGATCGGACCGATGACAGTTCACAGAG | 37 |
| AGACTCCAGGTCGATCATTGGATAACCAACCAGTG | 38 |

(continued)

| TAG SEQUENCE | SEQ ID NO: |
|---|---|
| AGAGCAGCTATAAGACCATCACGCTACGGGTATCG | 39 |
| AGATTATGCGACTCTTGACGAACGTCATCGCGTGG | 40 |
| AGCATGATGTCCTAGTGAGGCACATGATGCATAGG | 41 |
| AGCGACGAGTTCCGATAATTTCGATCTAGGTGGCG | 42 |
| AGCTCTTCTACCTTCCCTTTTCCTATATATGTAGG | 43 |
| AGCTGCCGTTACAGTTCCTTCACCCTGTACATCAG | 44 |
| AGGAATTTGCGGGTCCTCATGCAAGTCTTGACCAG | 45 |
| AGGATATTATCTCATATGGCGGAGTAGAACGTCTG | 46 |
| AGGGAATACTTTTGCGAAGTTCCGTATAACTCAAG | 47 |
| AGGGTGTCAAGTAAATGATAGATATAACCCGAAAG | 48 |
| AGGTTTTTTGCATCGACATATTCTGCCACAAATAG | 49 |
| AGTACCCGACCCACGAATTAGATACCTAGACCAGG | 50 |
| AGTGACCCGTTTCTTTACATAGGTCTTCAAGAAGG | 51 |
| AGTTTCCCGATCAAACTGGAAGATAGGCGTCTTTG | 52 |
| ATAAGACGGCTTGGCATTTACCCTAGTCACTATCG | 53 |
| ATAAGTATTCCTTACGAGACATCCAATCCGAGCTG | 54 |
| ATAATAGGCGCAAGCTGATAGCATCCGAGCTAATG | 55 |
| ATACTAGGCATCGGACAGTCTGCCTCTGTACAAGG | 56 |
| ATATAGATTACTCCATGATACACCCAAGCCTCGAG | 57 |
| ATATGCGTTATCTCTGAGTTGCCGTCCACACGGTG | 58 |
| ATCCAGCTACTCTCCCTCCATATAGAGTGCATTTG | 59 |
| ATGAGCACGACAGACGCCATTATAGCACGACATAG | 60 |
| ATGCGACAAAGAATACATGATCAATGGTTTTCCCG | 61 |
| ATGGAAAGGCTGTTGGAGCAGTTGTTGATGGATGG | 62 |
| ATGGTCATAGTCGTTTTGTACGAGATCGAGACCTG | 63 |
| ATGTTACCACCCTGTAGTGTTTTTTATACAGATGG | 64 |
| ATGTTGCTCTGCAAGAACCTTTAGGCTAGGCCTTG | 65 |
| CAACAGATCGCCTGGGCAGTTTAATTGCAAAAAAG | 66 |
| CACAACATGCAGCAGGCAAGTAGGGTTTCTGATTG | 67 |
| CACCATTCAGCCTGATATTGCGTTTGGTGTTGATG | 68 |
| CACCGCCATAACTGATATTGTTCTATTGATTCAGG | 69 |
| CACGACATACCGCTGCATAACACGACACGTTCATG | 70 |
| CAGATTCTCCAGCCGCTCAAGCAGTCATGGAGATG | 71 |
| CAGCCATTTTTGAAAATTTCATCATCAGTATCGCG | 72 |
| CATACTTGATTGATTAACCCACTCATCTGAGACGG | 73 |
| CATATCTTCTTCCGTCTTGTTCGCAAGCTCTTGAG | 74 |
| CATTCCGTAGAATTACTACACCGCGGGATCATTAG | 75 |
| CATTCGAGGCATTACAGACACATTCGGCGCACTAG | 76 |

(continued)

| TAG SEQUENCE | SEQ ID NO: |
|---|---|
| CATTGACGAGAATCCTAGACATTCAGCGATAAGAG | 77 |
| CATTGATATAGGCCTTCATCCGTGAACAGAAATAG | 78 |
| CCAAAGCCCTCGCTATAGCAACTCTCTGTTGTTGG | 79 |
| CCAATTTAGCAAGTGCTACTGCTAAAGATGCTGAG | 80 |
| CCAGCAGGTGTTTCATTAGAAAAGTTCAGAAGAGG | 81 |
| CCATCAATAGCTAAAACCTTTTTTCCCAATTTAGG | 82 |
| CCCACAACATAAATCTCCTCAACAACAACATAGGG | 83 |
| CCCTAACCATGTTCTACGAGCGGTCACAGATTATG | 84 |
| CCCTCTCAACTGCAACATTTCCTTTAACAACCTCG | 85 |
| CCGGTTTTGTTCACTTATAAAGACGGTAACCGTAG | 86 |
| CCTCAAATCCCGCCACGAAACTAAGCGATTGAACG | 87 |
| CCTCCAACTCTGCCATCTTTAAGCATTCTAAAGCG | 88 |
| CCTTACAGTTACTGGTGGAACTGGCAAAACGGTGG | 89 |
| CCTTTTTAATTCCAAATGTCTCCTCATCAATCCCG | 90 |
| CGACCCATTGAGAGAGCCAATGGAATTAAGAACTG | 91 |
| CGACTAAGTGCTTGCCGACGTTACTAATGTGTCAG | 92 |
| CGATAATTCCCGTACATTTACTTGGGAAAGGGGAG | 93 |
| CGATGTATCATGTGAAAGACAGCTCATGCACTCGG | 94 |
| CGCAAAAAAATGACAAGATCGAGTGCATTGGCAGG | 95 |
| CGCAGCTACCCGGCTTGGTTACGATATAGTTCATG | 96 |
| CGCATAGTTATCAGTGTGCGTATCACTGTTCGAGG | 97 |
| CGCCAAATCGATTTACATCATGCTAGTGTGGACGG | 98 |
| CGGTAATTTGTCGTCGCACGGACAATTAGTGAGTG | 99 |
| CGTCGTCTTATTCCCAGTACACATCATTCCAAATG | 100 |
| CTAAATGAGTAGCCATTTCTCTATTTAACCCAGCG | 101 |
| CTACGTCAAGCGTTACATAGTGACGGAACTGTTAG | 102 |
| CTAGGATGTAACTTGCGTTAGTTGCAGATTCGCTG | 103 |
| CTCAGCATAGCGAAAGGTGCAAAATACAGATCGTG | 104 |
| CTCGAGAATCACACACAGTCGTCTAAGACACGACG | 105 |
| CTCGCTTTCACTTCTTCAAGTGATTTGCGTCCTAG | 106 |
| CTCTACGATGCTGCTCTACCTGCGATGTGAGCATG | 107 |
| CTCTCTTGCTATAAGTTCCATACTCCTTCTTGCTG | 108 |
| CTGGACGCTTGTTGCTGCGTATTTACAATAGCTCG | 109 |
| CTTAATCGGACGTATCGACTTTGGGTCCACGATAG | 110 |
| CTTAGGACTATGGATAAGTCATCTAAAGCGTCCGG | 111 |
| CTTCAATAGCCGTTTTTGCAAGACATAGAAAAGAG | 112 |
| CTTCCTGGCCCGTTGTAATGTAGTCATGCGTATGG | 113 |
| CTTCTGGTCATGATGAAGCTCAATAATCTCAACAG | 114 |

(continued)

| TAG SEQUENCE | SEQ ID NO: |
|---|---|
| CTTGATAGGAGAACTGTATCAGCGCTCAAAACGAG | 115 |
| GAAAAACAGCATCCCTGTATTTATAAAACGCACTG | 116 |
| GAAACAATAGGAATGTAGCGAGGAGTTAGGTTCG | 117 |
| GAAAATCATGGAGTTTCATAACCCAAAGCTAACGG | 118 |
| GAAATTTGACATCTATGAGCATGAGGATATTCCAG | 119 |
| GAATCAGCTACCGCCTGAAGAAGCTGAGATAACGG | 120 |
| GACGAGTCTTCATGGCATACTCCAAGTCAACTAGG | 121 |
| GAGAACGAGCGGAGCAAGATAGCCTTTAACTGAAG | 122 |
| GAGCATCTTCTCAACACCAAGAAAAGAAGAGGATG | 123 |
| GAGCATTATATCGCCCGTATCACGATGTATTAGAG | 124 |
| GAGCGGATGTTATTGAGAAGCACTTTACCTTAGAG | 125 |
| GAGCTCGTGTTGAACCCTTCAAGTAACAACCTGAG | 126 |
| GAGGTGGGAAGCATGTCCGTACTCCCATATATAAG | 127 |
| GATAACAGCACATACATTGCGCTAAGAGCTGCGTG | 128 |
| GATCGGAGTTTATTGATTTTGACTCTCTGTCAAAG | 129 |
| GATCTTCTTCTTCTTCAACCATGATTTCAGCATGG | 130 |
| GCAACAAGATGGAAAAAGCCAGTGTTTGTTAAAAG | 131 |
| GCAAGGTCAGTAACAGTTACATCAGCAAAATATCG | 132 |
| GCACGCACGATCAGGATACATACTGCAAGCATTGG | 133 |
| GCACTTAAGGACGGCGGTGCATGTCGTCTTTTTAG | 134 |
| GCAGTCATCGTAACCTGATAGCAATCTACGTCAAG | 135 |
| GCATTGCGGCTCATACTCTAGAAGCGATGTCACAG | 136 |
| GCCAAAAGCAAGAACGTCAGCATTATACATTCGGG | 137 |
| GCCGGCATGGTTACACCTCTAGCTAGAAAATAAAG | 138 |
| GCCGTCGGACATAACCACTTGGATATATACGTAGG | 139 |
| GCCTGGTACGCTCTATTCTTGCACCTAAACCGTAG | 140 |
| GCGAGCACAAAATAACAAGAGACTTTTCACCAAGG | 141 |
| GCGCATAACTCCTACACGGTGGTGAATCATAGCCG | 142 |
| GCTAATGTTGTTTTACCACTATCAACTCCTCCAAG | 143 |
| GCTCACCAGCTACTGGAAATACCGTTGCTAAGGTG | 144 |
| GCTTGTTAGGGATATTAAATGTTTCCTGGCCTTTG | 145 |
| GGAATGAATCCATTGCATTTCCATGAGAATGCAGG | 146 |
| GGATACACTGTTGAGCCGACCCTATTAGCTGATAG | 147 |
| GGCACCGCTCCATAAATTCAACTACGGCTTAATCG | 148 |
| GGCCTATCCGTACATATCGAGGAGCGATAGTCCAG | 149 |
| GGCTCTTGCAAAATTTGGAAAAAAAGTGGCTGTTG | 150 |
| GGGACTATATGAGGTTATCGCAACGAAACGCGGCG | 151 |
| GGTACCAGTCACCTAGTACTAGGCAACACCAAATG | 152 |

(continued)

| TAG SEQUENCE | SEQ ID NO: |
|---|---|
| GGTGTAACTTAAATCATATCTTACGCTGATAGGCG | 153 |
| GTAAACGATGGCGGGTCTCCGTTAACTCCAACATG | 154 |
| GTAACAATGGATGACTTTAGAAAGGCAGTTGAGAG | 155 |
| GTACATCCCTTAGGCGTTATTCTCGCTTTTTATGG | 156 |
| GTACTGATAGACAGTGTCACATTTGCTCTGCCTTG | 157 |
| GTAGCGGCAGTTTATACAAGAAAAGCTCCTAACAG | 158 |
| GTAGGAGCAAGCAAGCGTGTAAGGAATATAGATTG | 159 |
| GTATTCTGGAGAACCTCGTGGCAATGGCAATTCTG | 160 |
| GTATTGATGGACAGGGATCGTTTTTTGATCTTCTG | 161 |
| GTCGGATTAGCTCTTCTTTGATTAGCATGAAACTG | 162 |
| GTGATCTCGTGTCTGGCTTCATTAGGTTTGCTTTG | 163 |
| GTTCATCCCATCACGCCAACGTCTTGACAACTCTG | 164 |
| GTTCCCTAACCTTCCTGCTAACCCGCAGAATTGTG | 165 |
| GTTCTGCCAGAGAATATAACCGTTGTTCCAGGGCG | 166 |
| GTTGACTATCAACGAGTGGCAACCGACTCCTACGG | 167 |
| GTTGCGAATTTGCGTACCACCCGCAATAAGTATTG | 168 |
| GTTTCTAAGTTTGTAGCTGGAACACAGGAAGGAAG | 169 |
| GTTTGACCCTCCTTCAGCTAGCAGGGATTCTTGTG | 170 |
| GTTTTCTACTTGCGCTTCAAGCGTCCCAACGAAAG | 171 |
| TAAACCTCTTCACAAACCTCCCACTTGTTTCCTCG | 172 |
| TAAATGACCATGTTGATTTTGTCTGCTTGTGCCGG | 173 |
| TAAATTCTTCGTTATTGTAGGGTGGCAAACAACTG | 174 |
| TAACGTGAGAGAATGCGACGTAACTTTGCAGAATG | 175 |
| TAAGTACAAGCAGGTTCGGATCTTTGGAATATGGG | 176 |
| TAAGTGGGCAATCTTTAGCCAAACTTCGCCAACTG | 177 |
| TACAACGAGGTCGTTTTACGCATGTTCTTTAATTG | 178 |
| TACACTAGTCCAATGTCTCAACCAGGGATACCACG | 179 |
| TACCGACACTATTCCAGCAATGGCAATTGAGCTAG | 180 |
| TACTACAGGATCAAGCCGTCACTTCTCTCTTCCTG | 181 |
| TACTTCCTCAAGCGTTGAGCGGAATGCAGCAATCG | 182 |
| TAGTTACGTAGCTCATCTCGTAGGATCTGGGCTAG | 183 |
| TATATGTGGACCAGTATGCGTATAGACGGACACAG | 184 |
| TATCGAAGTAGGTATTTACGTGATACTGCAACAGG | 185 |
| TATCTATTGCGCGTCCATACATAATCTGGTTCACG | 186 |
| TATGGTGTTTTTCCAATAATTGCAGCTGCTAATGG | 187 |
| TCATCAAAAGTGAGTTGTGATGAAGAGCTTTTGGG | 188 |
| TCATTAGCGGTCAGCTAGGGTAGATCACGTGAGCG | 189 |
| TCGTGGAAGAGTTGCTACAGCTTTAACAGCCTCAG | 190 |

(continued)

| TAG SEQUENCE | SEQ ID NO: |
|---|---|
| TCTAAATCTAAATCAGCAACTGCCTTGGCAACTGG | 191 |
| TCTAAGCCTTCAATTGCTTGCTCAAATTTCGTATG | 192 |
| TCTATTTGTCTTAGCTCCTAAGACAGAGTTTGCAG | 193 |
| TCTTAGACGCGCGTGCAATTCTGAACTATATGATG | 194 |
| TCTTTAATTCCACCATTACCCAGCGGACAAATTCG | 195 |
| TGAAACAAGGTCACGCTGTCGCTTAGGTCTTGAGG | 196 |
| TGAACAACTAAATGCCATATGTATGCAGGTTAGAG | 197 |
| TGAATACTTAGCGAGGATCGTAGATCATTGACGAG | 198 |
| TGACCATTAAGGTCGTTTTGGGCCTAGAGCTCAAG | 199 |
| TGAGGACGAAGTGGGGTTTATATGGGTGGCGAAAG | 200 |
| TGAGTATCTACAGGTGTTCTCATGGGATCGTAGTG | 201 |
| TGATTTCTCTCCTCTGCCAGCTCGTCTGCATAGCG | 202 |
| TGCACTCGGATATATTCCACTCAGTGACCCAGTTG | 203 |
| TGCTAAGGTTGTTAATGGCATTGCAGATAGCTTAG | 204 |
| TGCTTTATAGGACCAGGAGGTTAGCGACACATCCG | 205 |
| TGGCAACTGCATACTGAATAACTCCCTGAAATAGG | 206 |
| TGGCATATTCAGGCTGGATTAACAGAGGACATGAG | 207 |
| TGGGTCTCCAACAACCAAATCAGCAACCTTAGGAG | 208 |
| TGGGTTAAATCGTCTTCACTTCTCTCTCCAGTTTG | 209 |
| TGGTGTGATTTCCTTCAGCAATCAACATACTTGAG | 210 |
| TGTAGTAACATCCTCCACAATAACAACCTTATCTG | 211 |
| TGTATTAGACACCTACACGATTAGTCAGGCACCGG | 212 |
| TGTGTTCCGATTGTAATACTTGTTCAATGGCCCGG | 213 |
| TGTTAACAACATGGTATACGCCACGCTAACTGGTG | 214 |
| TTAACTGAATCGTCAATTGCAGTGAAGTCGTAAGG | 215 |
| TTATCATGGTATTATCGAGCCGACCACGGCAGACG | 216 |
| TTCCATTTGCCAATAAGAATGCGTTTGGAGGGGTG | 217 |
| TTCCCTGCTCTTATTGTTTCCATGAAAGTGGATGG | 218 |
| TTCCGACTTTTAATAGGACGAGTTGCGCGGGCTAG | 219 |
| TTCGATCCAAATTTCCGGAATGTCAAAACCGTAAG | 220 |
| TTCTGGACTGGATGATCGGGGGTCACGAATTGATAG | 221 |
| TTGAAAAGAAGGGATAGAGATGGCATTCCCAACG | 222 |
| TTGAAAACAAGAACTGACTGCTAGATGTGTAAATG | 223 |
| TTGTTTTTGTGGCAATAGGTCTTGAAACCACTGCG | 224 |
| TTTAGATCCTAAGAATGCGAAATGCCGATTCCCGG | 225 |
| TTTATATCCCAACGTATATCCGGCGGTTGTTGGGG | 226 |
| TTTCAGCTGGCTTTAAATTCATTTGGTAGCCTAAG | 227 |
| TTTCCGTAATCGCAATCGTATGTTCAAAATGAGCG | 228 |

(continued)

| TAG SEQUENCE | SEQ ID NO: |
|---|---|
| TTTCTATATCAGCCACCATGGGAGTGACATTTCTG | 229 |
| TTTGATACGCTAAACCTTGGGGCGTAAGGCGTATG | 230 |
| TTTTATCTGCGCCTAATATGCGGGCTGCTTCAGCG | 231 |
| TTTTTAAGTATTGGGGAGAGGATTGCTTCAATAGG | 232 |
| TTTTTGGTGAGGTTGTTGGTAGTAGTGAGTTTGTG | 233 |
| AAACCTCTCTTAACTCTTCCTCGCTGATAATTTCG | 234 |
| AAACTGACCGTACCGTTAGAAGAGAGTTCCGCTTG | 235 |
| AAATTCATAGCCACAAATTCTCTTTGGGCAGAGAG | 236 |
| AAATTTCGATCTCTTAGAGTGGCTTATGACGGTAG | 237 |
| AACAAATACAACATAGTTGTTGCTGGTGGGCAGAG | 238 |
| AACATCTAATCTAACCCGGACGAACCATGGACTTG | 239 |
| AACCTCCTCCAAGATTCAGCACCTTGGATACAAAG | 240 |
| AACGTCGGTCTACCTAACGACATTTGTGGCTACGG | 241 |
| AACTCTCGTTCTGGTCGAAGCGACGTACCTTAAAG | 242 |
| AACTTTCTAGTTAACAGTCACCTAGTAAGTGGGCG | 243 |
| AAGAATGATTCCTGAGGGAAGTGATGCTATCTCAG | 244 |
| AAGACTCATTCTGACGGCCTCTAGTCGTTGATATG | 245 |
| AAGATAGTCTACCTCGGGCTCTCGATAAGAGAATG | 246 |
| AAGATATGGGAGTATTTCTCCAGAGATGCTTGCAG | 247 |
| AAGCCAGAAGAAGTTGTTGTAGCTTTCCCACCAGG | 248 |
| AAGGAAGGATATTAGTAGGGAGAAACGCTTGATGG | 249 |
| AAGGACATTCTTTCGAATGCAAGTTCAAGGCACAG | 250 |
| AAGTAGTAGATCCCGCCGTCTTAGTCGGATTGAAG | 251 |
| AATACGCTCACAATCCAGGCTATATCGCTGTAGCG | 252 |
| AATAGAGTTGTTTGCCAAGGAGAGGGCAAGGTCAG | 253 |
| AATGAACGTCGTACCGGTCACGTTTCGGTATCGAG | 254 |
| AATGTATGAGCGGACACTATGCTAAGAGAGACTCG | 255 |
| AATGTGTCAGCGGCCTAACTGTAATTGATCCACAG | 256 |
| AATTACCCAAGTTGCAAGTGGAAGATTTGGAGTTG | 257 |
| AATTAGTGGTGTTCCAGCCTCTAAGATGATGTGGG | 258 |
| AATTGCTTCTTTCGGTCCAGTGCTTCCATCAGTCG | 259 |
| AATTTCGCACTGACCATAATGTGATCCCTTCCGGG | 260 |
| ACAAAGAAGTGGGCTAAGATTGCAGCTACAGAGGG | 261 |
| ACACAGCTATCGACAGAGTCGTGACCATCATCGAG | 262 |
| ACAGATTATAGGTGGACTTGCGGAACCTCGCATTG | 263 |
| ACAGGTGTTGGAAAGCCAGTGTATGTGTTTCATAG | 264 |
| ACATGGATCTCATAGTCACCACATAGATCGCGACG | 265 |
| ACATTTGCAGCACTAGGGCGCTATATTCGAGACGG | 266 |

(continued)

| TAG SEQUENCE | SEQ ID NO: |
|---|---|
| ACCGGAATAAGGCCTGCTAGTCACGAATAGGTTAG | 267 |
| ACCTCGTGTCAATGAAAGGAGAGCGTTGCATTACG | 268 |
| ACCTGGCGGCGATAGTAGATGGATACCGGCATTAG | 269 |
| ACCTGGGTAAAGGACTATGGGTCATTCTGTCTGCG | 270 |
| ACGGGCCTTTAGTAGAACGACGTCTGAACACAGTG | 271 |
| ACGTCCATTAAGTTGGGACTTTCAGTCCCAATTAG | 272 |
| ACTCACACATAGTACTGACACGTAAGATAGGATGG | 273 |
| ACTCCAATGATTCCATATAACGGCCATAATGGAGG | 274 |
| ACTCCCATTCCTACCTCTCCAAAGTTAGAGGAGAG | 275 |
| ACTCGCATTCTCACCAAGAGTCGCGATATGAAGAG | 276 |
| ACTGCGTTATTGATATGTCGAAGTTTGTGGAATAG | 277 |
| ACTTGTTCGACTGACAGTTTAACGCCTGACATGAG | 278 |
| AGAATCATGGCGTATCTGAAGCGTTTGGCCATCCG | 279 |
| AGAATGCAGATGCTGTAGGAGTAAGCGACACCGTG | 280 |
| AGACACGACACACTGGCTTACGACACGACTAAGTG | 281 |
| AGAGAGCATCCACACCTCCGATTTCTAAATGACCG | 282 |
| AGAGTCACCATGATAACCATTTAATTTAGCACCGG | 283 |
| AGATGTCGCGCCAAGGAAAGGAGATAGCGGTACTG | 284 |
| AGATTATACGATTGTTGTTGTTACCCACAACATGG | 285 |
| AGCCCACGATCATTTCGTCTACCACACACTGTGAG | 286 |
| AGCGCAACGCAGTTAAGGTACTATAATTGAGCCCG | 287 |
| AGCGCTGTCACGGATGTATAAATCGCTCGAGAATG | 288 |
| AGCTCTTGAACCGTGTTAATACCCGCACGCTTTAG | 289 |
| AGGACATGATTGGCCAATGTAGAGTCTGCTACCGG | 290 |
| AGGACATGTTTCAACAATCACCGGATTAAAGCCTG | 291 |
| AGGCTGCACACCTTCTGAATGAGTCACACAGCTAG | 292 |
| AGTATAGCTATGCAGCTCGATGGACACGTCTAGCG | 293 |
| AGTCCACGGATAGCGTTTAGGGTCTCTTAGTTCGG | 294 |
| AGTCGTAAGATGCAGCATCAAGCACAGTGAGCTTG | 295 |
| AGTGAAACCTTCAAGCATGAATTGTAGCTGACGGG | 296 |
| AGTGCAAGCATACTCGGACTTACGATAGAGACGTG | 297 |
| AGTGGTTCTATCTCGCTACTCTCCTGGTGTAACTG | 298 |
| AGTTAAGCTCTGCACCTGTTACACTATAGTCATCG | 299 |
| AGTTTCGCTGGTTCGTTCTTGTTGTGCGCTCGTAG | 300 |
| ATAACCTGGTCTCCGGTTGATCGTTTACCTGAAAG | 301 |
| ATAACTCAATCATGCGCGTCCAGCAAAGACAAATG | 302 |
| ATAAGCCCTCGAATACAACTTGAGGTATCCCGCAG | 303 |
| ATAATGGAGCTATAGAATACAACACCAACGTCGCG | 304 |

(continued)

| TAG SEQUENCE | SEQ ID NO: |
|---|---|
| ATAGAACCATTTGCTGATGAGGTGACAACAGATCG | 305 |
| ATAGATGCAGAGGACTGATGCAAACAGCAGGTACG | 306 |
| ATAGTTCATTTCCCTAGTTCGATGGGCTAGGCCGG | 307 |
| ATATAGCTCCACCAGAGTATTGGTACAGACACTGG | 308 |
| ATATCTTTCTCGGGTAAAGATTAGGCGTCCGATAG | 309 |
| ATATGAGACGACTAGCACGCCATAGCGTTACATAG | 310 |
| ATATTCTGTACTCAGTGCCTATCCACCTAATAGGG | 311 |
| ATCAATCCCTCTATGCAAGATAACAACATCTGGCG | 312 |
| ATCACCGCAGTTATTACCAGATAGGCGAGTTTGAG | 313 |
| ATCCTCCAAGAAGATCCTTCACATCTGAGCTCGGG | 314 |
| ATCGGCTGTGCGATTGCTATTGATGTGTTAAGAAG | 315 |
| ATCTCTCTTGTGTTCAGACGAGGCCCAATTGAGCG | 316 |
| ATGAAGGGCAAGGAGTAATTTGTTCCCATCTATTG | 317 |
| ATGACCGACAGACGTTTGCCTATAGCAGACGACGG | 318 |
| ATGAGATTAGCAACGACCCAAACATGCCACTTCAG | 319 |
| ATGATCCAAGTTATATACATTAGGACGCGGTTGCG | 320 |
| ATGATGAGCTGAGGTTCTGACAGCAAATACGCTCG | 321 |
| ATGATGTGGTCGCTATTTGGAATTGTTTGTAACAG | 322 |
| ATGCAGATCCCTTCTGGTGCGTAAGGAGTGATAGG | 323 |
| ATGCTCGATCAGTGTCTCAGAGTCGAGCATGATGG | 324 |
| ATGGTTAGTAAACAGCTTTGATTTCTACATCCGCG | 325 |
| ATTCTCTTTACGGGCCACCAGGAACTGGAAAGACG | 326 |
| ATTGAGCAGTAATTTGTGCGAAGCCGCTCCTAGAG | 327 |
| ATTGAGCTAGCTACTGCAACCATCCTTGGACTTCG | 328 |
| CAAACTTGTAAAGCCCTACTTCTGCATGCAATCTG | 329 |
| CAACAACTTCCCTATCTTTAATCCTCTCACTCCAG | 330 |
| CAACGATATCTGCAAATCTTGCTGTGGCTCTTGCG | 331 |
| CAAGAGTAACTACCTTCGCGATAAGGCGCATAACG | 332 |
| CAAGCAATACTCTACCATAAAGGTGGAAGATTCCG | 333 |
| CAAGTTTCGCTCCTACTAGAGTTTAATACCCAAGG | 334 |
| CAATAGCTCCAGTAGTAATTGTTGTCGCTCCGCTG | 335 |
| CAATCTGCACAGAGGCAGGGATGAATGCAATTAGG | 336 |
| CACAGCCAATCTCTTAGGACAGTACATGGTTAGTG | 337 |
| CACCTAACTGTATGGCATAGTTATGCAGAAGTGCG | 338 |
| CACCTGTGACTACATGCTAGGAGCCTTGCACTTAG | 339 |
| CACGTTCATACTACTCACGATGACTCGGTTATTCG | 340 |
| CACTACGACTTCGGATACATTGCACTCACGAAGAG | 341 |
| CAGAATTGTTGTGTTCCTCGCCCTCAAGGTGATTG | 342 |

(continued)

| TAG SEQUENCE | SEQ ID NO: |
|---|---|
| CAGACACTGCGACAACTCACGATCATGACACAGAG | 343 |
| CAGACGACGTTCGCCATTTAACGACGAGGATACCG | 344 |
| CAGATAAACTATGGGTGAGCATGATCGAGCTAGTG | 345 |
| CAGATAGACTCACCTCGATATACAGGGAGCCACGG | 346 |
| CAGATAGACTGATAGGAGCCTGCTGTATGGATCTG | 347 |
| CAGCTTCCACTTTAGCGGAGAGCCTCGCATTATAG | 348 |
| CAGGGCGCTATATTAGACCAGAGGTGGCATAGTGG | 349 |
| CATATATAACGTACGTGCTGTACCACTCGGCTCTG | 350 |
| CATCACAATCACTGGAAGATTGAGCTTAGGAAAGG | 351 |
| CATCATTAAAGATGAGGAGATCAGCTTCAAGCTCG | 352 |
| CATCCCTCCCGACAGCCCTTTAATCTGATCATTCG | 353 |
| CATCTATGGAACGAATGAAGATCAAGGGTCGCCCG | 354 |
| CCATCTAGTACAGAGTAGTCTCATCCATCGCTGGG | 355 |
| CCCAAGTATGGTGTTTGGGTACAAGACGCCAAATG | 356 |
| CCCACCTCTTGCTGTAATGACCACAATCAACGTAG | 357 |
| CCCTAATCTCTTCTGGAGAGTCATCAACAGCTATG | 358 |
| CCGAAGAGTGTAATGGGCCTATCTGATGATCCAAG | 359 |
| CCGCAGACAATTAGTGAGCCGCGACGATTGATTAG | 360 |
| CCGCTCCATAGTACATTGTCACGCGCCATAGAGAG | 361 |
| CCGGATTCGTACTACTCGTTTACGGGATTTACAGG | 362 |
| CCGTCCCTTGAGTTCAATACGTCGCTCTCATGATG | 363 |
| CCGTTGATTTACGCAACAGCGGCTTATATAGCTCG | 364 |
| CCTCAGGAAGTCCAGAACAAGAGATACATTCATAG | 365 |
| CCTGCACAGTGAGTTTCTTTCACTCTAACTCTCTG | 366 |
| CGACACCGAGTTCGACCGTTATGTTGGTAGGATCG | 367 |
| CGACCTATGAGGACCTACAGCACTCTGAGAGGACG | 368 |
| CGAGCTAATGTATCAGCCTATACGCTAATGTCAGG | 369 |
| CGAGCTAGTGGATCAGATATCCAGGTAGTGAACTG | 370 |
| CGAGTTTGATCGAATAGTAGCCTCGTAAGTAAGAG | 371 |
| CGATTACAAGGCGTGGTCAGATATTAGACTCCAGG | 372 |
| CGATTAGCCGTAGACGCAACTCATTGCCGAAGATG | 373 |
| CGCAGATGATTTAAGCGACTCTCAGATCAGTGTCG | 374 |
| CGCATTATAGCGGTGCATCTTCAGTATCGCAGGAG | 375 |
| CGCGTAATGACTGCGTGGTTGTATGGTAGGAGCAG | 376 |
| CGCGTCATCAGTTATTGACCGGCAGGCTAGTCTAG | 377 |
| CGCTATTGTTCAACGGAATTAGGAACAAACTTGTG | 378 |
| CGGACGGAGCTATATTTGCCGTATCGAGCATTATG | 379 |
| CGGCAATGACCGACCATCATACATTCGCTATTGTG | 380 |

(continued)

| TAG SEQUENCE | SEQ ID NO: |
|---|---|
| CGGCGATGAAGTCCGCGAGGATATGTTTCTATATG | 381 |
| CGGCTTGCTTATAATGACTGGCAGGGTTATGAATG | 382 |
| CGGTATCGAGCCATGTAAACCCTAAATAGCTTACG | 383 |
| CGGTGACGTCATGGATCTCGCTTAATTCTACTATG | 384 |
| CGTAGATGTCAATACTAGCCTAGCACTTCACATAG | 385 |
| CGTATCAAAGATTTGCGAGCCGATATGGCAATGGG | 386 |
| CTAACTGGTGCGATTGTAAAGAAACATTATGGCCG | 387 |
| CTAATTCGACTACGACCTGGCATTCTAGCGTACCG | 388 |
| CTACAGGACATTTGGCGTTATCAACGATACACGCG | 389 |
| CTACATCACTATCGTGTGTAATATCAGCTGCCGTG | 390 |
| CTACCAATGCAGCGTGGGCTGAACATGAGGAGTAG | 391 |
| CTAGGCCTTATTAACCTCTCTCTCCTACATTTGCG | 392 |
| CTAGTAAGCTCACACCAGAGGCGCTAGTTACATTG | 393 |
| CTAGTAGAAGCTGTCGACAAGCCTTTGCTCGGTTG | 394 |
| CTATAACTCCCAATCTTGTGTCCATTAAACCTCCG | 395 |
| CTCAGAATATGTAACGCCTCGTCGAAATTATCACG | 396 |
| CTCCAGCATCTGAGCGCAATACATATCATGCGAGG | 397 |
| CTCCTAACATGACTTTAGGTTGTAACGGTTCAAGG | 398 |
| CTCTCCCTTATGCACCTGAACCTAATATTTCAACG | 399 |
| CTCTCTACCCTTATGCAGACCACATAATTACCCAG | 400 |
| CTCTGTTCGAACTTGTAATTGACCAAGTGCAAGCG | 401 |
| CTCTTCTGCCCTACATCACTATCGACTATAGCAAG | 402 |
| CTGACCGCTCAGTCACTGGTGTCATTGAGTACCTG | 403 |
| CTGAGTGCTGTTTAATGCGGGACATAAGGAAGGAG | 404 |
| CTGCATAGCTTCTCAGCACACGATTGAGAACGAGG | 405 |
| CTGCGGACGAGTATTGATATCGAGGGACGAGTCTG | 406 |
| CTGCTAATGCTGATGGCCCACCTTCTCTATTTGTG | 407 |
| CTGGCCTTTAAAGCTATTGGCACGGCGGTTTAGAG | 408 |
| CTGGTTAACTGCTCGAAGTTAATCTGCGACGCTCG | 409 |
| CTGGTTCACTTAAAGTCGCCTAGGCAACATCTAAG | 410 |
| CTGTTGTTGCCCTCCACTCAACTGATTTGGTTTGG | 411 |
| CTTAGTTCGGGAGCTACCGATCTAATCAACCGTTG | 412 |
| CTTCACATACGAGTTGACGATTACACATTCGAGGG | 413 |
| CTTCACTTCAATTGCTGTTGCCAATGACTTCAGCG | 414 |
| CTTCAGCACACGGTGCCATGAGTGTTGCTTTATAG | 415 |
| CTTGTGCCGTGTAAGAACAATGTCATTCCCTCTTG | 416 |
| CTTTCACGGTATCGGCTTCTATGGCGAATGACAGG | 417 |
| CTTTGTCATGTCGTGGAAGTATGTCTATATGTGGG | 418 |

(continued)

| TAG SEQUENCE | SEQ ID NO: |
|---|---|
| GAAAGGCATTTGACGGGAGCATTGACGAAGACATG | 419 |
| GAAAGTTAAAGTTAAGGAACCAGCACACTTGGATG | 420 |
| GAACAGCTTTCCTTGCTCCCTCTAAATCACCATTG | 421 |
| GAACTCATCTTTCCTTCTCCATCCAAACCCGTTAG | 422 |
| GAACTGTTAGCATATGCTCGGAACGTGTCGCACAG | 423 |
| GAATATCTGTATCCTTCACAACCACCCGATACCAG | 424 |
| GAATCCTCGACGCTATGACAGAACTACGCACACGG | 425 |
| GAATCTTGGAAGGTTTCCAGTTAAATAGGGCGTGG | 426 |
| GAATTGATAACTCCAAAGCAGAGGAAATGAACGTG | 427 |
| GACCATGTTGGAATCCCAATAGAAATGGCTATTGG | 428 |
| GACGCTGAGGTTTATATGAACGGCCGCAATTATGG | 429 |
| GACGGATGAACCAACATCTGCCTTAGACCCTATCG | 430 |
| GACTGGCCTCGATTGGATCGCTACAGCAAAGCTAG | 431 |
| GAGAAAGATAACTAAGAGGCATCATCGAGCAAAGG | 432 |
| GAGAATGAACGAGACCGCGTGACATGTACGAAACG | 433 |
| GAGAGATAACGACCCTCTGTCGTAAGCACTTAAGG | 434 |
| GAGGCATCTCTGCTAACTATATGCTGAACAGCTTG | 435 |
| GAGGTCTTGTTTCATCTAAACCGAGCAGGATGATG | 436 |
| GAGTACATGTTCGATGCCTGATTGTGTACCTGCTG | 437 |
| GAGTGATAGGATCACTCTAAGATCGGCCACTATAG | 438 |
| GATACACGGGAAACTGGCGTATAGATAGAATCGAG | 439 |
| GATAGCTTAGTAACAAATGCTATAGCTCAGGCAGG | 440 |
| GATATTCAGAATTGGACACATGGGAATCTGTGGAG | 441 |
| GATATTCAGCTCGGGATGGTCACTGACAAACTTTG | 442 |
| GATCCGAAATACCTAGAATCTAGCGATTATGACGG | 443 |
| GATTAAGGCTCCAAACGTCTGTCGCTGCATAGCTG | 444 |
| GATTCAGATGCGACTTAAGGCAAGTATCCGACTTG | 445 |
| GCAACAAGTGATGCTGACGCAGTTGTTATAGATGG | 446 |
| GCACCCAGTGGGAGGATGTTATTTCGCTTACATGG | 447 |
| GCACGGTGATCTTTCGAAGTCCATCAGAGCAGCTG | 448 |
| GCAGGCTAAATGTAACCCTTGGAAGGGATATCTCG | 449 |
| GCATCAGCGAGGATAGACTGATCCGCAGATGAGAG | 450 |
| GCCAGGTATGCCGTGAACGAGTTCTTCATTAACTG | 451 |
| GCCTCTCCAGAGAGGTTTGATATGTCAAGTTTCGG | 452 |
| GCCTTGCAACCTCTGGGTTTAAGCCGAGTAAGATG | 453 |
| GCGAAGTATCACCCATACATCTGAAGTAAGCGCCG | 454 |
| GCGATAAGACCGGATCTATTTAGGAGACGCTCGTG | 455 |
| GCGATATTATGCATTATTCAACGGACGCGGTCCAG | 456 |

(continued)

| TAG SEQUENCE | SEQ ID NO: |
|---|---|
| GCGCTCGTATCAGGCTATTCCTATAGCAGTTCACG | 457 |
| GCGCTCGTTTACTGTCTATTCACCATAGGTTCTCG | 458 |
| GCTAAGTTTGGAATTAAGAAAGGAGTTGCTGGAGG | 459 |
| GCTAATTAGACCTCTTAAGGCCTACATGGGTACGG | 460 |
| GCTACCTTAAACGCGTAGTTAGTTCGTTGATCAAG | 461 |
| GCTAGCATGTAATAGTAAGCACAAACGACATGATG | 462 |
| GCTATCAACTTCCCTATCCAAACCGTTGGATGAAG | 463 |
| GCTATCTCACCAGCTCCTCACCATGACATTTACTG | 464 |
| GCTCAGAGATAACCTCAACTGTGTGCTACGTACGG | 465 |
| GCTGGTGGAATACCTGGAGCTTCGTTATCGAAGTG | 466 |
| GCTGTCTATATTGGAACTGCTGCAATGGTTGCTCG | 467 |
| GCTTACATGCCATATGCTGTATATTCTTGCGTTAG | 468 |
| GCTTCAACGATTTCAATATACCCATTCGTCAGAGG | 469 |
| GCTTGTTACAAACTGTGGAAGCTACTTCCATTTGG | 470 |
| GCTTTGTAGGTTCAAGGGTGAGGCTATTTCGATCG | 471 |
| GGAAATCTATTGTGAGGTGGTATTATGGCTGAGCG | 472 |
| GGACGTCTTTAATGTAAGCGGGAATGGCCTCACTG | 473 |
| GGAGCTAAATATGAAGCACAACTTGAGAAGAAGAG | 474 |
| GGATAAGTCTATACGGTAATGGTTGATGGGTTACG | 475 |
| GGATACGACGTAAGGAGTTACCCAGAGTTGTACCG | 476 |
| GGGCACATCAAGTATATCAGTCCCTATCTGAACCG | 477 |
| GGGCTGAAGGGATTGTAGAGGAGATTGGAATAAGG | 478 |
| GGGTTACGAGAACACGCCAGAACCAATACTATCGG | 479 |
| GGTAGCAAATGAAATGCCGGATGCTGTTGAAGTAG | 480 |
| GGTCTGTCCAACATGACGTTATAGGCATAACTCCG | 481 |
| GGTCTTGAGACAGAACACTAAGCATTTCCTGCCCG | 482 |
| GGTGTACCATATTTCTCCGCTAAATAGAGAGCATG | 483 |
| GGTTCATTGTCTCATCGTACGGCTAATGTAGATAG | 484 |
| GTAACCGTAGTCGCGCAAACCGTTATATTACGGAG | 485 |
| GTAACGATAATGAGTACAACGCCCAATGGTCATAG | 486 |
| GTAAGCGCCATCACTGTCAAGTATAGCCACACTGG | 487 |
| GTACGAAACCTCGATGCCAAGATTACGGAACCCGG | 488 |
| GTACGGGTTGACCATGTCACTATATGTCGTCCGTG | 489 |
| GTCAGCTTATTCCCGAGGCATATGGCCCTACTTAG | 490 |
| GTCCTTCTGCTTATGACATTCCGTGCATTCCGTAG | 491 |
| GTCCTTTGTTGGGCGGACCGTAATGAGGAATTTGG | 492 |
| GTGAATGGAAAGAACGTTGCTTCCAGAATCAGCTG | 493 |
| GTGATCCGAAGAAGAACATCGATGGAGTGACCCGG | 494 |

(continued)

| TAG SEQUENCE | SEQ ID NO: |
|---|---|
| GTGCGCGAATACTATACGAGGTGGCTGAATACTTG | 495 |
| GTGGAAGCCGTATGCTCGATCAAGATCATGCGTCG | 496 |
| GTGGGCAGAAGCACTTAGCTGGAAAGATATTCAGG | 497 |
| GTGGGTTAGTATTCACTTAGCCTGCCTGTACGAAG | 498 |
| GTTAAGATTAGATCGCGAATCGGGCGACCTCAAGG | 499 |
| GTTACCTTGATGCAATAGTCTCTGTATGCGATCGG | 500 |
| GTTACGCACCTACAGTCGGATATACGATTACGCGG | 501 |
| GTTATGAATGTTTCGGGTATTTATCCCGTTTCACG | 502 |
| GTTGTTCCGACAACTGGACGGACTACGTGCTCTAG | 503 |
| GTTTATCATAGTTTGCAACTTGGCCTACACGAGTG | 504 |
| TAACATCCCTAAATCCAACTAATGGATGCAAAGCG | 505 |
| TAAGAGAATGGCGAACCTATGAATCGGTACCAGTG | 506 |
| TAAGTAAGAAGATCGGCTAAGGGTTACGAACATCG | 507 |
| TAATATTCGGGCGTTAACATTAGAAGGACCCTCCG | 508 |
| TAATGTCAGAGTCTTATAGTAGATGCAGCGGCAGG | 509 |
| TAATTCTTCCTTGATTCCGTGATTGGATGTCCCTG | 510 |
| TACACGAGTGTTCTCTACCTGATAAGATACACGGG | 511 |
| TACATAATGGCAGAAGACCCTCCGCATGCGACAAG | 512 |
| TACCATCATCAGCCTATCTCCGCAGTATAAGCCTG | 513 |
| TACCTTCTAGGCACATCTAAGCCGTTGGAGGTAAG | 514 |
| TACGACGATGGTGTATTCGATAGTACGAGCTGGAG | 515 |
| TACTCCGCGTACAGGGTTATGATAGGCATAGTTAG | 516 |
| TAGAATCGATCGGAATCACGCCGATTGGCTGATCG | 517 |
| TAGCACTTAGTCAATTAGCCAGGTAAGCATGTTGG | 518 |
| TAGCGTACCCTATATGCTATCACTGTAGTTACGTG | 519 |
| TAGGCGTTGAGGCTTTGTTTCTTTGCCTCTATTGG | 520 |
| TAGTGAACTGCTATCAGACTCACGTAACGCATATG | 521 |
| TATAACCAGAGTTTGGTGATGGAACCTTATTAGCG | 522 |
| TATAAGGCGTGGTAGAATTACTGGCACTCCAATGG | 523 |
| TATCCCGCATACGATGACTGTCAATTACACTAGTG | 524 |
| TATCGAACCTTCACTAACCCTAGAAATTAGTGGTG | 525 |
| TATGGATCTCTTGATCGAGCGAACCTCCCTTTAAG | 526 |
| TATTGCTTAAGCTCTGAGCTCCATGTCCAGTAATG | 527 |
| TCAGAGAACATTAATGCAGTTGTTGGCAGAGATGG | 528 |
| TCAGTTATCTTCCCTCCCATTAAAGAGCCAGCTAG | 529 |
| TCATCCAAATATAGTGTATGGCGTCGGAACCGTGG | 530 |
| TCATCTTCCGTATAACGAATGCCGAAACCTCCTCG | 531 |
| TCATTAACTGATACGCAAATGCTCCCGCGAAACCG | 532 |

(continued)

| TAG SEQUENCE | SEQ ID NO: |
|---|---|
| TCATTCACGGCGCTCATGGATCATACTGAGCGATG | 533 |
| TCATTGGGAGCAAACCATCTGTCTTTCGTATGGAG | 534 |
| TCCGGGTCGGGATTGCATATTTGAGGGCATGAAAG | 535 |
| TCCGTCTGATAGCGATACGTCCGTGATATGTGCAG | 536 |
| TCCTGATGAGAAGGGTAGATTGGAGATATTGAAGG | 537 |
| TCCTTTCCAGCATAAGAACCAGCCATATTGCTTAG | 538 |
| TCGACAACTTAACGGGCTAAAGTGAGCTTTGTAGG | 539 |
| TCGCCAACTAGTACCCGGGTATTTGCATCTATGGG | 540 |
| TCTAAGTAGCAAGCACCCTAGCGTATCAGCAAGAG | 541 |
| TCTACTCCGGTTGTAAACGTGACCAAATGGAGATG | 542 |
| TCTCATGATGTGCGCATCTCCCACATTATTTGACG | 543 |
| TCTGCTAATTGGGCGATTTCCCTCTTAACGACCGG | 544 |
| TGAATAAATTCGTTGGCGCTGTAGAGATCGGAGTG | 545 |
| TGAGAGGACTCCACGACATCATAGACGACTCCACG | 546 |
| TGAGGAGTAAGTATACGACGCCTGCACTAGTCACG | 547 |
| TGATGACAGTGACAATTGACCGAATTGCCTGATCG | 548 |
| TGATGGATGTCCAACTAATCTGCCTTTATCTGAAG | 549 |
| TGCAACATTCGAGCCCGACATGATACATACGACTG | 550 |
| TGCAGGGAATGTTAAGGTTGGCTACGAGTTTACCG | 551 |
| TGCGTCCTAGATTTCGAACTTTCATCATATCTTCG | 552 |
| TGCTCATTAGCTCCGAGCTAATGCACAGACAACTG | 553 |
| TGCTGGCTTTGAGCCAATAGATGTGTTAATGGCTG | 554 |
| TGGAACTCTACCAATTGGAGCTTTCTTAGCTGTCG | 555 |
| TGGAGGGTCGTAACCGCTATAGATGTGATTCACTG | 556 |
| TGGAGTTGGAGGATGTTATTGTATTAAAGCATCCG | 557 |
| TGGGCGTATGCTTTCTTTATCTTAGCCCTAATCTG | 558 |
| TGGTATGAGTAGAAGTCCCATGTACAGTCACATAG | 559 |
| TGTCACTCGCGCGGTACGTGTTTCGTTTATATCCG | 560 |
| TGTCGCACACGCACGGAATAGTATCCAATAGGACG | 561 |
| TGTGGAAGGACTGTGATAAACCAATAGGGTGTCAG | 562 |
| TGTGTAAATGTAGCTGCTGGACCTAAATAACCGAG | 563 |
| TGTTATAGCTCCAGGGCCAGAGATTAAAGGAATAG | 564 |
| TGTTGAAGCAATTGAACACTTCAGACAAGTTTGGG | 565 |
| TTAACAACCGTTGCGACGGGTCCGAGACATTATAG | 566 |
| TTACCCTATCTCGTCTATGTACGTCAGGCTGAATG | 567 |
| TTATACTTAATTCACGACTGGGATGCTGTGGAAAG | 568 |
| TTATAGGTGTTGTTCCAGAGGACCCTCATGTTAGG | 569 |
| TTATGGATTCCGATGATCCTCCGCGTGGTACAAAG | 570 |

(continued)

| TAG SEQUENCE | SEQ ID NO: |
|---|---|
| TTATGTCTCGGGAGTCTGATATTGGTACTTCTCCG | 571 |
| TTATTGGAGCTCCTACAAAGGAGGCATTAGTTGAG | 572 |
| TTATTTGACCGGATGGCCACCTATTGTTTGCAGGG | 573 |
| TTCAAGAAGCGCGATTTCATAGAAATTATCCACCG | 574 |
| TTCAAGCTCTTCCACGAGTGCCTTCAGCTCTTCTG | 575 |
| TTCAATAGGCGCCACTTAGGTGGAATATCGAGCGG | 576 |
| TTCACCAAGCTGAACAGGGTTGCGCTGAATAAATG | 577 |
| TTCAGCATGTTGAGCTTCGTCAGTTAAACCAGCGG | 578 |
| TTCAGTTATAATGTGTCCAGCAGAAGCAGGAATTG | 579 |
| TTCATCGCACACTACAGCTAAGGTAGACCGCACAG | 580 |
| TTCCACAGTGTGGGCAAACTGCCTTCAATATCTTG | 581 |
| TTCCATACTTCTCCTGGAGGTATGTCAATATTTGG | 582 |
| TTCTAAAGCTCTCTTCCTCCTCTCTTCTCCGCTCG | 583 |
| TTCTACTATGATACTAATTCGCTGTGCACCCAGTG | 584 |
| TTCTCGCAGTTGTAAACTTATAGTGTCGCGCCTAG | 585 |
| TTGCACTTATGCTATCCCGTTAGACTATCTGCTAG | 586 |
| TTGCAGAAGCATTCCCAATATGGGTTTCAAGAGTG | 587 |
| TTGCATTACAATGGCCGATCAAGATAAGGACATTG | 588 |
| TTGCTGCTAACTTCCCATACCATAGATATTTCTCG | 589 |
| TTGCTGGAGGAAACTTCTTTATAATGGCAGATACG | 590 |
| TTGTATTGTGTCTACACTGGTCCGTTCTTAGACGG | 591 |
| TTTCGGCCCAACTTATATGCTCTCCGAATCTTGGG | 592 |
| AAAAATACAAAGCTCCAATGGTTGTTGCTGGCTTG | 593 |
| AAAACAGGCAAGCCGGTGATTTTATCTACAGGAAG | 594 |
| AAAACCGCTTTTGGCCGACAGATTCTGATGACAGG | 595 |
| AAAACGCAGCAGGAACTACGTGATGTGTGACAAGG | 596 |
| AAAACGCCATAGTTTGTATTGATCGCAAGCGCCTG | 597 |
| AAAACTCAGCCAATTCATCGTAATACTTGAAGGCG | 598 |
| AAAAGACATTACAGTCCTCGGAGACCCTCTGCTAG | 599 |
| AAAAGGCTAAAGAAGCTGTTTTAAAAGAGGGGGAG | 600 |
| AAACCGTAACGGGAAGCATTTTCTTTCACAGCTTG | 601 |
| AAACGCACTTCTACTTAAATCGACCTTTTGAACGG | 602 |
| AAACTGACAGAAATCATGCCCCAAACCTGCACAGG | 603 |
| AAACTGTTGGAATTAGACCAGACCCAAGAGGGGGG | 604 |
| AAAGAAATAATGGCCTAATCCGGTTTTAGTCGGAG | 605 |
| AAAGATGACATGGCGCAAGTAGGGTCTATTTTTCG | 606 |
| AAAGCTGGGATTGTAACAACAAAACTTCCTTATGG | 607 |
| AAAGCTTGAACCTCACGATTTACTTTTGCTGTGGG | 608 |

(continued)

| TAG SEQUENCE | SEQ ID NO: |
|---|---|
| AAAGGTGTTCCAGCCATTTCAGCACTTTCTTTTAG | 609 |
| AAATACCGTTACCACAAGTGCAAATACTCCCATTG | 610 |
| AAATCAATCAAGACATCCGGTTGTGTTTCTGTAAG | 611 |
| AAATCTTTGATGGAAAAGCAATCTGAGGGTTGTGG | 612 |
| AAATGTTAGAGAGATTGGGGCAGTGTGTCTTAACG | 613 |
| AACATGATACGAGGTCATCAGACGTATATGAGACG | 614 |
| AACCACTGCTCCAACTACTGGGGCTGAACTAATAG | 615 |
| AACCCGAAACAGAGACCACATATGCAACTCCCCTG | 616 |
| AACCCGTTAAGACAGGGTTGTGAATACAAACAACG | 617 |
| AACCCTATTTAACACAACACCCATTAAAGGTGTTG | 618 |
| AACGTCGAAGAGCTTCAGAGGTAAGTGAAACAAGG | 619 |
| AACTCCCCTTGCTAAGTACCAGCGACCTAACACTG | 620 |
| AACTTGTATAGTACCGAAGAAGCCTTTATCCGGCG | 621 |
| AAGAAAGCGGCTTTGTGGGATGAGGTTAAAGATGG | 622 |
| AAGAAGAACACTGTAGCCGCTTGGCAGGACCATTG | 623 |
| AAGAGGGGGGGCTAATCTATTAGAGGTTTTGAATG | 624 |
| AAGCACTGTTTTTTCATGTCCCGCATAATCCTCAG | 625 |
| AAGCATTTTCCAAAGGAACAAAAGCGAAATCAACG | 626 |
| AAGCCATATTGATCACCCAAAAACGAGCGCTCGTG | 627 |
| AAGCGTCCGACGAGCTAAGGTACTTGAAAGTCCCG | 628 |
| AAGGCGTATCCGTTTCCCGCGATGTACATTTGTGG | 629 |
| AAGGGATTGACGCGTGTATTCAAGTCCGGATTCGG | 630 |
| AAGTCACTTATGGATAACCTCTGAGCAGAAGGGGG | 631 |
| AAGTCCGAGTCCAAGTTCTTCTAGTCTCGCTTTCG | 632 |
| AATAACCGACTTTCATGACGATTTTCTCTCCCTTG | 633 |
| AATAAGAAACCAGACTCAGCTTTAGGAACGGCTCG | 634 |
| AATATTCTCCGGCATGAATGGCGTGGGAATGAATG | 635 |
| AATGCATTTGCCAATGTAGCCATTGTATAACCAGG | 636 |
| AATGGGGAAATTAATTGAGTTTGGAGAGACAGAGG | 637 |
| AATGTTAGCCTACCTTCAATCACGCCCGATACCGG | 638 |
| AATGTTGCATTTGGCCCAAGAATTCATGGAATTAG | 639 |
| AATTCAGTCATTGTGTGCTGATGCTGTAGCGGCAG | 640 |
| AATTCCCCCAGCTACTCTAAACGCATCTATTGTAG | 641 |
| AATTCCCTGCAGATGTAGAGCATATACCGGAGAGG | 642 |
| AATTCTCCGTCATGTGGTCGTCTGATGCCTAACTG | 643 |
| AATTCTCTCCCCTCTTATATTATGCCTGTCTGCCG | 644 |
| AATTGCAAGAGATAACCGGCGTGATCCTGGCAAAG | 645 |
| AATTTCTGAGATTGTTGGTAGAGGGAGAAATGGGG | 646 |

(continued)

| TAG SEQUENCE | SEQ ID NO: |
|---|---|
| AATTTCTGGGTTTGTGGTGGCTTTTTTTATGTCTG | 647 |
| AATTTGAAGTTTGCCTTCTCTCGTTCTCGCCCGCG | 648 |
| ACAGAAACAGAGTTGGACGAACACATAATAAAGCG | 649 |
| ACAGAACTGAGTGTCATGTGTCCAAAGTTAAGCTG | 650 |
| ACAGAAGACGCAGTGATCGCTCAATGCGATATTAG | 651 |
| ACAGCAAACAGATAGGATCGGTAATCCGTTTCAAG | 652 |
| ACAGGCATCACATCAGATAATTTTTGCTGATCGTG | 653 |
| ACAGGTTTAAATTTCTCCAAGAAAATGCAGACAGG | 654 |
| ACATCACCAATGTGTCCTCACTGTCCTGCAGCTAG | 655 |
| ACCAAATTGATTGGGACGTGATGTCACATCAAGCG | 656 |
| ACCAACTCCTCCAGCAATTGCTAATAAGCAGTTTG | 657 |
| ACCAAGCTCTACTCCAGCAACTTTTACATCTTCAG | 658 |
| ACCACCCTTTTAAATGCATTCTCTCTTTTCATCCG | 659 |
| ACCACTATACATGATTCACGAAAATGCGCACGCCG | 660 |
| ACCACTGGATGTACTGAGCACCACCGAGAATGAAG | 661 |
| ACCATAAGATTGGCCTACCAATAGGTGCGTCGCAG | 662 |
| ACCATCTATCTGGATTGATGTTACAGCGGCACCAG | 663 |
| ACCCACAGGTTATACGGGATTATCCGGTTATCCAG | 664 |
| ACCCCATAAAGAACGATTTTGGTGGTATTGCCCAG | 665 |
| ACCCCTCCTTTACCCGAAGCTATAGTAATTATCAG | 666 |
| ACCCCTTTCTCTAAGATACTCTGGGTTTTGCTAAG | 667 |
| ACCGTTACAACCGTCCAGTTATCAGCCAATGTTTG | 668 |
| ACCTGACTCCTTATGCTTGCGTCAGCAGTTAGTAG | 669 |
| ACCTTAGGAACAGAGCCAAACATCTTTAAGCTTAG | 670 |
| ACGCCTTGTTATACCGTAGGACGTGCTGATAAGTG | 671 |
| ACGGACATACAGAGTGACGACAGAATTGCTTCGGG | 672 |
| ACGGGTATCTATCATCTTGGCTGACGAGGTGGGAG | 673 |
| ACGGTAACCGCGACGATAATAACCCGGACCAAATG | 674 |
| ACGTCCTCATCTCTTTTTGCTGTTTCTTCAGCTAG | 675 |
| ACGTCTAGCACATCAGAGGAACCTTATGAGCACGG | 676 |
| ACGTTTCAGAAGTACGCCAGACGTACCAATAGGGG | 677 |
| ACTACCATGTACTGCGCGAGACTAGCCTATCATTG | 678 |
| ACTAGTCACAATCGGTGACAATGGGTGCGTTTTCG | 679 |
| ACTAGTCACTTCGTCGTGATTTTGGCAAAGGGGAG | 680 |
| ACTATGTCGGACCACTGAGCCGATAGTGATACCAG | 681 |
| ACTCCCTAACAACTGAAAACTGCTCATTTTCGACG | 682 |
| ACTCCTACGAGGTGCGATTATTCGATACGACGATG | 683 |
| ACTCGACGATAACGTGCATCCCCTTTAGATAACGG | 684 |

(continued)

| TAG SEQUENCE | SEQ ID NO: |
|---|---|
| ACTCGGTGAGTGAATTGCATGGGGAGTTGTTACGG | 685 |
| ACTCGTACTAGCGATCTGATAGACTGCTACCAGCG | 686 |
| ACTGACGACTCATTCGCAGGCATGACCATCTAGTG | 687 |
| ACTGCAGTGAGGGCAACCAATACAAATTAAATCTG | 688 |
| ACTGGTGAAGGAGGATTGCCAAAAGCTCTCTACCG | 689 |
| ACTGTACAATATGCAATAAACCGACTACCGGCCAG | 690 |
| ACTGTCCAGCAGGTATTGGAAAAGAGACTTTAATG | 691 |
| ACTGTCTAATACAACCGGATTCTAAGACCACATGG | 692 |
| ACTTTATCTCTCCACAGTGTGGGCAGATTGTAACG | 693 |
| AGAAATGGGATGTTATGCCTTTTACAAAACTCAGG | 694 |
| AGAACGGTACCCGCTCTTACTGATAACTCCGCATG | 695 |
| AGAAGTTTTTTGTCATCATCGCTGATGTTTCCTAG | 696 |
| AGACATGAGAGATTAGCAAAAGCAACAAGGGCTGG | 697 |
| AGACATGGCGATAAGCTCTAAGACACGCAGATGAG | 698 |
| AGACGCACACCGATAGAGGAGAGATCTTACATACG | 699 |
| AGAGCACCAGACGTTTGCTCGCACCTACTTGTTTG | 700 |
| AGAGCAGCAAACCCATAAATCAGCATTCAATTTTG | 701 |
| AGAGTAATGCAAATCTCTTCATCATCATCCCCCAG | 702 |
| AGAGTTACAGTTTTTGGAGAAAGTCATGGAAAGGG | 703 |
| AGATCGGCCCCACTCCTGTTCTAACTTGTCATTCG | 704 |
| AGATGCTGATGTTGTAGTTTTTCCAACCCCTCCTG | 705 |
| AGATGGGATTGCACCCTGCTTCTTAATAAAACGTG | 706 |
| AGATTCGCTAGCCTAGTATGCCAAAGCTCCTCCGG | 707 |
| AGATTGGGAACTGACATCATTGGGGCTATTGTTAG | 708 |
| AGCAAACGCGTATCTAGGGAGAAAGTCACAAACCG | 709 |
| AGCAAAGCGGAGGTTTGCAATAGGCTTACCCTATG | 710 |
| AGCAAGTCATCAGTGGAGAGGCAAAGGTTGAAAAG | 711 |
| AGCAGCTTTTCCAGAGTTTGTTGCAACTCTTTTAG | 712 |
| AGCAGTTAATCCTTATGTCAACAACCTCAGCATAG | 713 |
| AGCCAGCTAAAACTAAAATTCCTACTCGTGGAAGG | 714 |
| AGCCATAATTCGTAACCCGAGGGTATAATTCGTTG | 715 |
| AGCCCCTCACTTACCAGCCTCATGCAACTTTCTAG | 716 |
| AGCCCCTCTTCCTAAATATCTTTCCAAATCCATAG | 717 |
| AGCCCCTTTATGGTGTGGATACCACACGTCCATTG | 718 |
| AGCCCTGCAGAAATAAACGCCTGCTTAAAGCTTTG | 719 |
| AGCGGTACTAATATGCTATGAGCGAGTTCCCTAAG | 720 |
| AGCGTACTAGGCATCTATTGGCTGAACTACCATGG | 721 |
| AGCGTTCACCATAGGTTCAATAGCGAGAACCATGG | 722 |

(continued)

| TAG SEQUENCE | SEQ ID NO: |
|---|---|
| AGCTTTTGGAGCTTTGTGAGAGATTATCAAGGATG | 723 |
| AGGAAAGTGTCGATGAAGAAATTTATGGCATTGCG | 724 |
| AGGAGTAGTAGTGTGGATGTTGTTGTTAGACACTG | 725 |
| AGGAGTCTTCACACTACCAATATTCTCCACAACTG | 726 |
| AGGCAGTTTTTGATCACGTTTATTGTAAGCCGTCG | 727 |
| AGGTACATCTTACGCCACCTCGTCTGTTAAGATTG | 728 |
| AGGTCACAGAGACTGCAACGTCATCACATGGATCG | 729 |
| AGGTCACGGAATTCGAAAACACCTTCATCAAGTGG | 730 |
| AGGTCCTGAAGGAGTTTTAGTTATTCCAGCAGGTG | 731 |
| AGGTTACAGCAGAAATTTCAGCAACAAGAATGATG | 732 |
| AGTAGGATAAGCCACGCAGTTGAAATAAAGAACAG | 733 |
| AGTATATATAGTAGCCAAATCCGGCATTTGTGCAG | 734 |
| AGTCACATAAAGTGGCCCACCGCCAAGAATGAAGG | 735 |
| AGTCGACTATACTTGGTGGGGATAGAGGTGCACAG | 736 |
| AGTGAAAACGTTGAAGCCGTCATTGTCCTGGTATG | 737 |
| AGTGAGCTTTTTGCCATACTTTTTCGAGAAGGTAG | 738 |
| AGTGCAACAGCAATCCACATCTTAGATGAGATTAG | 739 |
| AGTTACATTTGGGTACGGTTAGGGTCTCCGGTGTG | 740 |
| AGTTATGATCCATACCGTGTCCAAACCAGTACGGG | 741 |
| AGTTGTACCATATCCACGCTCAAGTGGCTCTACGG | 742 |
| AGTTTTTCTTAGACGAGGCGTGTCGACGCGCTTAG | 743 |
| ATAAAATTGGCAATATCATCCAACCTTGCTGCTAG | 744 |
| ATAAATGCGTCGCTTGGGTAGAATTCGCCAGTTCG | 745 |
| ATAAGTTAAGTCTGCTCAATACAGGGGTCTGTCCG | 746 |
| ATACACAAGCTGATCAATCTTCATGACTTGTTCGG | 747 |
| ATACCCAAGGCAAATGTGCGTAGACAACTTGTATG | 748 |
| ATACTATCGGATCAAAAAATAGGTACCCCAAGAGG | 749 |
| ATAGGATAGTCACAACGAGGCCCCAGACAATTCGG | 750 |
| ATAGTCATCGTCCATAACACGATCTAGTGAAACCG | 751 |
| ATAGTCTTTAGAGCCTCAGAATAGGCTGTGACGCG | 752 |
| ATATCAATTGCGTGCGGTCAATCATCTTCACTTCG | 753 |
| ATATGTCGCCGGCTTACTTACGAGTTCTTTTTAAG | 754 |
| ATATGTGCAGAACCCGCGACATATGACCTGAACCG | 755 |
| ATATTTCGTAAGCTCGTTCGGGACTTTGTATCGGG | 756 |
| ATCATAGCCTGACCTTATTAATTACGCTGCAGGTG | 757 |
| ATCCAGTATATGAGCTACCGAGTCGTTCTGATAGG | 758 |
| ATCCCGCCAGGGGATAAAAATGCGATGTTGACATG | 759 |
| ATCGACGCTACAAAAAACTCGTCGCCGTCAGTAGG | 760 |

(continued)

| TAG SEQUENCE | SEQ ID NO: |
|---|---|
| ATCGCAGGATGGTACAGCATCATACATGATGAGCG | 761 |
| ATCGCCGCATCCTATATGATACGCGCACTGTCTAG | 762 |
| ATCTCAACATCCTCAAAATAGTTGGCAGCCATTTG | 763 |
| ATCTCATCCAACTCTCCTTTCTGGTTTAAATAGGG | 764 |
| ATCTGAACCGCAGCCTAGACCGTTTGTAAAATATG | 765 |
| ATCTGCATGAACGGGAAAGGAGTTCGATGAGACTG | 766 |
| ATGAACCTTTCGGTTATTTAATACCCCTGAGCTAG | 767 |
| ATGATCGTTCCGCATTTTGAATTTACGGTCATGAG | 768 |
| ATGCCATATCTGTTATTTTTGGCTCATGCGGCTTG | 769 |
| ATGCTGAAAGATTTAACAGATGCAAAAGGCATACG | 770 |
| ATGGCCCCTGGAATCAATACATCATCAAACGCTTG | 771 |
| ATGGCGATCCTTTTTATACGGCATAAAAACCGCTG | 772 |
| ATGGCGGTTTCGGGTCCTGCACTATTCCTAATAAG | 773 |
| ATGGGTACGGCGACTACTGAATCGTTCTTTGAGAG | 774 |
| ATGGGTTACTGGGAGCTAATGACTTAAAACGCAGG | 775 |
| ATTAAGTTAGGGCTTTCAGCCCTAATTAATGTCCG | 776 |
| ATTAGGTTGTATCATGAAAACTGGATTGCTGGAAG | 777 |
| ATTAGTGGTGGGAATCAGCGAAGTTACAATGTGGG | 778 |
| ATTATGGCCCCCTTCCGAAATTTGACACTCCGCTG | 779 |
| ATTCACAGCGAGTTAGAAGCATTTTGTGTCGCCGG | 780 |
| ATTCCAAATGCCGTGTTTTCGCGCCGCTTATCTAG | 781 |
| ATTCTCCTAATGCCGTTCAATTCTATCCCTCTAAG | 782 |
| ATTCTTTTGTCGAGATTCCTGGTTTAATGTGCTTG | 783 |
| ATTGAGAGAGGAAGGTTTGAGAAACGAAATTAGCG | 784 |
| ATTGGAGGGCACTTACCTGGAGAGAAGGTTACAGG | 785 |
| ATTTCAGCAGTGTCGTTCCAGTTACCGTCCCCATG | 786 |
| ATTTCGATCTCTCAGTTTGATTCGGATGGTCAAGG | 787 |
| ATTTGGATGAAGTCGGCTTTATGGTGACACAAATG | 788 |
| ATTTGTGCAATGTAACGAGGTTGGCCAAACGAACG | 789 |
| ATTTTCGACATACGTTTGTATTGCGTGGGAAATAG | 790 |
| ATTTTTGATGCTGTGGGAGACATGGCTGATGAGCG | 791 |
| CAAAACCTAACACCTCCTCGCTTATGCTCGGAGGG | 792 |
| CAAACAACTTAACCTCAATTTCCCCGCACGTCGTG | 793 |
| CAAATCGCGCCTAGTTCCATATTATCACTACGACG | 794 |
| CAACGTCGCAAAAAACCAGCAAAAATTCTTAACAG | 795 |
| CAAGATAAAATGTCTCCTCTTTCATTTGCATCCCG | 796 |
| CAAGCATTGCAAATCATAGCCGACTGCTGCTCATG | 797 |
| CAAGGGCTGGTTTGGAGGCAATGGGAATAGAGTTG | 798 |

(continued)

| TAG SEQUENCE | SEQ ID NO: |
|---|---|
| CAATAACAGTCAGTGAAAAGGCATGGGAAGTTATG | 799 |
| CAATAGGACGGAACGCCATCCAATAACTCGGAAGG | 800 |
| CAATCACCATGGATACACTCCAAACAGCAAACG | 801 |
| CAATCTAGAACACGCTTATCAAACTTCGGCCCGCG | 802 |
| CAATGGCAAACTTAGAGCCTATCATGGGGTTAGAG | 803 |
| CAATTGTCGAGAATTCGTGCAGTACACCATCTATG | 804 |
| CACAGAAGAAGGAGACAGATGACTACATTAGTGGG | 805 |
| CACAGAGAGGTCGAAAAGGTATTTAGAAAGGCATG | 806 |
| CACGAGTGCTAAGATCTGAGCCGTTTACCAAAGAG | 807 |
| CACGCATTATACGTTTGTCATGTTTTCCAATAGTG | 808 |
| CACGCTGCACCATATCTCTTATTAGCCAGTCGGGG | 809 |
| CACGCTTTAAGCAGTTGTAAGAACGAACAGAAAGG | 810 |
| CACGTGAGCATGAGGTACTATGACTCATGACGCTG | 811 |
| CACTCGGGATAGTCAGCGATTTTCTGTGATCTCGG | 812 |
| CACTGTCTATACATGGACGACACTTTGCACATCAG | 813 |
| CAGAAGGCCCTCAACGTAAATCTGCTCCACATTTG | 814 |
| CAGAAGGGGGACTATGTTTTGCTAGATATGTCGCG | 815 |
| CAGAAGTGCGCTGCTTAAGAGCGATACCCCATAAG | 816 |
| CAGAATACTTAGCAGAGGCTGTTGAAGAGATTGCG | 817 |
| CAGACAACTCGACCCTTGATCAGGGAGTATATATG | 818 |
| CAGAGGTTATGTATAGCGAGAGCGATAGCGGTTAG | 819 |
| CAGATGAGAGTGCTCACATCGCTGTCTATAGGCTG | 820 |
| CAGCAAAGGTTTTTCCAGGAGATGTTGGAACTCTG | 821 |
| CAGCATGGCAACTATACACGTCTCACTTGTTCTCG | 822 |
| CAGCTTATCCACTTCTTTTTGAGAGCCAACCGTAG | 823 |
| CAGGAATTTTTGAGGGGAAAACTACTGGAGCTCCG | 824 |
| CAGGATGATAAACGGCACGGATTCATCAATAATTG | 825 |
| CAGGGTTCCAAAAACGATTTGATACAAAACGCCAG | 826 |
| CAGGGTTTTAGAACGCGCATTCGGGAGATACAGTG | 827 |
| CAGTATTCACGAAATGCTCCTCGCTAATAAGAAAG | 828 |
| CAGTTAAAATCTTTGAACCAAGCGCAATTGCTTCG | 829 |
| CATAACTCCATGTTGGACTTGGGAATCATCAACCG | 830 |
| CATAAGCGCTTGATTCATGGCTTTTAGGTTCTCCG | 831 |
| CATACTGACAGCACGCATGGCATATCTCCAGCATG | 832 |
| CATCAAAAACACCAGATGGAAGACCAGGATTTATG | 833 |
| CATCATCGACAGTTCGCAGCCCTATAACATGATAG | 834 |
| CATCTCCGGGTTATGAAAAGAGTTAGCACCTTTGG | 835 |
| CATGCGAACATAGATTGCGTTATAACCCACCTCTG | 836 |

(continued)

| TAG SEQUENCE | SEQ ID NO: |
|---|---|
| CATGGACTTATCCCCTGTCAAGCTAACAGTGGTTG | 837 |
| CATGGGAGGGGAATTTATAACTGAAGCTAAGTTTG | 838 |
| CATGTTTTGCAAACTAAACCTGGGTCTATAACTCG | 839 |
| CATTACATGGTATAGGTTCTACGGGACAATCCCAG | 840 |
| CATTCGTCTAGTTTTTTGAAGATTTTTTCCGCTGG | 841 |
| CCAACCAGTCTGTCAGCACACTATAAGCGCTGTCG | 842 |
| CCAACTCTATATGCCCAAAATGCCCTGGACACTCG | 843 |
| CCAAGAAACATTAGAGCTGCTGCTGAAAAGGCTAG | 844 |
| CCAATAGGGAAACTGATACTAACGTAGGAGCACGG | 845 |
| CCACAAATAAGGATAGCGATCACAGGCGGCAGAAG | 846 |
| CCACACGGTCCATTCTAGGATATAAAAGGGATTGG | 847 |
| CCACCATTTTCCCTAATCTCTTTAACTGCCTTTAG | 848 |
| CCAGAAAGGTACAGGGCCAATTAACACGTAATCGG | 849 |
| CCAGACACTGTGAGCGACAACCAACGCAGATTAGG | 850 |
| CCAGCGCCCGGTCGTGAAAAAATAATCATCTTGGG | 851 |
| CCAGCTGGCATTCGTTGGAGGTAATTCGTATCACG | 852 |
| CCAGTATGCGCGCTCATAGTGTCAATTCTCGCAGG | 853 |
| CCATAGAGAAGTGACCACCCATATAGCGAAGTATG | 854 |
| CCATAGGGGGAAACCTCCTATTGGTATGAACCTTG | 855 |
| CCATGCATTCTCTCTTGAGGGATGGACGAGCAAGG | 856 |
| CCATTAGATGAAACCGACTTCATTCCAGACTCAAG | 857 |
| CCCAACCCCTTATGAAGATGTCAATTTAAACGCTG | 858 |
| CCCAATAACCGCTTATATTAGGGGAGGCGTCACTG | 859 |
| CCCCAAGAGCATCAACTCGTACTGATAAGTACAAG | 860 |
| CCCCTCTCTCAGATCTGCGCTTAAGTTGTATTGTG | 861 |
| CCCGAAGGCATAATCAACATCCATTGTACATCCCG | 862 |
| CCCGCATGATACCAAGTTCACGTGGGGTTTTACAG | 863 |
| CCCTAAGATTCGACTAGTCGGGTTTGGGTCTATGG | 864 |
| CCCTACAAGGTCAAAATGTGGTGTTCGTTCTGCCG | 865 |
| CCCTACTTAACTGATCTGAAGTATTACGGTAACCG | 866 |
| CCCTGCAGCATATTTCTACCACATCTAGAGCCTAG | 867 |
| CCGAAAAACGGTTGACGAAATTACGTACCAATAAG | 868 |
| CCGAAGGGATTACACAGTATCACCGATAAGCCCTG | 869 |
| CCGAGGGTACGACCTTAATACGCCGTATATGGTCG | 870 |
| CCGATACCACGACGTCAAGCACAATACTGTCTAAG | 871 |
| CCGCAGATTATCGTTTACGATGCATCCATGGTCTG | 872 |
| CCGGTTCAACTGAATTATATTCCCCGTTGTTTACG | 873 |
| CCGTATTAAAATACCTTCCCATGACAGCGCAACGG | 874 |

(continued)

| TAG SEQUENCE | SEQ ID NO: |
|---|---|
| CCGTCTACATTCCCCATTATAGGCTACTCGGTGAG | 875 |
| CCGTTTTTGTGTGACGCTGGTCAGTACTTTTCCGG | 876 |
| CCTAACACTAGGGTCAAAACACACTTAATCACTGG | 877 |
| CCTCAGGCCAATTTTAGTGTGCCTGCAATCACCAG | 878 |
| CCTCCTATTGGGATACCTCCCGTCCATTAAGTTAG | 879 |
| CCTGAGCTAGTTAAACGTGATCAGACTTCGCGTCG | 880 |
| CCTGATCATGCTTTGTCAGCAGACCCAGAAGAATG | 881 |
| CCTGGCAAAATTGTAGGTTCGATTCTCCACACTTG | 882 |
| CCTTAACCATTGGCTCTCGAGATATCTAGAGATTG | 883 |
| CCTTTGGCTCACGCTAATTGAGTTACTGTAGGAAG | 884 |
| CCTTTTCTAGACAACCTTTTGCGACCTTGATAGGG | 885 |
| CCTTTTGTTAAGGATCAGCGGTCACCGCCAAATCG | 886 |
| CCTTTTTCGAATTGTCGCCTATAATACCCACAGGG | 887 |
| CGAAAATTGGGACGCCCTTCGCTAGCTAGGATGTG | 888 |
| CGACGTTTGTGTAACATGCGGGGATGGTAACATTG | 889 |
| CGAGCAAAGCGAGATGATGCATCCATTTTTGGTGG | 890 |
| CGAGCTGGACTAATCTTGAATTGGCGGCAACAGTG | 891 |
| CGAGTGGCACGAATCGCACGGATGTTTGGTTAAAG | 892 |
| CGATCAGCGTACTCTGAATGCCGTCAGCGTACTAG | 893 |
| CGATGAAAGACGTATCTATAGTTCGTGCAGAGGGG | 894 |
| CGATTGAACTCTTGCCTGGTTACTGTATGCCCCTG | 895 |
| CGCAAACAGGCCTGACATTTTAGACCCTGCAATAG | 896 |
| CGCATAACTCGAACCACAGTTACTATCAGTCGACG | 897 |
| CGCCAGTTCCGTTTAGTTTGTAGTGTATGACTACG | 898 |
| CGCCATGCGCCGGATCTGATAGTAGTCAATTAAGG | 899 |
| CGCGAAACCCATTATACCCCCTAAAAGATGGGATG | 900 |
| CGCGGGCTAAGTAGTAGGGTTCTAATGCTACTTTG | 901 |
| CGCGGGTGTCTTACGATATTCGGCTCAGTATTCAG | 902 |
| CGCGGTTGCTTGAAAACTTACAGAGATATCTTTCG | 903 |
| CGCGTTTTTGCTAGAGCAAGGCACCTACCATCATG | 904 |
| CGCTATAGGACTGAATCAGACCGCATTTGTCCTCG | 905 |
| CGCTTGATGCCGGAAATATCCTTGCCTGGTTAACG | 906 |
| CGGATAAGCTCTCTACTGCAGCCGATAATACATGG | 907 |
| CGGCATCGTCGCTGATTTCAACCGTTTCGATTTTG | 908 |
| CGGCTTTGTGTTTATTGTACATAGACGTTGTCCCG | 909 |
| CGGTCATGAACAATGAAAAATTCCTACTCGCAAAG | 910 |
| CGGTCTGGAAGCGTTAGCTGAATTCTTTTATCTGG | 911 |
| CGGTGTGTAAGCGTAACGATGTTGGTGTCGCTCTG | 912 |

(continued)

| TAG SEQUENCE | SEQ ID NO: |
|---|---|
| CGGTTCAAGAAAATACGCTGGAATTAAGCCAGAAG | 913 |
| CGGTTGAACCATGTTGATTTCCCTGCGTTTGTATG | 914 |
| CGGTTTAGATGGGACACCCTATCTCGTTTTCTACG | 915 |
| CGTAGAAGAATTGCTGGTATATGTACGCGTAATGG | 916 |
| CGTATCTCGCGTAGGTTAGACTGTTCCGCTATGGG | 917 |
| CGTCAGTAGAGCATAAAATAGAATGCAGGTGTGTG | 918 |
| CGTCCCCATCTGCTCCTGGATATATTGCATGTAAG | 919 |
| CGTGCTCTAATGCAATTTTTGTATGTACTTTTCCG | 920 |
| CGTTACATACTCAGCCATAGGCTTCGATAACAGCG | 921 |
| CGTTCTGCCAATTTAACAGCTTCCTGCCCCATTCG | 922 |
| CGTTGTCAGCTTCCTGCTTAAGGGCTTTTTCATAG | 923 |
| CGTTTGTATAGCCGACAAGCGCAATTTGAAGCACG | 924 |
| CTAAGCCGCCTCATATTTTGTCTCCTGAAGCAAGG | 925 |
| CTACAGAGGCAACAGGTTTTGGTTGTTCAGTTATG | 926 |
| CTACAGGAATGTCTGATATTGGGGAAATTTGGGAG | 927 |
| CTACCTAATTCCATTGACCGAAAAGACAGAAACAG | 928 |
| CTACGTACGTAGTCGTTGTGTACCGTAGCACTTAG | 929 |
| CTAGACCAGGTAAGATACTCATAGCACCGGAATAG | 930 |
| CTAGAGATTCGGACTTGAAATGCAGTTAGAGCTTG | 931 |
| CTAGGTGGCGAATTTCCAGGCGACGTTCCGAATAG | 932 |
| CTATAGGCTCACATGCGCGTCGATAAGGTCACAGG | 933 |
| CTATATGATTAGATCCTGCAGCCGTACTTCCGTCG | 934 |
| CTATTTTTCGAGTAACATGAACCGGCGCACACCGG | 935 |
| CTCAATCGCGCGTAACACCTGACACTCTGCTAATG | 936 |
| CTCACCTATCATTTGCTAAGGCAGTTAAAGAATGG | 937 |
| CTCAGAGCTTCAAATCTATCCTCTGGAATCTCTGG | 938 |
| CTCCAGTTTCCAAGGTAATTGATGGCCTTACAGTG | 939 |
| CTCCCCAAGGGCATGCTGTTCCTTCAAATTCATAG | 940 |
| CTCCTCGTTCATGATATCACAAGGTTTCCAGCCGG | 941 |
| CTCGACACCTGAATCACCGAAACAGGGTGGAAAAG | 942 |
| CTCGCCCGCTTTGCAAAAATATCTAATATCAATTG | 943 |
| CTCGGCTCTAACGGAAATCGTGAAGGAAGTGGTGG | 944 |
| CTCGGTTCCCTAATTACAGGCTACGGCCTAGTCCG | 945 |
| CTCTGCTGTAATCTCAGCTCCACTTGTTTCTAAGG | 946 |
| CTCTTGACAACTGGAGCGGATCGGACAAACTTCCG | 947 |
| CTGACATGAAAGAAGCACGGTTATAAGAATCATGG | 948 |
| CTGATAAGTCGTAGGAATGTCGCTTAATACGGATG | 949 |
| CTGATAGGCGCTAGCAAAATATGACTAATATTCGG | 950 |

(continued)

| TAG SEQUENCE | SEQ ID NO: |
|---|---|
| CTGCAGCTAAAAGAGTTGTTGAAGAGGTAGCAAAG | 951 |
| CTGCCATATCATTGGCAATGACCCCGATATTCAGG | 952 |
| CTGCCTTTAGCACACTTCCTCCAGTTGTAGTAACG | 953 |
| CTGCTGACCTAACATTTTCATCTTCAGGGACTTCG | 954 |
| CTGCTTTTTTTCGATCAAGCAGCTTTTTGACTTCG | 955 |
| CTGGCCGAGAGACTACCCCGTAGTGAAAGATGACG | 956 |
| CTGGGGATGAGTGATAATCAACGGACCAGAAAGGG | 957 |
| CTGGGTTTTGACTTACAGCACGTGAGTGGACTCTG | 958 |
| CTGGTTTTGCTTCCTGCAAGCCTTTATATAAAGAG | 959 |
| CTGTACGAATGCTAAAGGTGTTATAGTTTGACCCG | 960 |
| CTGTAGGAAACACCTAGCCGCTCAATCTTAAAAAG | 961 |
| CTGTCGCCTCATGAATTTTCAACCCTGTGGTTCCG | 962 |
| CTGTGTTACTGGTTCTCAAAAATGTTTGGCAGCTG | 963 |
| CTGTTACTATGGGTGTAACTCCGTAATCCCTTATG | 964 |
| CTTAAAATCGGTGATTTGCATGCCCGAATGTTTAG | 965 |
| CTTAATATCACCGCAGTAACTACATGCCCCGCTAG | 966 |
| CTTCAGCAACCTTTGGATTTTCATCCTCTCTTGCG | 967 |
| CTTCGCGTCGACGTAAACTGTACAAGAGATACCGG | 968 |
| CTTCGTGCTGTAACTAGGCAAGAAGCTTTTCTCCG | 969 |
| CTTCTAATCATCCCCTCAACAGCACTCTTTCCAAG | 970 |
| CTTGCACACACGAGTAACATTTGCCATGACCGACG | 971 |
| CTTGGAAGTGGGGAAAAGATACCAATGCCTTCTGG | 972 |
| CTTGTTCTCGTTCTGCGTACGCTATGAACTATCCG | 973 |
| CTTTAACTCTGCATTCAGCTGTCAAGTTTTTTGCG | 974 |
| CTTTAACTGGTGGAGATAGGGAAGTTCAGAGAACG | 975 |
| CTTTGACGGGATAAACTGGCTTTTGTAGGCGTTGG | 976 |
| CTTTGATGGGCAAGCGAGCACATAGATATGCGTTG | 977 |
| CTTTGCTGAGGCATAGAAGTATTGGAAGAGTTTTG | 978 |
| CTTTTTATGCGTCGCGTCGGGTTAGCGAAAATTGG | 979 |
| GAAAGTCCCATACGACAAGTTGAGACCGAGGGTAG | 980 |
| GAAATCAACTTCGCCTGCAACGGCTGCATCTATAG | 981 |
| GAAATCAGATCAGTTCTACATTCGGTGGGAGCCCG | 982 |
| GAAATTAGCATCATAGCAAGTGGAGGAATCAGATG | 983 |
| GAACACAGTAGGGGTGATAGGGTCAACTAGTCACG | 984 |
| GAACCCCACTAGTCACAGTTGAAGTATCTGCATGG | 985 |
| GAACGACATTACTGGTGTTAGTTGCATCCCGCCAG | 986 |
| GAACGGCTCCCAAGGTTCGTAAATAAGCGACGAGG | 987 |
| GAACTTATTCTCTCCAACGCTAGAGGGTATTCTTG | 988 |

(continued)

| TAG SEQUENCE | SEQ ID NO: |
|---|---|
| GAAGATAACTCATAAGTGCCTCCCTCGGTAATTTG | 989 |
| GAAGATTCCAGGCAGATTTCTCAGGAATTCAGTCG | 990 |
| GAAGCTAGACTCATGTCACACGCGGAGAGATCACG | 991 |
| GAATGACAGTGGAAAGCTGTGTGTTGATTTCATGG | 992 |
| GACAACTCTAACGCCAACTGGTGGCTAAATTCTTG | 993 |
| GACAATTGTCTGCAACAAGGGCCACAATCGCAATG | 994 |
| GACATTTCTTCAGCGATATGTGTTGAAGACTCATG | 995 |
| GACTTCAGCTGACTTGGCGACAGTTCATCATTAAG | 996 |
| GAGACAGAGCAGATATTCCTAAATCCACAGAAGAG | 997 |
| GAGACTGTCGCATGATGATTTAGAGCGATGTATCG | 998 |
| GAGAGACTCCACTGAGCACTATGGGGCATACATCG | 999 |
| GAGATAACGCACCTGACCTATCCTCCAAATGAAAG | 1000 |
| GAGATACCGAGGTCACAATCATGATACCATTTACG | 1001 |
| GAGCCTACGACACTATTCACAACGCTATCGAAGTG | 1002 |
| GAGCGCTACACGGTTGAGAAGTTCACTGGGTTTTG | 1003 |
| GAGGATACCGAATTCGGGTCAACAACGCCCAATAG | 1004 |
| GAGGATTTTTATCTTGGATGAGTGTTGATGGGATG | 1005 |
| GAGGCTTCTATGTGCATTTTAGCGGTCTCAAGTCG | 1006 |
| GAGGTAGCCGAGTATGACACACCACAGCAGTTAAG | 1007 |
| GAGTACAGAGTTGGGGGGTTAAAGCTATAGAGACAG | 1008 |
| GAGTTTACCATGTAACGTCAACGCGTGTCACTCGG | 1009 |
| GATACACGCAAAATCCCCAGAGGCAGTTATAAGGG | 1010 |
| GATAGGATGCGACTGCGTATCATATAGGCTGCACG | 1011 |
| GATAGTCCATTCGGCTGCCACTTAGTTCAATAGGG | 1012 |
| GATCGCGACATATCAGCATACATGGCATACTGACG | 1013 |
| GATCTGTAAGTATGGGATTAGGGATGTTCTGCCAG | 1014 |
| GATGAAGAGGCAGCTAAAAAAACAGTTGATGCAAG | 1015 |
| GATGAGACTTCTACATGTCCGATGTTTTTGTGCTG | 1016 |
| GATGTCACATCGTTTCAAGCGTCTGCGCATAGTTG | 1017 |
| GATTGAAAACGTTCAATTTGAAGACCTGTCGCCTG | 1018 |
| GATTGCAGCGATGACTATATCTGAGCACCTGTGAG | 1019 |
| GATTTCATGAATGCGATTTCTGATATGGCGGCGGG | 1020 |
| GATTTTGAGAGGAGAGAAACTGCCAACTGACTGCG | 1021 |
| GCAACAACCTCATCTATACTGTGAATAGTCCCTCG | 1022 |
| GCAATGGGGGTCTTTAGAAACCCACCAGAACCATG | 1023 |
| GCAATTTTCATTGTTTATCCCCCCGTCTAATCAAG | 1024 |
| GCACGTCGTAATGACAGTAAGTATGGTCGTTCCCG | 1025 |
| GCAGAACGTCTGAAGTGGCGTACGTAATTCTCCGG | 1026 |

(continued)

| TAG SEQUENCE | SEQ ID NO: |
|---|---|
| GCAGAGATGGATGGATTCGATGCAAGGGGAGATGG | 1027 |
| GCAGCAATCAATGTCGTCGGAAGATCCTGAATAAG | 1028 |
| GCAGGAATTGAGAAATATGTCCCTCCATCAAAAAG | 1029 |
| GCATAGTTACTTCTAAGTGCGATTACCTGCACTCG | 1030 |
| GCATCGGGCAATACATCTTCACGGACAAGATAAAG | 1031 |
| GCATCTATACACTCCGGAATGGTGCGGTAAGCAAG | 1032 |
| GCATGCAAGTTACAAACCCATCCATCGACCCATTG | 1033 |
| GCCACTATACCACGTTGTGTGTAGGTTCATCGCAG | 1034 |
| GCCACTTCACACAAGAACACAAATTTGGAGTATTG | 1035 |
| GCCATTATATGCGTTGAGGTTAGTTCAAGCAATAG | 1036 |
| GCCCAACCCATTGTATAGTATACTGCACCGCCATG | 1037 |
| GCCTAGGAAGTCTTATCAACAACACCCCGCATAAG | 1038 |
| GCCTGTCCCCCTACTTAACGTTGTTACTGCGTTAG | 1039 |
| GCGAGCTATGTCTCTGCACGAACTTTAAAACTCAG | 1040 |
| GCGCGTCACCATTGTCACAAAAAAAGGAGAAATCG | 1041 |
| GCGGTACCCTCAGTACAAGAGGCAAACCATAAGAG | 1042 |
| GCGTTACACGTAACAGCTCGACTGAACGCTAACAG | 1043 |
| GCTAAAGGAGACTCCGGTTTAAACGTCATCGCAAG | 1044 |
| GCTATGAGCGCACAGTCTCGTCATATAACGATCAG | 1045 |
| GCTATTGCAGCAAAGAGAACAGACGCTTTAACTGG | 1046 |
| GCTCGGAGGTGTAAATTAGCAATATTAGGGGAGTG | 1047 |
| GCTCTCGTACCAGTCCAAGTCAGTAGCGTCTTTGG | 1048 |
| GCTGACGCTGATAGTTTTATTTAACGTCCGCGAGG | 1049 |
| GCTGAGAGAGTTAGCAGAGCAGCTGCAGAATACTG | 1050 |
| GCTGATCGTATGTATGGTCTATGGCCCCTACAAGG | 1051 |
| GGAACACCTCTCGTTATTATGTATCCAGATTCTCG | 1052 |
| GGAAGGAATCGAGAATAGGGTTAAAAGACATGAGG | 1053 |
| GGAATATGGGGTCAAGACACCTAGCTAGCCCAAGG | 1054 |
| GGAGAATTTCTTTTTCATCCGGATGTCCTTGCTGG | 1055 |
| GGAGCCACGACCTATATCAGCCGACGATGATACTG | 1056 |
| GGAGTGGGGTGTACTTCCGGGAGATATGATCGTTG | 1057 |
| GGATAAGATTGTTGAGTGGGCTTTAAAGAAAGCGG | 1058 |
| GGATACCACACCTGAGATCCCCGTAATAGGATAGG | 1059 |
| GGATGAGATGGGAAGATTCTATATGTATATGCCCG | 1060 |
| GGATGTGTAGGGGCTGAGTTAAAGGCAATCTGCAG | 1061 |
| GGATTTAATGCCAGTCCAAGCTCTCTTCCACATTG | 1062 |
| GGCAAAATTAGATGAAACATTGACCATGCTGAAAG | 1063 |
| GGCACTCTCTCACAGCCAATAACTTCAACAACTTG | 1064 |

(continued)

| TAG SEQUENCE | SEQ ID NO: |
|---|---|
| GGCATGAAATACAGACTGAGGGTACCTTGGACAGG | 1065 |
| GGCCCCATGGTTGTCGGTAACGAAACGATAATTCG | 1066 |
| GGCCCGAAAATTATGATCGCCGGACATTTGGATGG | 1067 |
| GGCCGAAGCAGACTTAATCACCCCTCTCAGAATAG | 1068 |
| GGCGCAAATAGCGCTGAATCGCTTCTTTAAAGGCG | 1069 |
| GGCGTCACTTATAACCACATCCACCTTTTTTTCAG | 1070 |
| GGCTCAGCGTTATTTGATCACACTCGGATAAGTCG | 1071 |
| GGCTCTACGACAAACTTACCAAATTCGGCATCGTG | 1072 |
| GGCTTTTTGCAGAATTCGAATAATGATTTGTAGCG | 1073 |
| GGGAAACTAGTCAATCGTCTTTGCGAAGTCCGAGG | 1074 |
| GGGATGATGTATGAAGCACGAATTAAGGTTTTTTG | 1075 |
| GGGGAGATGTTAAGATAATTGGGGCCGCAAACAGG | 1076 |
| GGGGTTAGAAGGAAAGCCAGTAACCTTAAACGATG | 1077 |
| GGTAAGCAACATGTTCGGCGCCGTTTTGAAAACAG | 1078 |
| GGTATTCTTACAACGCGTATGGTCGTGTGGAAGGG | 1079 |
| GGTGGCTTGATTTAACTGAATCAGGCCCTAACCAG | 1080 |
| GGTGGGTGCTAACTCTTTAATAGCCTTCAGTGACG | 1081 |
| GGTTAAGAAGTTTATTGGAGAGGGGGCTCCGTTAG | 1082 |
| GGTTATCCATGACGAGTGAATAATCTTACCGCAGG | 1083 |
| GGTTGTTTTGTGATTGTTTGAGATGCTGAGTGCTG | 1084 |
| GGTTTCCCAGTTGTTAAAAATGGTGGTTTTGGATG | 1085 |
| GGTTTTCCCTTTCAAATCCTGCAAGAAAGCTTGAG | 1086 |
| GTAAAATATGCCCTACCAGATGACTAATGTTAGCG | 1087 |
| GTACGTGTCTGATGTACCAGCGTGCAACTAGAGGG | 1088 |
| GTATATGCGAGCACAGGATGCTCACTACGTGCATG | 1089 |
| GTATCGGCGAACACGAAATCCTCTACTCTTGACAG | 1090 |
| GTATTCAGTGGCATGAAGCGGTTCATCATCTTCCG | 1091 |
| GTCAACTAGTAGACATCCAACCTGACTAATTCGAG | 1092 |
| GTCAAGCGTCCACGATCACCGTACATCTTAGTCGG | 1093 |
| GTCCTTGGTTCTAGACCCCATTCCACACAGAGAGG | 1094 |
| GTCGCTACAACTGCGCAGTCAGTAGTTATCATGGG | 1095 |
| GTCTACTCGGCAATGGAGCGGCTATGATTCAGATG | 1096 |
| GTGAGTAAATTTTGTCGAGCTCTTTCCATGCATTG | 1097 |
| GTGCACTTACACCTGTTGCGGTCATCACGCATTAG | 1098 |
| GTGCATATTGCAGCTGAGCCAGCTCAATTTGAAGG | 1099 |
| GTGCGAAAAAATCGCTTTAATGGTGGGCTCAGCTG | 1100 |
| GTGGACTCTGAGGTGTGAAGTCGATTCCACTGACG | 1101 |
| GTGGATGGTTCTCTCCCAGATGGTAGCAGGCTAAG | 1102 |

(continued)

| TAG SEQUENCE | SEQ ID NO: |
|---|---|
| GTGGCTGTTTTGGACGCTGATATAGCAATGGCAAG | 1103 |
| GTGTCGATCCGAGAACATCACTCTAATGACGAGTG | 1104 |
| GTGTGCAAGTGAAGATGTACCATCAACCTGACTCG | 1105 |
| GTGTTCTGGGGATTATTGCGGTTGGTTACCTTACG | 1106 |
| GTTAATTTCACTGCAAATGCCCCAGTGACCGTATG | 1107 |
| GTTACAGGATGACAGTACAGTTGACAGACATGGCG | 1108 |
| GTTACTCTATGAGACGAAGATTAACTCCAGAGGTG | 1109 |
| GTTAGGTTCAGCCTCATTCCCTAAGAATCCAACTG | 1110 |
| GTTATAAGGAGGTTGAATGCTGAACCAATGAACAG | 1111 |
| GTTCACAGAGCATCCTTATACAGTACGCAGCGACG | 1112 |
| GTTCATTGAGAGGGCGTTCCCAACATATACGGTTG | 1113 |
| GTTCCGCTTCTGTCAAATCGCATATCATTACTTTG | 1114 |
| GTTCGACATCGGAATCGTTGCATTTTTTGATACGG | 1115 |
| GTTGTAACATCTTCCACAACGCCTTCAATTGTCGG | 1116 |
| GTTGTGCTCACGCGTGCTTGATTGCTATAGTTACG | 1117 |
| GTTGTTTACCTTGTAGATCGACTTCACATCAGCGG | 1118 |
| GTTGTTTAGGGATGCCAATATCTATAACGTCGAAG | 1119 |
| GTTTGATCTGCGAAGCATAGTGATAGAAAAGCCGG | 1120 |
| GTTTGCAGGGTTTGGATTGCCTACTCAATGGGGTG | 1121 |
| GTTTTTGGAATTTCTGCGTGAAGCATGTCCCAAGG | 1122 |
| GTTTTTTGCGTGATATAAGGCGATACCACCACTTG | 1123 |
| TAAAACTCATACTCGAAGGTGGGGCACGGACATAG | 1124 |
| TAAACCAATGAGAGAGCCTCACTTAGTTACAGTTG | 1125 |
| TAAACCGTAGGCTGGGGATATTGGGTTCCAAAACG | 1126 |
| TAAAGAACACACACTCCCCATTGCGGTCGCTACAG | 1127 |
| TAAAGAATGTGGAACATTCATGGGAACTGGTGAAG | 1128 |
| TAAAGCCACATCATATACGTAAAGAGGTGTACCAG | 1129 |
| TAAAGCTTTTAGCACGCTCACGTATTAAAGCCACG | 1130 |
| TAACGATCACGGCAGTGTAGATCAGAGCATCGGAG | 1131 |
| TAACGGAGGTTAACTTCCCTAATCCTTCCGACTTG | 1132 |
| TAACTTGCTAATATGCTCTGCAAATCCAATTCCCG | 1133 |
| TAAGCATCCAGCAATAAAGCCTCCTTCAAACCAAG | 1134 |
| TAAGCCGTCAGCATCGGGATATCATCTGCTTCAAG | 1135 |
| TAAGGCTATAGCTTTAGGAGCAGATGCTGTCTATG | 1136 |
| TAAGTTGAAGTTTTTCGGAGACGGTTATGAGAAGG | 1137 |
| TAATACTGGGTCACAAGATTAGATTCCAGCTGTGG | 1138 |
| TAATCACTGTATTTGTTAATCATGGCTAGGCGGGG | 1139 |
| TAATGGAATAGCTATCGCGATAGCATCTGGAAAAG | 1140 |

(continued)

| TAG SEQUENCE | SEQ ID NO: |
|---|---|
| TAATTCGTTACCTAGACCAACGTCGCTTAATCGGG | 1141 |
| TACCAAAGAGCGCAACGTATCTAGGATTGAGCAGG | 1142 |
| TACCCATTTCACCAAAATCTCTACCAACCCTATTG | 1143 |
| TACCCCAATAATGATTGCCCATATGTCTTATGGAG | 1144 |
| TACCTTAAACTGCGCTGGTAACTTGGATCGTGTAG | 1145 |
| TACTTGTTTTACATTTGAACCACCCCCTTTTGTTG | 1146 |
| TACTTTTCCGATTTCGGGCGTTGTTAAATCAATCG | 1147 |
| TAGATAACGATGCTCCATGTTAGTGAATGCGAGTG | 1148 |
| TAGCAAACCCATAGTTCTGCAGTAGATTCACAGCG | 1149 |
| TAGCATCCTGACAAGATGACTAGCTGATTGCAGCG | 1150 |
| TAGCCCAAGAAATCGTATAGTGAACATACTAGGCG | 1151 |
| TAGCGGATTTGGTTAGGTATTGACTTGTTTTTCGG | 1152 |
| TAGCGGTTAAGCCAGAGGTTTTATTGACGGATGAG | 1153 |
| TAGCTGATATTCTACACGAGAACGAGGCACGACTG | 1154 |
| TAGCTTCGTTTGCCACCGTAAAATCGTAACGATAG | 1155 |
| TAGTATCATCGTCGGCTGATATAGGTCGTGGCTCC | 1156 |
| TAGTTGTATGGTTTCAGATGAGGGAACGTGTAGGG | 1157 |
| TATAAGCCTGGGGACCGACATGGGAATAACCTGGG | 1158 |
| TATACGTAGTCTGCTCTGGGTACTCGAACCGGGTG | 1159 |
| TATAGTTACCAAGTACTATGGGTTGGTGGAAGCCG | 1160 |
| TATATCGGCACCTCTCGCTAGTGTCTCGCTCAAGG | 1161 |
| TATATGGTCATTGGTCACCCGAGTTACGATCAAAG | 1162 |
| TATATGTAGCGGCGTCAGCCCTGTTCCGTTTTTGG | 1163 |
| TATCCTTAGCCCAAAGGTGTGGAAAATCTTTAACG | 1164 |
| TATCGAAGTATCCCAAGTGACTCGAAGTATAGCTG | 1165 |
| TATCGAGCGCTTAGATGGCTATATGGTCTACTAGG | 1166 |
| TATCTGCTATCAATGTAGAGGATCGTGCATTACCG | 1167 |
| TATGAATGTCTTCTTCCATGCCGACGTACTGATAG | 1168 |
| TATGCCCCTGTGTTATTGCAGCGTCTCGATTAGGG | 1169 |
| TATGGTGGCCCCATGGTTAAGCGCTATATTTCGTG | 1170 |
| TATGTATAGAGTGCCGGGAAGTGAAAAATCTTTGG | 1171 |
| TATGTGTCGACTCACACAAGCACGGAGGACTTCGG | 1172 |
| TATTGCCCAAAGATAATGTCCCACGTTATCATCTG | 1173 |
| TCAAAACGAATACACTCCATGTAGTAATTGCGCGG | 1174 |
| TCAAACCAACATAATGTCTCTCCAACCTCAGGAAG | 1175 |
| TCAATTAAGAAAGACCGATCCAACGAGTGGTTCTG | 1176 |
| TCACAAACCCAAGCGCTATGGTTCTATTCCCCAAG | 1177 |
| TCACGAAGACGAGACCTCATAGACGAAGCGAGGAG | 1178 |

(continued)

| TAG SEQUENCE | SEQ ID NO: |
|---|---|
| TCACTCGATCTGAATAACGCACACTAGACTAATTG | 1179 |
| TCACTTCCATAAACATATTTTGCCTTTAACCCCAG | 1180 |
| TCAGAAGAGCTAAACCAGAAAAACTTGAGGAAGTG | 1181 |
| TCAGCGGCATAACCCTTTTAGAGCGTTACGAGCTG | 1182 |
| TCAGGTGCTTGTAGGCTCATGATAGGGGTAATGCG | 1183 |
| TCAGTCGACATGGTGTAACCTGATGCGAAGACTCG | 1184 |
| TCAGTCGTGTCAAGCGCGTGTCATACGATTACAAG | 1185 |
| TCATCAACCTTAACTCCCTCTGGGTTCATTGGGAG | 1186 |
| TCATGTTAGGAAGGCAGCTGCATTTGGAACACCTG | 1187 |
| TCATTGCGACTGATGAGAATGCTTTGCTCGCATAG | 1188 |
| TCCAAATCTTATACAACCAACCTCTTTTAGCAGGG | 1189 |
| TCCAATAGTGTACCGATAGGGGAATGACTTTCGCG | 1190 |
| TCCACATTTATCTGCGACCTGTTTCGTAAACGATG | 1191 |
| TCCACGATATAGGTACATTGGACGCTTACAGGATG | 1192 |
| TCCAGAGGTCAGGACAGAACCATTGAGAAGCGGAG | 1193 |
| TCCCAAACTCAGAATTGTTGGATTCAGCCATTGAG | 1194 |
| TCCCACCAGAGAAATTGAAGGATATTGTTGAAGCG | 1195 |
| TCCCATCCGCATCCGGAACAATATGCTTAGTCACG | 1196 |
| TCCCGATGAATCGAAGCTTAACAACCATTACATGG | 1 197 |
| TCCCTTGCTAACATGTGTATTTTTCTCTGCTCCAG | 1198 |
| TCCGTTCATTTTCTTCCTAACGGTCCGTAGAAGAG | 1199 |
| TCCTCCGGATACGACATCTAAAGAAAGTCCCTCTG | 1200 |
| TCCTTGCTGCTTTTCTTTACGGACTTCTGAAATCG | 1201 |
| TCGATTAGGGGGAAACCTTGTCACCGTCAGCTTAG | 1202 |
| TCGCTCAAGTTGTCTCCTGGTCTAGTCAGGTGCTG | 1203 |
| TCGCTGATTGCAGATGTTTGCCATTAGAGCACCAG | 1204 |
| TCGGTACGCGTTTTGGTAGTGAATACTAGTAGAAG | 1205 |
| TCGTATGGAGTGAGAAGTCATAAGGTGAAAAAACG | 1206 |
| TCGTCAGAGGAGTAGAAACGGAATCCTTTGACGGG | 1207 |
| TCGTCGCGAAAGTACTCATACGAGTAAGTTTTCGG | 1208 |
| TCGTGTAGCATGTTGTGGCACCGTGATCCAGTATG | 1209 |
| TCTAAGATCTCTCGCTACCGCTTTTATAAGACGGG | 1210 |
| TCTACTGTTCCGGCTACTGTTGTTATTTTTGGTGG | 1211 |
| TCTATCGCGTCTTTTTACTGGTTTCGAACCTTCTG | 1212 |
| TCTATCTCTCTGGAGGACAACAGCAGAGGTTAG | 1213 |
| TCTATTGAACGAGCGTGGTCTATATCCCAATAACG | 1214 |
| TCTCCGCTCATTGCTCCCCTATATTTAAAAATCAG | 1215 |
| TCTCTTCCTGGAGCCGATTGGAAATGTGACAGGTG | 1216 |

(continued)

| TAG SEQUENCE | SEQ ID NO: |
|---|---|
| TCTGAAATCATTTCCGCTGTTTTAAGCGCAGTTCG | 1217 |
| TCTGGAAAAGGAGGTACTGGAAAGACAACGATATG | 1218 |
| TCTGGGCTACTATCTAACGAGCCTGGTTGACTATG | 1219 |
| TCTGTACCTTGGCACTCCATCTGGTAAGTCACTTG | 1220 |
| TCTGTCTGCAAGTTCACACATCCACATGAACCTTG | 1221 |
| TCTTAAAAAGAGACGTGCGCGTTGGTGATCGCTCG | 1222 |
| TCTTACAAAAGCTTGGTAGATAAACAGCAGCTTTG | 1223 |
| TCTTATGGAGCTTTGTCTTTAAACGCTCACCTATG | 1224 |
| TCTTGACCAACACCATGTCCGACATACTCCCTAAG | 1225 |
| TCTTTGAGAGTCCGCTATCTTGGGTACCGAACTGG | 1226 |
| TGAAAGCATAGATGTTCCTTGGAGAGGTTTCCCAG | 1227 |
| TGAAAGGTTCTGCAATAGAGATTAAAATAGGGCAG | 1228 |
| TGAAGATCGAAACCAAACTTTGAATCTTCCTGGCG | 1229 |
| TGACCGTATGCTCCGATGCGTACTTGATTAAGGCG | 1230 |
| TGACTTTATGCGCCGGAAGGCTTTTTTCGTCTTTG | 1231 |
| TGAGAATTTGGAGGATATCAGTTGCACAGGTGTTG | 1232 |
| TGAGAGAATATTGAAAAAGGCTGGTGCTGAGAGAG | 1233 |
| TGAGTGAACGTATGGCATCATCTGGAAGATAGTCG | 1234 |
| TGAGTTTGTAGGGTCGATACCAACGATAAATGCGG | 1235 |
| TGATCATCCGTAATGTCTGGGAGATGCCTCTCCAG | 1236 |
| TGATCATTCCACTTTGACGACGTGAATTCGAGGGG | 1237 |
| TGATCCACACTGACGAATCATGTACTCACTCGATG | 1238 |
| TGATCGACTGGGACACCTGGTTCGCATAGTCTTTG | 1239 |
| TGATCGCTCTATTCGCTCTGAAACAACACCCCGTG | 1240 |
| TGATTACCATTCTACAGCAGATCCCGTCTACTCGG | 1241 |
| TGATTCACTCTGCGTCAGTAATAAATTGGTTTCGG | 1242 |
| TGCAATTAGGGAGTTAAGGGACTATGTAACAATGG | 1243 |
| TGCACATCATAGTGCGACGTTGATCCAGATAGACG | 1244 |
| TGCACCCCTTAAGTCGATCCCGGATTACTACAGGG | 1245 |
| TGCACTAGGATCAGTCGCAGACCTACTGAGGAGAG | 1246 |
| TGCATCCCTAACATCTGCCTCTTCACTCAAAACTG | 1247 |
| TGCATGTAACGCCCACCACATGCTTAAATTATACG | 1248 |
| TGCCTAACTTCGTCGTAAAGTCGCCGGTAGCAGTG | 1249 |
| TGCCTTCTGAAAGAGACGTTATTGTTGAAGCAAGG | 1250 |
| TGCGTAATCAACGCCGCAACTTTACGTCGGATTAG | 1251 |
| TGCGTTTTCATCCGTCACGCTTTATATATTCTGTG | 1252 |
| TGCTACTCCACTTATTGCCACTGCATTAGCTGTTG | 1253 |
| TGCTACTTTACCACGCCTGCACTATAATGGACCCG | 1254 |

(continued)

| TAG SEQUENCE | SEQ ID NO: |
|---|---|
| TGCTCCAGCTATTGCTGTTGGAATAGCAACAAGTG | 1255 |
| TGCTCGGTTTTGTGAATTGAACATCGAACTTATTG | 1256 |
| TGCTGGAGAGGTAGCTACTGGAAAAACCACCCTTG | 1257 |
| TGCTTCAGCTGCTTCTTCTTTACGCAAACTGACCG | 1258 |
| TGGAAAGACATTATTAGCTAAAGCTGTTGCTACAG | 1259 |
| TGGAAGATGTTATACTGGATTGTGTGCTTGGGGAG | 1260 |
| TGGACACTCTCCAATCCTTCCTCCACATGTTTTGG | 1261 |
| TGGAGAATCCTCAAAAGGCAATGGAATATGGGATG | 1262 |
| TGGCATATTCTGGCTGGATTAACAGAGGACATGAG | 1263 |
| TGGCATTGCGCAATCGCGTGTGAATGTGAGTAAAG | 1264 |
| TGGCTATTGCCGCAGTAGATCAAAGATTGAGAGAG | 1265 |
| TGGCTGAGCTTCCAGTTGCACCATTTGAGAGAATG | 1266 |
| TGGGAAATATTCGACAAACGTTCACCTGGTTTTGG | 1267 |
| TGGTTGCTCTTGGCTGTAGAGTTTGTGGAAGATGG | 1268 |
| TGGTTTAGGAGTAGGGGGTTTAGCTTTAGCTTTGG | 1269 |
| TGTAGATATGGAGGATACTCCAATCTAACATCCCG | 1270 |
| TGTCCCAAGCTATTTTAAAGAGCAAAATTCCCCCG | 1271 |
| TGTCGCTCTAGTGTGACTTTTCCACCTCGCATCTG | 1272 |
| TGTGAGCATTTCAGTACGAGTGATGCAGATAAACG | 1273 |
| TGTGGACAGGAGCCAATACTAGTTGGTGCACTTAG | 1274 |
| TGTGGTTCCGGTTGCGTATAGATCATGATTCTTTG | 1275 |
| TGTGTAAATGAAAGCATCTGACTCAACAGGCATCG | 1276 |
| TGTTAAAGGGGAATTTTTAATGATAGCCGCGATGG | 1277 |
| TGTTCTTTTACCATGGTGTAGAATGGAAAAACAGG | 1278 |
| TGTTGACATCCGCAACAATGTACCTTATATCGGCG | 1279 |
| TGTTGCCCTGACACACAATTTTTACTTGGGGCACG | 1280 |
| TGTTGGAGAGGTTAGAGGTGAGGAGGCGAAGATAG | 1281 |
| TGTTTCCTACCGGATATGTCCATGCAGAGTCACCG | 1282 |
| TGTTTTTCGCAAATCATCCCTCATTCCCGAAGGCG | 1283 |
| TTAAAAGCTCTTCAACATTCTCCACACCAACTCCG | 1284 |
| TTAACATAGACTGCCACACTTCGTATCATTTAGCG | 1285 |
| TTAAGCTTATCACGGGAATGCCAGTCTTTTCCTTG | 1286 |
| TTAATGCTCACGCATACATCTTTCGCCGAAGGGAG | 1287 |
| TTAATGTCTTCCACTTCTGTGCTTAGCTGGTGGAG | 1288 |
| TTACAGGATACTATGGACAGGTTCAGAATCCTCGG | 1289 |
| TTACCGCAGGGGTCAAATAACATAGCATGCGAACG | 1290 |
| TTACCTCAACCCTTCCAGTGTCTAAGGTTTTTAAG | 1291 |
| TTACCTTACAGTGCGCAGATTGGGATAATCGATTG | 1292 |

(continued)

| TAG SEQUENCE | SEQ ID NO: |
|---|---|
| TTAGATTAGACGAGAACGGAGAATTTAACCCCTGG | 1293 |
| TTAGCACCGATATCAATACTGATGATGTCACCGTG | 1294 |
| TTAGCTGTTGCTTCAAATGCCAATCTTACCTCAAG | 1295 |
| TTAGCTTTGGCTATGCAAGACACCATAAAAAACTG | 1296 |
| TTAGGCCATTGGGTTAAAGTTAAAGGGGCTGAAGG | 1297 |
| TTAGTCGGACGTGACTCAATTTTTGACAGGTTTAG | 1298 |
| TTAGTGAGTTGCCATACCGCGAGGTTCGCTGATTG | 1299 |
| TTATAGATGGATATAAAGGAGGGACAGGGGCAGCG | 1300 |
| TTATCATCTGGTCAACGATGAGGTGGGTTGTTTTG | 1301 |
| TTATCCCTTATTAGAAAAAGTGGCAAAAACAGGCG | 1302 |
| TTATGAGTAGGGATGAGCATAAACCAACAACTCTG | 1303 |
| TTATTGGAACCTCTGGGACATTAACAGAGACAACG | 1304 |
| TTCAACTACAAGTGTAAATGTACGAGCGCCGAGAG | 1305 |
| TTCCAAAACTATCCTCCATCTTAGGAATTGCAAGG | 1306 |
| TTCCAAATCGATAGATACCAGGGCAGTGTTCTGGG | 1307 |
| TTCCACATTCGTAAATAACTCCATGAGCCCCTCTG | 1308 |
| TTCCCTCTTTCTCCGCTTATGGATGAAAGGACAGG | 1309 |
| TTCGAAGGCGTACTAAGCATCTCTAACTCGTACTG | 1310 |
| TTCGTCTTTATATTTATGGATTCCGGCGGAAAAGG | 1311 |
| TTCTCCCGTAGTTCCATGATCTGTTGAAAGAGCTG | 1312 |
| TTCTGACCATACATTGGGAATACTCGCCCCAGTAG | 1313 |
| TTCTGATAGATCCCGCGTCAGGCATATAATAGGCG | 1314 |
| TTCTTTACCGTAAAGCTTTTCTCTCGCTTCAACGG | 1315 |
| TTGAAGCACACCGTTTTTCTTTCTTCTTTCACGGG | 1316 |
| TTGAAGCTAACTTCATATAAGCTTGAGAAGCTGGG | 1317 |
| TTGATAGAATCATAGAAGTCCCAGCTCCTGATGAG | 1318 |
| TTGATTCAGGTGGCACACTAATCTGCCTAAAATCG | 1319 |
| TTGCAGAATGTCGCGTAATGGCTTAGCAGTCATCG | 1320 |
| TTGCCCAAACGATTGGAACTCCACTAAATGTGAAG | 1321 |
| TTGCTCCTGAAAGGAGCAACTTAATGGACGGGGAG | 1322 |
| TTGCTGGAACTACAAGAAGTGGATTCGGTGGAGAG | 1323 |
| TTGGACTTCTAGTACGTGGTTACTCAACCACGCTG | 1324 |
| TTGGAGAAACAACCATACAGGTGTCTTTAACTACG | 1325 |
| TTGGATGAATACTCTCTGGCAACTCCCCAATGATG | 1326 |
| TTGGTTAAAGAACAGTCGCAGTTTTCCTCAAATCG | 1327 |
| TTGTCATTCGACTGAGGCTAGCGGATGTTGTGTCG | 1328 |
| TTGTCGGTTGTGTAGATCTCACGGTTAATACTGGG | 1329 |
| TTGTTAAAATTGCAGCTGTCCATAATGCTCCAGCG | 1330 |

(continued)

| TAG SEQUENCE | SEQ ID NO: |
|---|---|
| TTGTTTTGAAAACCATAGGAGGAAACCTCCTATTG | 1331 |
| TTTAAAGGCTGCAGCGTCGTCCTCAAATTTCGCAG | 1332 |
| TTTCCGCTGCTAACACAAAACCGGCCGTATCAAAG | 1333 |
| TTTCTGATTACACTGCCTTTTTCTTAATGGGGAAG | 1334 |
| TTTGACTTCTAATGTTTTTCTCATTGCATCGGGCG | 1335 |
| TTTGAGCAGTAAGGCGAACTCGGAAACTCGCATTG | 1336 |
| TTTGATGCTATTGGTTTGTTGGCTGAAACTGTTGG | 1337 |
| TTTGCACTCCATTAAATCCAGTTGGTAGTTGTATG | 1338 |
| TTTGGTTTGTGATTGGCAAATCTCTCCTCCAACTG | 1339 |
| TTTTAAATCAGCTTCTGAGAAACCGGTTGTTCCGG | 1340 |
| TTTTATTAGCGCCTGTGGAGCTACTAAATAGGTCG | 1341 |
| TTTTATTGCATTGTATTTCATCTTACCCAACCCCG | 1342 |
| TTTTGAACGGCATCTGCTACTGAATCTGCTTTTTG | 1343 |
| TTTTTTCTTGTCATGCGCGATCAAAGCAATTTTCG | 1344 |
| TTTTTTGACAGTGTAAATGAGCAGTTTGCCCAAAG | 1345 |

Target-Specific Sequences

The term "target-specific sequence" refers to a molecular entity that is capable of binding a target molecule. In the context of the tag-based nanoreporter system, the target-specific sequence of a capture or reporter oligo is in a first region that does not overlap with the second region, does not bind to the target, and binds or hybridizes to a capture or reporter probe.

The target specific sequence is generally an amino acid sequence (i.e., a polypeptide or peptide sequence) or a nucleic acid sequence.

In specific embodiments, where the target-specific sequence is an amino acid sequence, the target-specific sequence is an antibody fragment, such as an antibody Fab' fragment, a single chain Fv antibody.

The target-specific sequence is preferably a nucleic acid sequence, and is most preferably within an oligonucleotide that is covalently attached to a tag sequence, resulting in a oligonucleotide that is hybridizes or binds to a capture or reporter probe, i.e. a capture oligo or a reporter oligo. A target-specific nucleic acid sequence is preferably at least 15 nucleotides in length, and more preferably is at least 20 nucleotides in length. In specific embodiments, the target-specific sequence is approximately 10 to 500, 20 to 400, 30 to 300, 40 to 200, or 50 to 100 nucleotides in length. In other embodiments, the target-specific sequence is approximately 30 to 70, 40 to 80, 50 to 90, or 60 to 100, 30 to 120, 40 to 140, or 50 to 150 nucleotides in length.

A target-specific nucleotide sequence preferably has a Tm of about 65-90°C for each probe in 825 mM Na$^+$ (5xSSC), most preferably about 78-83°C.

Target Molecules

The term "target molecule" is the molecule detected or measured by binding of a labeled nanoreporter (i.e., a reporter probe/oligo pair and a capture probe/oligo pair) whose target-specific sequence(s) recognize (are specific binding partners thereto). Preferably, a target molecule can be, but is not limited to, any of the following: DNA, cDNA, RNA, or mRNA. Generally, a target molecule is a naturally occurring molecule or a cDNA of a naturally occurring molecule or the complement of said cDNA.

A target molecule can be part of a biomolecular sample that contains other components or can be the sole or major component of the sample. A target molecule can be a component of a whole cell or tissue, a cell or tissue extract, a fractionated lysate thereof or a substantially purified molecule. The target molecule can be attached in solution or solid-phase, including, for example, to a solid surface such as a chip, microarray or bead. Also, the target molecule can have either a known or unknown structure or sequence.

In certain specific embodiments, that target molecule is not a chromosome. In other specific embodiments, the target molecule is no greater than 1,000 kb (or 1 mb) in size, no greater than 500 kb in size, no greater than 250 kb in size, no greater than 175 kb in size, no greater than 100 kb in size, no greater than 50 kb in size, no greater than 20 kb in size, or no greater than 10 kb in size. In yet other specific embodiments, the target molecule is isolated from its cellular milieu.

Design of Label Attachment Regions

The present invention provides reporter and/or capture probes that are artificial nucleic acid molecules (DNA, RNA, or DNA/RNA hybrids) designed to have features that optimize labeling and detection of the tag-based nanoreporter.

A reporter probe or a capture probe can have 1, 2, 3, 4, 5, 6, 7, 8, 9, 10, 11, 12, 13, 14, 15, 16, 17, 18, 19, 20, 21-100 label attachment regions or more.

The label attachment regions of a reporter or capture probe will vary in size depending on the method of labeling. In various embodiments, a label attachment region can have a length anywhere from 10 nm to 10,000 nm, but is more preferably from 50 nm to 5,000 nm, and is more preferably from 100 nm to 1,000 nm. In various embodiments, the label attachment region is from about 100 nm to about 500 nm, from about 150 nm to about 450 mm, from about 200 nm to about 400 nm, or from 250 to about 350 nm. In a preferred embodiment, the label attachment region corresponds closely to the size of a diffraction-limited spot, i.e., the smallest spot that can be detected with standard optics, which is about 300 nm.

Where the probe is a nucleic acid, 1 nm corresponds to approximately 3 nucleotides; thus, an approximately 300 nm-label attachment region corresponds to approximately 900 bases. In other preferred embodiments, the label attachment region is from about 300 nucleotides to about 1.5 kb, from about 450 nucleotides to about 1.35 kb, from about 0.6 kb to about 1.2 kb, or from 0.75 kb to about 1.05 kb.

In these aspects of the invention, a reporter or capture probe is designed to have one or more regions, useful as label attachment regions, comprising a regular pattern of a particular base (the "regularly-repeated base"). In such regions, the regularly-repeated base occurs with a periodicity of every nth plus or minus 1 residue, where n is any number, and preferably from 4 to 25.

Preferably, not more than 25% of the regularly-repeated base in a region appears at other than said regular intervals. For example, if in a region of 100 nucleotides there are 12 thymidine bases, and thymidine is the regularly-repeated base, in this aspect of the invention not more than 25% of these, i.e., 3 thymidine bases, appear outside the regular pattern of thymidines. In specific embodiments, not more than 20%, not more than 15%, not more than 10%, not more than 9%, not more than 8%, not more than 7%, not more than 6%, not more than 5%, not more than 4%, not more than 3%, not more than 2% or not more than 1% of said base appears at other than said regular intervals in said region

The regularly-repeated base in the regions in a reporter or capture probe, or its complementary regularly-repeated base in an annealed segment can be used to attach label monomers, preferably light emitting label monomers, to the nanoreporter in a regular, evenly spaced pattern for better distribution of the nanoreporter signal. Preferably, where a region is labeled, at least 10%, at least 20%, at least 30%, at least 40%, at least 50%, at least 60%, at least 70%, at least 80%, at least 90%, at least 95% or at least 98% of occurrences of the regularly-repeated base is attached to at least one light-emitting label monomer, either by covalent attachment of a label monomer to a base, or by hybridization to a nucleic acid in which the complements of the regularly-repeated base are so-labeled.

This percentage of occurrences can be measured by any means known in the art. In one method, the amount of nucleic acid produced in a labeling reaction is purified (for example, RNA can be purified using a Qiagen RNeasy kit) and subjected to UV spectrophotometry. The absorbance ("A") at the appropriate wavelengths is measured for each of the nucleic acid (260 nm) and the label monomer whose occurrence is to be measured (e.g., 495 nm for Alexa Fluor 488; 590 nm for Alexa Fluor 594; 650 for Alexa Fluor 647; and 550 nm for Cy3). The absorbance of the nucleic acid is corrected by adjusting the value of the absorbance at 260 nm ("A260") to remove the "noise" contribution from the label monomer by subtracting the absorbance at the peak wavelength for the label monomer ($A_{LM}$) minus the correction factor for that label monomer. Where the nucleic acid is RNA, the number of label monomers per one thousand nucleotides is calculated according to the formula:

$$\frac{\text{no. of label monomers}}{1000 \text{ nucleotides}} = \frac{A260}{A_{LM}} \times \frac{9010}{EC_{LM}} \times 1000$$

where $EC_{LM}$ is the extinction coefficient for the label monomer. From this formula, the percentage of occurrences of the regularly-repeated base that are attached to a light-emitting label monomer can be calculated.

Generally, the preferred regularly-repeating base in a label attachment region is thymidine, so that the region can be labeled by hybridization to one or more complementary RNA segments in which the regularly-repeated base is uridine. This permits the use of amino-allyl-modified UTPs, which are readily commercially available, as label monomer attachment sites, in an otherwise random sequence. Preferably, in addition to the regular periodicity of the regions, the regions (and the nucleic acid comprising them) contain minimal secondary structure. The overall GC-content is preferably maintained close to 50%, and is preferably consistent over relatively short stretches to make local Tm's similar.

The artificial nucleic acids of the invention, or at least the regions therein, preferably do not have direct or inverted repeats that are greater than 12 bases in length. In other embodiments, the artificial nucleic acids and/or regions do not have direct or inverted repeats that are greater than about 11, about 10 or about 9 bases in length.

In an exemplary region in which the regularly-repeated nucleotide is a thymidine and a GC content of approximately 50%, excess adenines would make up the loss in abundance of T's. To generate the selected sequence, random sequences with fixed patterns of T's ranging from every 4th base to every 25th base are created and screened to minimize the presence of inverted and direct repeats.

Sequences are also screened preferably to avoid common six-base-cutter restriction enzyme recognition sites to aid in the ease of manipulation for conventional molecular cloning techniques. Selected sequences are additionally subjected to predicted secondary structure analysis, and those with the least secondary structure are chosen for further evaluation. Any program known in the art can be used to predict secondary structure, such as the MFOLD program (Zuker, 2003, Nucleic Acids Res. 31 (13):3406-15; Mathews et al., 1999, J. Mol. Biol. 288:911-940).

An appropriate sequence is divided into label attachment regions ranging from 50 bases to 2 kilobases long (could be longer). Each label attachment region is a unique sequence, but contains a consistent number and spacing of T's in relation to the other label attachment regions in a given reporter sequence. These label attachment regions can interspersed with other regions whose sequence does not matter. The synthetic label attachment regions in a nanoreporter scaffold can be of different lengths and/or have different regularly-repeated bases. An optimized start sequence for transcription by RNA polymerase T7, T3, or SP6 (beginning at position +1 of the transcript) can be added to the 5' end of each label attachment region. Restriction sites are optionally added at the boundaries of each label attachment region to allow specific addition or deletion of individual label attachment regions to the sequence using conventional cloning techniques. The number of synthetic label attachment regions in a nanoreporter preferably ranges from 1 to 50. In yet other embodiments, the number of synthetic label attachment regions in a nanoreporter ranges from 1, 2, 3, 4, 5, 6, 7, 8, 9, or 10 synthetic label attachment regions to 15, 20, 30, 40, or 50 synthetic label attachment regions, or any range in between.

The synthetic nucleic acids of the present invention can be chemically synthesized using naturally occurring nucleotides or variously modified nucleotides designed to increase the biological stability of the molecules or to increase the physical stability of the duplex formed between the label attachment region and the annealed segments, e.g., phosphorothioate derivatives and acridine substituted nucleotides can be used. Examples of modified nucleotides which can be used to generate the synthetic nucleic acid include 5-fluorouracil, 5-bromouracil, 5-chlorouracil, 5-iodouracil, hypoxanthine, xanthine, 4-acetylcytosine, S-(carboxyhydroxylmethyl)uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl)uracil, (acp3)w, and 2,6-diaminopurine.

Alternatively, the synthetic nucleic acid (i.e., the reporter and/or capture probe) can be produced biologically using a vector into which the nucleic acid has been subcloned.

In various embodiments, the synthetic nucleic acid molecules of the invention can be modified at the base moiety, sugar moiety or phosphate backbone to improve, e.g., the stability, hybridization, or solubility of the molecule. For example, the deoxyribose phosphate backbone of the nucleic acids can be modified to generate peptide nucleic acids (see Hyrup et al., 1996, Bioorganic & Medicinal Chemistry 4(1):5-23). As used herein, the terms "peptide nucleic acids" or "PNAs" refer to nucleic acid mimics, e.g., DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup et al., 1996, Bioorganic & Medicinal Chemistry 4(1): 5-23; Perry-O'Keefe et al., 1996, Proc. Natl. Acad. Sci. USA 93: 14670-675.

To make the RNA molecules ("segments") for each label attachment region, polymerase chain reaction ("PCR") primers are designed to generate a double-stranded template beginning with an RNA polymerase promoter (T7, T3, or SP6) directly upstream (5') of the transcription start site and ending following the 3' restriction enzyme site. Using this template, in vitro transcription of RNA molecules is performed in the presence of amino-allyl modified regularly-repeated base in the RNA (e.g., UTP') and unmodified other bases (e.g., ATP, CTP and GTP). This leads to an RNA product in which every regularly-repeated base (e.g., U) is modified to allow covalent coupling of a label monomer at that position in the RNA molecule.

Coupling of light-emitting label monomers to the RNA molecules and annealing of the labeled RNA molecules to the scaffold are carried out as described below.

Some design considerations for the synthetic sequence are listed in **Table 2**.

**Table 2**.

| Feature of Synthetic Scaffold | Advantages |
|---|---|
| Novel synthetic sequence | Can be of any length and be designed to incorporate any desired sequence feature including but not limited to those listed in this table. |
| Minimal secondary structure (select against inverted repeats) | Allows for consistent transcription of full-length RNA molecules. Allows for consistent annealing of RNA molecules to scaffold at predictable temperatures. Minimizes self-annealing and/or cross-annealing between RNA molecules or scaffolds. |
| Minimal repeated sequences | Avoids mis-annealing between RNA molecules and inappropriate regions of the scaffold. |
| Unique restriction sites at borders of label attachment regions | Allows addition and deletion of individual label attachment regions using standard molecular cloning techniques. |
| Defined, even spacing of T's and transcription with amino-allyl-modified UTP | Controls number of coupling sites for monomers in each label attachment region, allowing for consistent brightness of individual labeled RNA molecules. Controls distance between monomers: spacing can be optimized to avoid stearic hindrance and fluorescence quenching. |
| Optimized start sequence for transcription by RNA polymerase T7, T3, or SP6 | Promotes efficient in vitro transcription of each label attachment region. |

Label Monomers

The tag-based nanoreporters of the present invention can be labeled with any of a variety of label monomers, such as a fluorochrome, dye, enzyme, nanoparticle, chemiluminescent marker, biotin, or other monomer known in the art that can be detected directly (e.g., by light emission) or indirectly (e.g., by binding of a fluorescently-labeled antibody). Generally, one or more of the label attachments regions in the reporter and/or capture probe is labeled with one or more label monomers, and the signals emitted by the label monomers attached to the label attachment regions of a reporter and/or capture probe constitute a code that identifies the target to which the target-specific oligos bind. In certain embodiments, the lack of a given signal from the label attachment region (i.e., a "dark" spot) can also constitute part of the nanoreporter code. In certain preferred embodiments, the label monomers are fluorophores or quantum dots.

A preferred example of label monomers that can be utilized by the invention are fluorophores. Several fluorophores can be used as label monomers for labeling nucleotides including, for example, fluorescein, tetramethylrhodamine, and Texas Red. Several different fluorophores are known, and more continue to be produced, that span the entire spectrum. Also, different formulations of the same fluorophore have been produced for different applications. For example, fluorescein can be used in its isothiocynanate form (FITC), as mixed isomer or single isomer forms of carboxyfluorescein succinimidyl ester (FAM), or as isomeric dichlorotriazine forms of fluorescein (DTAF). These monomers are chemically distinct, but all emit light with a peak between 515-520 nm, thereby generating a similar signal. In addition to the chemical modifications of fluorescein, completely different fluorophores have been synthesized that have the same or very similar emission peaks as fluorescein. For example, the Oregon Green dye has virtually superimposable excitation and emission spectra compared to fluorescein. Other fluorophores such as Rhodol Green and Rhodamine Green are only slightly shifted in their emission peaks and so also serve functionally as substitutes for fluorescein. In addition, different formulations or related dyes have been developed around other fluorophores that emit light in other parts of the spectrum.

Very small particles, termed nanoparticles, also can be used as label monomers to label nucleic acids. These particles range from 1-1000 nm in size and include diverse chemical structures such as gold and silver particles and quantum dots. In a preferred embodiment, only one oligonucleotide molecule is coupled to each nanoparticle. To synthesize an oligonucleotide-nanoparticle complex in a 1:1 ratio by conventional batch chemistry, both the oligonucleotide and the nanoparticle require a single reactive group of different kinds that can be reacted with each other. For example, if an oligonucleotide has an amino group and a nanoparticle has an aldehyde group, these groups can react to form a Schiff base. An oligonucleotide can be derivitized to attach a single amino or other functional group using chemistry well known in the art. However, when a nanoparticle is derivatized, it is covered with a chemical reagent which results in coating the entire surface of the nanoparticle with several functional groups.

When irradiated with angled incident white light, silver or gold nanoparticles ranging from 40-120 nm will scatter monochromatic light with high intensity. The wavelength of the scattered light is dependent on the size of the particle. Four to five different particles in close proximity will each scatter monochromatic light which when superimposed will give a specific, unique color. The particles are being manufactured by companies such as Genicon Sciences. Derivatized silver or gold particles can be attached to a broad array of molecules including nucleic acids.

Another type of nanoparticle that can be used as a label monomer are quantum dots. Quantum dots are fluorescing crystals 1-5 nm in diameter that are excitable by a large range of wavelengths of light. These crystals emit light, such as monochromatic light, with a wavelength dependent on their chemical composition and size. Quantum dots such as CdSe, ZnSe, InP, or InAs possess unique optical properties. Due to their very small size the quantum dots can be coupled into oligonucleotides directly without affecting the solubility or use of the oligonucleotide.

Many dozens of classes of particles can be created according to the number of size classes of the quantum dot crystals. The size classes of the crystals are created either 1) by tight control of crystal formation parameters to create each desired size class of particle, or 2) by creation of batches of crystals under loosely controlled crystal formation parameters, followed by sorting according to desired size and/or emission wavelengths. Use of quantum dots for labeling particles, in the context of the present invention, is new, but is old in the art of semiconductors. Two examples of earlier references in which quantum dots are embedded within intrinsic silicon epitaxial layers of semiconductor light emitting/detecting devices are U.S. Pat. Nos. 5,293,050 and 5,354,707 to Chapple Sokol, et al.

In specific embodiments, one or more of the label attachments regions in the nanoreporter is labeled with one or more light-emitting dyes, each label attachment region containing, directly or indirectly, one or more label monomers. The light emitted by the dyes can be visible light or invisible light, such as ultraviolet or infrared light. In exemplary embodiments, the dye is a fluorescence resonance energy transfer (FRET) dye; a xanthene dye, such as fluorescein and rhodamine; a dye that has an amino group in the alpha or beta position (such as a naphthylamine dye, 1-dimethylaminonaphthyl-5-sulfonate, 1-anilino-8-naphthalende sulfonate and 2-p-touidinyl-6-naphthalene sulfonate); a dye that has 3-phenyl-7-isocyanatocoumarin; an acridine, such as 9-isothiocyanatoacridine and acridine orange; a pyrene, a bensoxadiazole and a stilbene; a dye that has 3-($\epsilon$-carboxypentyl)-3'-ethyl-5,5'-dimethyloxacarbocyanine (CYA); 6-carboxy fluorescein (FAM); 5&6-carboxyrhodamine-110 (R110); 6-carboxyrhodamine-6G (R6G); N,N,N',N'-tetramethyl-6-carboxyrhodamine (TAMRA); 6-carboxy-X-rhodamine (ROX); 6-carboxy-4',5'-dichloro-2',7'-dimethoxyfluorescein (JOE); ALEXA Fluor.TM.; Cy2; Texas Red and Rhodamine Red; 6-carboxy-2',4,7,7'-tetrachlorofluorescein (TET); 6-carboxy-2',4,4',5',7,7'-hexachlorofluorescein (HEX); 5-carboxy-2',4',5',7'-tetrachlorofluorescein (ZOE); NAN; NED; Cy3; Cy3.5; Cy5; Cy5.5; Cy7; and Cy7.5; Alexa Fluor 350; Alexa Fluor 488; Alexa Fluor 532; Alexa Fluor 546; Alexa Fluor 568; Alexa Fluor 594; or Alexa Fluor 647.

A label monomer can be directly attached to a nucleotide using methods well known in the art. Nucleotides can also be chemically modified or derivitized in order to attach a label monomer.

A nucleotide can be attached to a label monomer first and then be incorporated into a nucleic acid. Alternatively, an existing nucleic acid can be labeled by attaching a label monomer to a nucleotide within the nucleic acid. For example aminoallyl- ("AA-") modified UTP nucleotides can be incorporated into the RNA product during transcription. In various embodiments, 20% or more of UTP nucleotides in a transcription reaction to generate RNA patches are AA modified. In various embodiments, about 20% to 100%, 20% to 80%, 30 to 80%, 40 to 60% or 50% to 75% of UTPs in a transcription reaction are AA-modified, in a preferred embodiment, approximately 50% of UTPs in a transcription reaction are AA-modified.

In addition, for example, different types of label monomer:nucleotide complexes can be incorporated into a single acid nucleic acid, where one component of the nanoreporter code comprises more than one type of signal.

Fluorescent dyes that can be bound directly to nucleotides can also be utilized as label monomers. For example, FAM, JOE, TAMRA, and ROX are amine reactive fluorescent dyes that have been attached to nucleotides and are used in automated DNA sequencing. These fluorescently labeled nucleotides, for example, ROX-ddATP, ROX-ddCTP, ROX-ddGTP and ROX-ddUTP, are commercially available.

Affinity Moieties

A variety of affinity moieties known in the art may be used to purify and/or immobilize the tag-based nanoreporters described herein.

Where an affinity moiety is used to immobilize a tag-based nanoreporter for the purpose of detection or imaging, it may be referred to herein as an "anchor." In a preferred embodiment, a biotin anchor is attached to the nanoreporter (i.e., the capture probe of the tag-based nanoreporter), allowing immobilization of the nanoreporter on a streptavidin coated slide.

An affinity moiety that can be used for attachment to beads or other matrices for a variety of useful applications including but not limited to purification.

Non-limiting examples of suitable affinity moieties are provided below. It should be understood that most affinity moieties could serve dual purposes: both as anchors for immobilization of the nanoreporters and moieties for purification of the nanoreporters (whether fully or only partially assembled).

In certain embodiments, the affinity moiety is a protein monomer. Examples of protein monomers include, but are not limited to, the immunoglobulin constant regions (see Petty, 1996, Metal-chelate affinity chromatography, in Current Protocols in Molecular Biology, Vol. 2, Ed. Ausubel et al., Greene Publish. Assoc. & Wiley Interscience), glutathione S-transferase (GST; Smith, 1993, Methods Mol. Cell. Bio. 4:220-229), the E. coli maltose binding protein (Guan et al., 1987, Gene 67:21-30), and various cellulose binding domains (U.S. Pat. Nos. 5,496,934; 5,202,247; 5,137,819; Tomme et al., 1994, Protein Eng. 7:117-123), etc. Other affinity tags are recognized by specific binding partners and thus facilitate isolation and immobilization by affinity binding to the binding partner, which can be immobilized onto a solid support. For example, the affinity moiety can be an epitope, and the binding partner an antibody. Examples of such epitopes include, but are not limited to, the FLAG epitope, the myc epitope at amino acids 408-439, the influenza virus hemagglutinin (HA) epitope, or digoxigenin ("DIG"). In other embodiments, the affinity moiety is a protein or amino acid sequence that is recognized by another protein or amino acid, for example the avidin/streptavidin and biotin.

In certain aspects of the invention, the affinity moiety is a nucleotide sequence. A large variety of sequences of about 8 to about 30 bases, more preferably of about 10 to about 20 bases, can be used for purification and immobilization of nanoreporters, and the sequence can be tandemly repeated (e.g., from 1 to 10 tandem repeats). Such a sequence is preferably not widely represented (that is, present in fewer than 5% of the genes, more preferably, present in fewer than 3% of the genes, and, most preferably, present in fewer than 1% of the genes) in the sample being assayed (for example, where the nanoreporter is used for detection of human cellular RNA, the sequence is preferably not widely represented in the human genome); have little or no secondary structure or self-complementarity either internally or with copies of itself when multimerized (that is, all secondary structures of the multimerized tag preferably have a Tm less than 25°C. at 1 M NaCl); have no significant identity or complementarity with segment or tag sequences (that is, the Tm of complementary sequences is preferably less than 25°C. at 0.2 M NaCl); and have a Tm of about 35-65°C., more preferably about 40-50°C., in 50 mM Na+.

In certain embodiments, different sequences are used as purification and immobilization moieties. In this case, for example, the purification moiety can be as described above, but the immobilization moiety can be in the range of 10 to 100 bases, with a Tm up to 95° C. in 50 mM Na⁺. An alternative embodiment would be to have the purification moiety nested within the immobilization moiety (e.g., the affinity moiety would comprise a 25-base sequence of which 15 bases are used as a purification moiety and the entire 25 bases are used as the immobilization moiety).

In certain instances, the affinity moiety can be used for labeling a nanoreporter in addition to purifying or immobilizing the nanoreporter.

As will be appreciated by those skilled in the art, many methods can be used to obtain the coding region of the affinity moieties, including but not limited to, DNA cloning, DNA amplification, and synthetic methods. Some of the affinity moieties and reagents for their detection and isolation are available commercially.

Tag-Based Nanoreporter Populations

The present invention provides tag-based nanoreporter (*e.g.*, compositions comprising reporter probe/oligo and capture probe/oligo pairs) populations, that contain at least 10, at least 15, at least 20, at least 25, at least 30, at least 40, at least 50, at least 75, at least 100, at least 200, at least 300, at least 400, at least 500, at least 750, or at least 1,000 unique tag-based nanoreporters. As used herein, "unique" when used in reference to a nanoreporter within a population is intended to mean a tag-based nanoreporter that has a code that distinguishes it from other tag-based nanoreporters in the same population.

In specific embodiments, the present invention provides nanoreporter populations with at least 5,000, at least 10,000, at least 20,000 or at least 50,000 unique nanoreporters.

The size of a tag-based nanoreporter population and the nature of the target-specific sequences of the reporter and capture oligos within it will depend on the intended use of the nanoreporter. Nanoreporter populations can be made in which the target-specific sequences correspond to markers of a given cell type, including a diseased cell type. In certain embodiments, tag-based nanoreporters populations are generated in which the target-specific sequences of the reporter and/or capture oligos represent at least 0.1%, at least 0.25%, at least 0.5%, at least 1%, at least 2%, at least 3%, at least 4%, at least 5%, at least 10%, at least 15%, at least 20%, at least 25%, at least 30%, at least 40%, at least 50%, at least 60%, or at least 70% of the different type of transcripts in a cell. In certain embodiments, tag-based nanoreporters populations are generated in which the target-specific sequences of the reporter and/or capture oligos represent at least 0.1%, at least 0.25%, at least 0.5%, at least 1%, at least 2%, at least 3%, at least 4%, at least 5%, at least 10%, at least 15%, at least 20%, at least 25%, at least 30%, at least 40%, at least 50%, at least 60%, or at least 70% of the different genes in a cell. In yet other embodiments, tag-based nanoreporter populations are generated in which at least some of the target-specific sequences of the reporter and/or capture oligos represent rare transcripts in a cell or tissue. Such tag-based nanoreporter populations preferably represent at least 5 rare transcripts. In specific embodiments, such tag-based nanoreporter populations represent at least 10, at least 20, at least 30, at least 40 or at least 50 rare transcripts.

In a specific embodiment, the cell or tissue is a mammalian cell or tissue, and more preferably is a human cell or tissue.

In certain embodiments, the tag-based nanoreporter population is a diagnostic or prognostic nanoreporter populations. For example, a diagnostic nanoreporter population can be generated that is useful for screening blood products, in which the target-specific sequences bind to the nucleic acids of contaminating viruses such the hepatitis B, hepatitis C, and the human immunodeficiency virus. Alternatively, the diagnostic nanoreporter population may contain reporter and capture oligos with target-specific sequences corresponding to cellular disease markers, such as tumor antigens. Prognostic nanoreporter populations generally include reporter and capture oligos with target-specific sequences that recognize markers that represent different stages of a given disease such as cancer. By selecting appropriate target-specific sequences, a tag-based nanoreporter population can be used both to diagnose and prognose disease.

Biomolecular Samples

The tag-based nanoreporter systems of the invention can be used to detect target molecule in any biomolecular sample. As will be appreciated by those in the art, the sample may comprise any number of things, including, but not limited to: cells (including both primary cells and cultured cell lines), cell lysates or extracts (including but not limited to RNA extracts; purified mRNA), tissues and tissue extracts (including but not limited to RNA extracts; purified mRNA); bodily fluids (including, but not limited to, blood, urine, serum, lymph, bile, cerebrospinal fluid, interstitial fluid, aqueous or vitreous humor, colostrum, sputum, amniotic fluid, saliva, anal and vaginal secretions, perspiration and semen, a transudate, an exudate (e.g., fluid obtained from an abscess or any other site of infection or inflammation) or fluid obtained from a joint (e.g., a normal joint or a joint affected by disease such as rheumatoid arthritis, osteoarthritis, gout or septic arthritis) of virtually any organism, with mammalian samples being preferred and human samples being particularly preferred; environmental samples (including, but not limited to, air, agricultural, water and soil samples); biological warfare agent samples; research samples including extracellular fluids, extracellular supernatants from cell cultures, inclusion bodies in bacteria, cellular compartments, cellular periplasm, mitochondria compartment, etc.

The biomolecular samples can be indirectly derived from biological specimens. For example, where the target molecule of interest is a cellular transcript, e.g., a messenger RNA, the biomolecular sample of the invention can be a sample containing cDNA produced by a reverse transcription of messenger RNA. In another example, the biomolecular sample of the invention is generated by subjecting a biological specimen to fractionation, e.g., size fractionation or membrane fractionation.

The biomolecular samples of the invention may be either "native," i.e., not subject to manipulation or treatment, or "treated," which can include any number of treatments, including exposure to candidate agents including drugs, genetic engineering (e.g. the addition or deletion of a gene), etc.

Separation of Label Monomers

In addition to detecting an overall signal generated from a tag-based nanoreporter, the invention provides for the determination of the spatial location of signals emanating from the label monomers (i.e., spots) on a nanoreporter, each spot representing the aggregate signal from label monomers attached to a given label attachment region. A spot may contain signals of the same wavelength or of different wavelengths. Thus, the nature of the spots on a nanoreporter and their location constitutes the nanoreporter code.

Any of a variety of means can be used to "stretch" the nanoreporter to separate the individual spots. For example, a nanoreporter can be stretched using a flowstretch technique (Henegariu et al., 2001, Biotechniques 31:246-250), a receding meniscus technique (Yokota et al., 1997, Nuc. Acids Res. 25:1064-1070) or an electrostretching technique (Matsuura et al., 2001, Nuc. Acids Res. 29: E79).

The use of flow-stretching, receding meniscus, or electro-stretching techniques allows for the separation of the label attachment regions within a nanoreporter so that one can determine spatially where a particular signal is positioned in the nanoreporter. Therefore, unique nanoreporters that have the same combination of label monomers and the same overall signal can be differentiated from one another based on the location of those label monomers within the nanoreporter.

This ability to locate the position of a label attachment region or spot within a nanoreporter allows for the position of the signal(s) emitted by the label monomers in each label attachment region to be used as a distinguishing characteristic when generating a set of unique nanoreporters. Hence, a complex set of nanoreporters can be generated using the same combination of starting label monomers by varying the positions of the label monomers within a nanoreporter.

Prior to stretching a nanoreporter, it is preferable to immobilize the nanoreporter to a solid surface using an affinity tag, as described above.

In certain aspects of the invention, one end of a nanoreporter is immobilized, either through specific or non-specific binding to a solid surface, the nanoreporter is stretched, and then the other end of the reporter is immobilized, also either through specific or non-specific binding to a solid surface. Accordingly, the nanoreporter is "frozen" in its stretched, or extended, state, to facilitate resolution of the nanoreporters code by detecting and/or imaging the signals emitted by the label monomers attached to a nanoreporter and their locations relative to one another. These aspects of the invention are described below.

Immobilization of Stretched Nanoreporters

The present invention provides methods and compositions that facilitate the identification of primary structures of the tag-based nanoreporters described herein. In certain aspects, the present invention provides methods for the selective immobilization of nanoreporters in an extended state. According to the invention, a nanoreporter can be selectively immobilized while fully extended under whatever force is used for the extension. In addition, the methods of the invention facilitate the selective immobilization of extended nanoreporters that are oriented with respect to each other. In other words, according to the methods of the invention, a plurality of nanoreporters can readily be immobilized in the same orientation with respect to each other.

In one aspect, the present invention provides methods for selectively immobilizing a nanoreporter in an extended state. For the methods of this aspect of the invention, generally, a first portion of the nanoreporter, or capture probe hybridized to the target-specific capture oligo, is immobilized by any technique known to those of skill in the art. In certain embodiments, the first portion of the nanoreporter, or capture probe hybridized to the target-specific capture oligo, can be immobilized selectively or non-selectively. In certain embodiments the first portion is immobilized by one or more covalent bonds. In certain embodiments, the first portion is immobilized by one or more non-covalent bonds. Exemplary immobilized first portions are described in the sections below.

With an immobilized first portion, the nanoreporter can be extended by any technique for extending a nanoreporter apparent to those of skill in the art. In certain embodiments, the technique for extending the nanoreporter is not critical for the methods of the invention. In certain embodiments, the technique for extending the nanoreporter appropriate for the class of nanoreporter according to the judgment of one of skill in the art. In certain embodiments, the nanoreporter is extended by application of a force capable of extending the nanoreporter. The force can be any force apparent to one of skill in the art for extending the nanoreporter. Exemplary forces include gravity, hydrodynamic force, electromagnetic force and combinations thereof. Specific techniques for extending the nanoreporter are described in the sections below.

The nanoreporter is in an extended state if it would be recognized as extended by one of skill in the art. In certain embodiments, the nanoreporter is in an extended state when it is in the field of a force capable of extending the nanoreporter. In certain embodiments, the nanoreporter is in an extended state when its average hydrodynamic radius is more than double the average hydrodynamic radius of the nanoreporter in its native state as recognized by those of skill in the art.

In this aspect of the invention, the methods generally comprise the step of selectively immobilizing a second portion of the nanoreporter while it is in an extended state. This can result in an immobilized nanoreporter that is extended between the first and the second portion. Remarkably, since the nanoreporter is selectively immobilized while extended, that extension can be preserved in the immobilized nanoreporter. The selective immobilization can be according to any technique for selective immobilization of a portion of a nanoreporter apparent to those of skill in the art. The selective immobilization can be through, for example, the formation of one or more covalent bonds or one or more non-covalent bonds, or both. Particular examples of selective immobilization techniques are described in the sections below. In particular embodiments, one or more binding pairs are used to immobilize the second portion of the nanoreporter, which is the reporter probe hybridized to the target-specific reporter oligo.

The second portion can be immobilized onto any substrate apparent to those of skill in the art. The substrate can be any substrate judged to be useful for immobilization known to those of skill in the art. In certain embodiments, the second portion can be immobilized to another molecule. Further useful substrates include surfaces, membranes, beads, porous materials, electrodes, arrays and any other substrate apparent to those of skill in the art.

In another aspect, the present invention provides a composition comprising a selectively immobilized, extended nanoreporter. The compositions generally comprise a substrate and an extended nanoreporter selectively immobilized onto the substrate. The substrate can be any substrate known to those of skill in the art. Exemplary substrates include those described in the sections below. At least two portions of the nanoreporter are immobilized onto the substrate, and the nanoreporter is in an extended state between the two portions. In certain embodiments, at least one portion of the nanoreporter is selectively immobilized onto the substrate. In certain embodiments, two or more portions of the nanoreporter are selectively immobilized onto the substrate. The nanoreporter can be extended and/or immobilized by any technique apparent to those of skill, including particularly the methods of the present invention.

In another aspect, the present invention provides methods for selectively immobilizing a nanoreporter in an oriented state. The nanoreporter can be any nanoreporter described above. In certain embodiments, the nanoreporter can be flexible, or in certain embodiments the nanoreporter can be rigid or semi-rigid. For the methods of this aspect of the invention, generally, a first portion of the nanoreporter is immobilized as described above. With an immobilized first portion, the nanoreporter can be oriented by any technique for extending a nanoreporter apparent to those of skill in the art. In certain embodiments, the technique for orienting the nanoreporter is not critical for the methods of the invention. In certain embodiments, the technique for orienting the nanoreporter appropriate for the class of nanoreporter according to the judgment of one of skill in the art. In certain embodiments, the nanoreporter is oriented by application of a force capable of orienting the nanoreporter. The force can be any force apparent to one of skill in the art for orienting the nanoreporter. Exemplary forces include gravity, hydrodynamic force, electromagnetic force and combinations thereof. Specific techniques for extending the nanoreporter are described in the subsections below.

The nanoreporter is in an oriented state if it would be recognized as oriented by one of skill in the art. In certain embodiments, the nanoreporter is in an oriented state when it is in the field of a force capable of orienting the nanoreporter. In certain embodiments, the nanoreporter is in an oriented state when its termini are arranged in parallel, as recognized by those of skill in the art, with the field of a force capable of orienting the nanoreporter. In certain embodiments, a plurality of nanoreporters is in an oriented state when the termini of the nanoreporters are arranged in parallel, as recognized by those of skill in the art.

In this aspect of the invention, the methods generally comprise the step of selectively immobilizing a second portion of the nanoreporter while it is in an oriented state. This can result in an immobilized nanoreporter that is oriented between the first and the second portion. Remarkably, since the nanoreporter is selectively immobilized while extended, that orientation can be preserved in the immobilized nanoreporter. The selective immobilization can according to the methods described above.

In another aspect, the present invention provides a composition comprising a selectively immobilized, oriented nanoreporter. The compositions generally comprise a substrate and an oriented nanoreporter selectively immobilized onto the substrate. The substrate can be any substrate known to those of skill in the art. Exemplary substrates include those described in the sections below. At least two portions of the nanoreporter are immobilized onto the substrate, and the nanoreporter is in an oriented state between the two portions. In certain embodiments, at least one portion of the nanoreporter is selectively immobilized onto the substrate. In certain embodiments, both portions of the nanoreporter are selectively immobilized onto the substrate. The nanoreporter can be oriented and/or immobilized by any technique apparent to those of skill, including particularly the methods of the present invention.

The methods and compositions of the present invention can be used for any purpose apparent to those of skill in the art. For instance, the immobilized and extended and/or oriented nanoreporter can be used as a label for a substrate on which the nanoreporter is immobilized. The primary sequence of the immobilized and extended and/or oriented nanoreporter can be identified by any technique apparent to those of skill. Advantageously, immobilization of the extended and/or oriented nanoreporter can facilitate such techniques. In certain embodiments, the immobilized and extended and/or oriented nanoreporter can be used to guide the manufacture of nanopaths, for example to create nanowires or nanocircuits. Further uses for the immobilized and extended and/or oriented nanoreporters are described in the sections below.

All terms used herein have their ordinary meanings to those of skill in the art unless indicated otherwise. The following terms shall have the following meanings.

As used herein, the term "binding pair" refers to first and second molecules or moieties that are capable of selectively binding to each other, i.e. binding to each other with greater affinity than to other components in a composition. The binding between the members of the binding pair can be covalent or non-covalent. In certain embodiments, the binding is noncovalent. Exemplary binding pairs include immunological binding pairs (e.g., any haptenic or antigenic compound in combination with a corresponding antibody or binding portion or fragment thereof, for example digoxigenin and anti-digoxigenin, fluorescein and anti-fluorescein, dinitrophenol and anti-dinitrophenol, bromodeoxyuridine and anti-bromodeoxyuridine, mouse immunoglobulin and goat anti-mouse immunoglobulin) and nonimmunological binding pairs (e.g., biotin-avidin, biotin-streptavidin, hormone-hormone binding protein, receptor-receptor ligand (e.g., acetylcholine receptor-acetylcholine or an analog thereof), IgG-protein A, lectin-carbohydrate, enzyme-enzyme cofactor, enzyme-enzyme inhibitor, complementary polynucleotide pairs capable of forming nucleic acid duplexes, and the like). For instance, immunoreactive binding members may include antigens, haptens, aptamers, antibodies (primary or secondary), and complexes thereof, including those formed by recombinant DNA methods or peptide synthesis. An antibody may be a monoclonal or polyclonal antibody, a recombinant protein or a mixture(s) or fragment(s) thereof, as well as a mixture of an antibody and other binding members. Other common binding pairs include but are not limited to, biotin and avidin (or derivatives thereof), biotin and streptavidin, carbohydrates and lectins, complementary nucleotide sequences (including probe and capture nucleic acid sequences), complementary peptide sequences including those formed by recombinant methods, effector and receptor molecules, hormone and hormone binding protein, enzyme cofactors and enzymes, enzyme inhibitors and enzymes, and so forth.

"Selective binding" refers to the any preferential binding of a pair of molecules or moieties for each other with respect to other molecules or moieties in a composition that would be recognized by one of skill in the art. In certain embodiments, a pair of molecules or moieties selectively binds when they preferentially bind each other compared to other molecules or moieties. Selective binding can include affinity or avidity, or both, of one molecule or moiety for another molecule or moiety. In particular embodiments, selective binding requires a dissociation constant ($K_D$) of less than about $1x10^{-5}$ M or less than about $1x10^{-6}$ M, $1x10^{-7}$ M, $1x10^{-8}$ M, $1x10^{-9}$ M, or $1x10^{-10}$ M. In contrast, in certain embodiments, non-selective binding has significantly less affinity, for example, a $K_D$ greater than $1x10^{-3}$ M

"Extended state" refers to a nanoreporter in a state that would be recognized as extended by one of skill in the art. In certain embodiments, a nanoreporter is in an extended state when it is extended relative to its native conformation in solution. In certain embodiments, a nanoreporter is in an extended state when it is in the field of a force capable of extending the nanoreporter. In certain embodiments, an extended state of a nanoreporter can be determined quantitatively. In such embodiments, those of skill in the art will recognize R as the end-to-end vector of the nanoreporter, i.e. the distance between two termini of the nanoreporter, and <R> as the average end-to-end vector such that 95% of R will be within 2<R> in a solution deemed appropriate to one of skill in the art. Exemplary solutions include, for example, a dilute solution of the nanoreporter in water or in a pH buffer. In particular embodiments, a nanoreporter is in an extended state when R is greater than 2.0<R>.

"Oriented state" refers to a nanoreporter in a state that would be recognized as oriented by one of skill in the art. In certain embodiments, a nanoreporter is in an oriented state when it is oriented relative to its native conformation in solution. In certain embodiments, the nanoreporter is oriented when it is arranged in parallel with the field of a force capable of orienting the nanoreporter. In certain embodiments, the nanoreporter is oriented when it is one of a plurality of nanoreporters that are arranged in parallel, as recognized by those of skill in the art.

Methods of Selective Immobilization

As described above, the present invention provides methods for the selective immobilization of a nanoreporter in an extended state. The nanoreporter, once selectively immobilized, can be used for any purpose apparent to those of skill in the art.

In the methods of the invention, a first portion of the nanoreporter is immobilized. For example, the first portion of the nanoreporter is the capture probe hybridized to a target-specific capture oligo. Generally, the first portion is immobilized if it would be recognized as immobilized by one of skill in the art. The first portion can be immobilized by any technique apparent to those of skill in the art. In certain embodiments, the technique for immobilization of the first portion of the nanoreporter is not critical for the methods of the invention.

The nanoreporter can be immobilized onto any substrate apparent to those of skill in the art. The substrate can be any moiety to which the nanoreporter can be immobilized without limitation. In certain embodiments, the substrate is a surface, membrane, bead, porous material, electrode or array.

In certain embodiments, the first portion of the nanoreporter can be immobilized non-selectively. In further embodiments, the first portion of the nanoreporter can be immobilized selectively. In advantageous embodiments, after the first portion of the nanoreporter is immobilized, some portion of the nanoreporter should be free to move sufficiently so that the nanoreporter can be extended in the following steps of the method. In particular, in certain embodiments, when the first portion of the nanoreporter is immobilized non-selectively, it is important that the entire nanoreporter not be immobilized non-selectively to an extent that prevents extension of any portion of the nanoreporter

The immobilization can be by any interaction with the substrate apparent to those of skill in the art. The immobilization can be via electrostatic or ionic interaction, via one or more covalent bonds, via one or more non-covalent bonds or combinations thereof. In certain embodiments, the immobilization can be via electrostatic interaction with an electrode. In further embodiments, the immobilization is via electrostatic interaction with a substrate other than the electrode

In certain embodiments, the capture probe of the first portion of the nanoreporter comprises a first member of a binding pair (i.e. an affinity moiety). The first member of the binding pair can be covalently bound to the first portion of the nanoreporter, or they can be non-covalently bound. Useful covalent bonds and non-covalent bonds will be apparent to those of skill in the art. In useful embodiments, the substrate onto which the first portion of the nanoreporter is bound will comprise a second member of the binding pair. The substrate can be covalently bound to the second member, or they can be non-covalently bound.

In certain embodiments, the first portion of the nanoreporter (i.e., the capture probe) can comprise a member of a binding pair that is capable of binding with a member of a binding pair on the substrate to form one or more non-covalent bonds. Exemplary useful substrates include those that comprise a binding moiety selected from the group consisting of ligands, antigens, carbohydrates, nucleic acids, receptors, lectins, and antibodies. The first portion of the nanoreporter would comprise a binding moiety capable of binding with the binding moiety of the substrate. Exemplary useful substrates comprising reactive moieties include, but are not limited to, surfaces comprising epoxy, aldehyde, gold, hydrazide, sulfhydryl, NHS-ester, amine, thiol, carboxylate, maleimide, hydroxymethyl phosphine, imidoester, isocyanate, hydroxyl, pentafluorophenyl-ester, psoralen, pyridyl disulfide or vinyl sulfone, or mixtures thereof. Such surfaces can be obtained from commercial sources or prepared according to standard techniques.

In advantageous embodiments, the first portion of the nanoreporter can be immobilized to the substrate via an avidin-biotin binding pair. In certain embodiments, the nanoreporter can comprise a biotin moiety in its first portion. For instance, a polynucleotide nanoreporter can comprise a biotinylated nucleotide residue. Similarly, a polypeptide nanoreporter can comprise a biotinylated amino acid residue. The substrate comprising avidin can be any substrate comprising avidin known to those of skill in the art. Useful substrates comprising avidin are commercially available including TB0200 (Accelr8), SAD6, SAD20, SAD100, SAD500, SAD2000 (Xantec), SuperAvidin (Array-It), streptavidin slide (catalog #MPC 000, Xenopore) and STREPTAVIDINnslide (catalog #439003, Greiner Bio-one).

In certain embodiments, the first portion of the nanoreporter (i.e., the capture probe) can comprise a nucleotide sequence that is capable of selectively binding a nucleotide sequence on the substrate.

In further embodiments, the first portion of the nanoreporter (i.e., the capture probe) can comprise avidin, and the substrate can comprise biotin. Useful substrates comprising biotin are commercially available including Optiarray-biotin (Accler8), BD6, BD20, BD100, BD500 and BD2000 (Xantec).

In further embodiments, the first portion of the nanoreporter (i.e., the capture probe) is capable of forming a complex with one or more other molecules that, in turn, are capable of binding, covalently or non-covalently, a binding moiety of the substrate. For instance, a first portion of the nanoreporter can be capable of selectively binding another molecule that comprises, for instance, a biotin moiety that is capable of selectively binding, for instance, an avidin moiety of the substrate.

In further embodiments, the first portion of the nanoreporter (i.e., the capture probe) can comprise a member of a binding pair that is capable of reacting with a member of a binding pair on the substrate to form one or more covalent bonds. Exemplary useful substrates comprising reactive groups include those that comprise a reactive moiety selected from the group consisting of succinamides, amines, aldehydes, epoxies and thiols. The first portion of the nanoreporter would comprise a reactive moiety capable of reacting with the reactive moiety of the substrate. Exemplary useful substrates comprising reactive moieties include, but are not limited to, OptArray-DNA NHS group (Accler8), Nexterion Slide AL (Schott) and Nexterion Slide E (Schott).

In certain embodiments, the first portion of the nanoreporter (i.e., the capture probe) can comprise a reactive moiety that is capable of being bound to the substrate by photoactivation. The substrate could comprise the photoreactive moiety, or the first portion of the nanoreporter could comprise the photoreactive moiety. Some examples of photoreactive moieties include aryl azides, such as N-((2-pyridyldithio)ethyl)-4-azidosalicylamide; fluorinated aryl azides, such as 4-azido-2,3,5,6-tetrafluorobenzoic acid; benzophenone-based reagents, such as the succinimidyl ester of 4-benzoylbenzoic acid; and 5-Bromo-deoxyuridine.

In further embodiments, the first portion of the nanoreporter (i.e., the capture probe) can be immobilized to the substrate via other binding pairs apparent to those of skill in the art.

Extension of the Nanoreporter

In certain methods of the invention, the nanoreporter is in an extended state. Generally, any nanoreporter is in an extended state if it would be recognized as such by one of skill in the art.

In certain embodiments, the nanoreporter is in an extended state when it is in the field of a force capable of extending the nanoreporter under conditions suitable for extending the nanoreporter. Such forces and conditions should be apparent to those of skill in the art. For instance, many nanoreporters can be extended by hydrodynamic force or by gravity, and many charged nanoreporters can be extended by electromagnetic force. In certain embodiments, the force can be applied to the nanoreporter indirectly. For instance, the nanoreporter can comprise or can be linked, covalently or noncovalently, to a moiety capable of being moved by a force. In certain embodiments, the nanoreporter can be linked to a moiety.

In certain embodiments, the force is an electromagnetic force. For instance, when the nanoreporter is charged, such as a polynucleotide, the nanoreporter can be extended in an electric or magnetic field. The field should be strong enough to extend the nanoreporter according to the judgment of one of skill in the art. Exemplary techniques for extending a nanoreporter in an electric or magnetic field are described in Matsuura et al., 2002, J Biomol Struct Dyn. 20(3):429-36; Ferree & Blanch, 2003, Biophys J. 85(4):2539-46; Stigter & Bustamante, 1998, Biophys J. 1998 75(3):1197-210; Matsuura et al., 2001, Nucleic Acids Res. 29(16); Ferree & Blanch, 2004, Biophys J. 87(1):468-75; the contents of which are hereby incorporated by reference in their entirety.

In certain embodiments, the force is a hydrodynamic force. For instance, many nanoreporters, including polysaccharides, polypeptides, and polynucleotides, can be extended in the field of a moving fluid. The hydrodynamic force should be strong enough to extend the nanoreporter according to the judgment of one of skill in the art. Exemplary techniques for extending a nanoreporter in hydrodynamic field are described in Bensimon et al., 1994, Science 265:2096-2098; Henegariu et al., 2001, BioTechniques 31: 246-250; Kraus et al., 1997, Human Genetics 99:374-380; Michalet et al., 1997, Science 277:1518-1523; Yokota et al., 1997, Nucleic Acids Res. 25(5):1064-70; Otobe et al., 2001, Nucleic Acids Research 29:109; Zimmerman & Cox, 1994, Nucleic Acids Res. 22(3):492-7, and U.S. Pat. Nos. 6,548,255; 6,344,319; 6,303,296; 6,265,153; 6,225,055; 6,054,327; and 5,840,862, the contents of which are hereby incorporated by reference in their entirety.

In certain embodiments, the force is gravity. In advantageous embodiments, the force of gravity can be combined with, for example, hydrodynamic force to extend the nanoreporter. In certain embodiments, the force should be strong enough to extend the nanoreporter according to the judgment of one of skill in the art. Exemplary techniques for extending a nanoreporter with gravity are described in Michalet et al, 1997, Science 277:1518-1523; Yokota et al., 1997, Nucleic Acids Res. 25(5):1064-70; Kraus et al., 1997, Human Genetics 99:374-380, the contents of which are hereby incorporated by reference in their entirety.

In particular embodiments, the force is applied through a moving meniscus. Those of skill in the art will recognize that a moving meniscus can apply various forces to a nanoreporter including hydrodynamic force, surface tension and any other force recognized by those of skill in the art. The meniscus can be moved by any technique apparent to those of skill in the art including evaporation and gravity. Exemplary techniques for extending a nanoreporter with a moving meniscus are described in, for example, U.S. Pat. Nos. 6,548,255; 6,344,319; 6,303,296; 6,265,153; 6,225,055; 6,054,327; and 5,840,862, the contents of which are hereby incorporated by reference in their entireties.

In particular embodiments, the nanoreporter can be extended by an optical trap or optical tweezers. For instance, the nanoreporter can comprise or can be linked, covalently or noncovalently, to a particle capable of being trapped or moved by an appropriate source of optical force. Useful techniques for moving particles with optical traps or optical tweezers are described in Ashkin et al, 1986, Optics Letters 11:288-290; Ashkin et al., 1987, Science 235:1517-1520; Ashkin et al., Nature 330:769-771; Perkins et al., 1994, Science 264:822-826; Simmons et al., 1996, Biophysical Journal 70:1813-1822; Block et al., 1990, Nature 348:348-352; and Grier, 2003, Nature 424: 810-816; the contents of which are hereby incorporated by reference in their entireties.

In certain embodiments, the nanoreporter can be extended by combinations of the above forces that are apparent to those of skill in the art. In the examples, below, certain nanoreporters are extended by a combination of an electric field and hydrodynamic force.

The nanoreporter is extended when it would be recognized as extended by one of skill in the art according to standard criteria for extension of a nanoreporter. In certain embodiments, the nanoreporter is extended when it loses most of its tertiary structural features as recognized by those of skill in the art. In certain embodiments, the nanoreporter is extended when it loses most of its secondary structural features as recognized by those of skill in the art. In certain embodiments, the nanoreporter is extended when its primary structural features are detectable in sequence when imaged according to standard techniques. Exemplary imaging techniques are described in the examples below.

In certain embodiments, an extended state of a nanoreporter can be recognized by comparing its hydrodynamic radius to its average hydrodynamic radius when free in dilute solution. For instance, in certain embodiments, a nanoreporter, or portion thereof, is extended when its hydrodynamic radius is more than about double its average hydrodynamic radius in dilute solution. More quantitatively, R represents the hydrodynamic radius of the nanoreporter, or portion thereof, and <R> represents the average hydrodynamic radius of the nanoreporter, or portion thereof, in dilute solution. The average <R> should be calculated such that R for the nanoreporter, or portion thereof, when unbound in dilute solution is less than 2<R>95% of the time. In certain embodiments, a nanoreporter, or portion thereof, is in an extended state when R is greater than 1.5<R>, greater than 1.6<R>, greater than 1.7<R>, greater than 1.8<R>, greater than 1.9<R>, greater than 2.0<R>, greater than 2.1<R>, greater than 2.2<R>, greater than 2.3<R>, greater than 2.4<R>, greater than 2.5<R> or greater than 3.0<R>. In particular embodiments, a nanoreporter, or portion thereof, is in an extended state when R is greater than 2.0<R>.

Orientation of the Nanoreporter

In certain methods of the invention, the nanoreporter is in an oriented state. Generally, any nanoreporter is in an oriented state if it would be recognized as such by one of skill in the art.

In certain embodiments, the nanoreporter is in an oriented state when it is in the field of a force capable of orienting the nanoreporter under conditions suitable for orienting the nanoreporter. Such forces and conditions should be apparent to those of skill in the art.

In certain embodiments, the force is an electromagnetic force. For instance, when the nanoreporter is charged, such as a polynucleotide, the nanoreporter can be oriented in an electric or magnetic field. The field should be strong enough to orient the nanoreporter according to the judgment of one of skill in the art. Exemplary techniques for orienting a nanoreporter in an electric or magnetic field are described above.

In certain embodiments, the force is a hydrodynamic force. For instance, many nanoreporters, including polysaccharides, polypeptides, and polynucleotides, can be oriented in the field of a moving fluid. The hydrodynamic force should be strong enough to orient the nanoreporter according to the judgment of one of skill in the art. Exemplary techniques for orienting a nanoreporter in hydrodynamic field are described above.

In certain embodiments, the force is gravity. In advantageous embodiments, the force of gravity can be combined with, for example, hydrodynamic force to orient the nanoreporter. In certain embodiments, the force should be strong enough to orient the nanoreporter according to the judgment of one of skill in the art. Exemplary techniques for orienting a nanoreporter with gravity are described above.

In certain embodiments, the nanoreporter can be oriented by combinations of the above forces that are apparent to those of skill in the art. In the examples, below, certain nanoreporters are oriented by a combination of an electric field and hydrodynamic force.

The nanoreporter is oriented when it would be recognized as oriented by one of skill in the art according to standard criteria for orientation of a nanoreporter. In certain embodiments, the nanoreporter is oriented when it is arranged in parallel, as recognized by those of skill in the art, with the field of a force capable of orienting the nanoreporter. In certain embodiments, the nanoreporter is oriented when it is one of a plurality of nanoreporters that are arranged in parallel, as recognized by those of skill in the art. For instance, a plurality of nanoreporters can be oriented when the vector from a first terminus to a second terminus of a nanoreporter is parallel, as recognized by those of skill in the art, to the vectors between corresponding termini of other nanoreporters in the plurality.

Selective Immobilization of Second Portion of Nanoreporter

As discussed above, in the methods of the invention, a second portion of the tag-based nanoreporter is selectively immobilized. The second portion of the nanoreporter is the reporter probe hybridized to the target-specific reporter oligo. The reporter probe optionally comprises a moiety for immobilization.

In certain embodiments, the present invention provides methods that comprise the single step of selectively immobilizing a second portion of a nanoreporter (i.e., via the reporter probe) while the nanoreporter is in an extended or oriented state, and while a first portion (i.e. via the capture probe) of the nanoreporter is immobilized. Exemplary methods for immobilization of the first portion of the nanoreporter, and for extension or orientation of the nanoreporter are described in detail in the sections above.

In certain embodiments, the present invention provides methods that comprise the step of extending a nanoreporter, while a first portion of the nanoreporter is immobilized, and the step of selectively immobilizing a second portion of a nanoreporter while the nanoreporter is in an extended state. Exemplary methods for immobilization of the first portion of the nanoreporter, and for extension of the nanoreporter are described in detail in the sections above.

In certain embodiments, the present invention provides methods that comprise the step of immobilizing a first portion of a nanoreporter, the step of extending the nanoreporter while the first portion is immobilized and the step of selectively immobilizing a second portion of a nanoreporter while the nanoreporter is in an extended state. Exemplary methods for immobilization of the first portion of the nanoreporter, and for extension of the nanoreporter are described in detail above.

In certain embodiments, the present invention provides methods that comprise the step of orienting a nanoreporter, while a first portion of the nanoreporter is immobilized, and the step of selectively immobilizing a second portion of a nanoreporter while the nanoreporter is in an oriented state. Exemplary methods for immobilization of the first portion of the nanoreporter, and for orienting the nanoreporter are described in detail in the sections above.

In certain embodiments, the present invention provides methods that comprise the step of immobilizing a first portion of a nanoreporter, the step of orienting the nanoreporter while the first portion is immobilized and the step of selectively immobilizing a second portion of a nanoreporter while the nanoreporter is in an oriented state. Exemplary methods for immobilization of the first portion of the nanoreporter, and for orienting the nanoreporter are described in detail above.

The selective immobilization of the second portion of the nanoreporter can follow any technique for selective immobilization of a nanoreporter apparent to those of skill in the art. Significantly, in advantageous embodiments of the invention, the second portion of the nanoreporter is not immobilized non-selectively. Selective immobilization can allow the nanoreporter to be immobilized while in a fully extended state or nearly fully extended state. Selective immobilization can also allow the nanoreporter to be immobilized in an oriented manner. In other words, the first portion and second portion of the nanoreporter can be immobilized along the direction of the field or fields used to extend the nanoreporter, with the first portion preceding the second portion in the field. When a plurality of nanoreporters are immobilized, the can be uniformly oriented along the field.

As discussed above, the second portion of the nanoreporter, or the reporter probe hybridized to the target-specific reporter oligo, is immobilized selectively. The immobilization can be by any selective interaction with the substrate apparent to those of skill in the art. The immobilization can be via electrostatic or ionic interaction, via one or more covalent bonds, via one or more non-covalent bonds or combinations thereof. In certain embodiments, the immobilization can be via electrostatic interaction with an electrode. In further embodiments, the immobilization is via electrostatic interaction with a substrate other than the electrode.

If the first portion and the second portion of the nanoreporter are selectively immobilized to the same substrate, the techniques of selective immobilization should of course be compatible with the substrate. In particular embodiments, the techniques of immobilization are the same. For instance, on a substrate coated with avidin, both the first and second portion of the nanoreporter can be immobilized selectively via biotin-avidin interactions. However, as will be apparent to those of skill in the art, the same interaction need not be used at both the first and second portions for immobilization on the same substrate. For instance, the substrate can comprise multiple moieties capable of selective binding, or the first portion can be immobilized non-selectively, or other techniques apparent to those of skill in the art.

In certain embodiments, the second portion of the nanoreporter comprises a first member of a binding pair. The second member of the binding pair can be covalently bound to the second portion of the nanoreporter, or they can be non-covalently bound. Useful covalent bonds and non-covalent bonds will be apparent to those of skill in the art. In useful embodiments, the substrate onto which the second portion of the nanoreporter is bound will comprise a second member of the binding pair. The substrate can be covalently bound to the second member, or they can be non-covalently bound.

In certain embodiments, the second portion of the nanoreporter can comprise a member of a binding pair that is capable of binding with a member of a binding pair on the substrate to form one or more non-covalent bonds. Exemplary useful substrates include those that comprise a binding moiety selected from the group consisting of ligands, antigens, carbohydrates, nucleic acids, receptors, lectins, and antibodies such as those described in the sections above.

In advantageous embodiments, the second portion of the nanoreporter can be immobilized to the substrate via an avidin-biotin binding pair. In certain embodiments, the nanoreporter can comprise a biotin moiety in its first portion. For instance, a polynucleotide nanoreporter can comprise a biotinylated nucleotide residue. Similarly, a polypeptide nanoreporter can comprise a biotinylated amino acid residue. Useful substrates comprising avidin are described in the sections above.

In further embodiments, the second portion of the nanoreporter can comprise avidin, and the substrate can comprise biotin. Useful substrates comprising biotin are described in the sections above.

In further embodiments, the second portion of the nanoreporter can comprise a member of a binding pair that is capable of reacting with a member of a binding pair on the substrate to form one or more covalent bonds. Exemplary useful substrates comprising reactive groups are described in the sections above.

In certain embodiments, the second portion of the nanoreporter can comprise a reactive moiety that is capable of being bound to the substrate by photoactivation. The substrate could comprise the photoreactive moiety, or the second portion of the nanoreporter could comprise the photoreactive moiety. Some examples of photoreactive moieties include aryl azides, such as N-((2-pyridyldithio)ethyl)-4-azidosalicylamide; fluorinated aryl azides, such as 4-azido-2,3,5,6-tetrafluorobenzoic acid; benzophenone-based reagents, such as the succinimidyl ester of 4-benzoylbenzoic acid; and 5-Bromo-deoxyuridine.

In further embodiments, the second portion of the nanoreporter can be immobilized to the substrate via other binding pairs described in the sections above.

In further embodiments, the second portion of the nanoreporter is capable of forming a complex with one or more other molecules that, in turn, are capable of binding, covalently or non-covalently, a binding moiety of the substrate. For instance, the second portion of the nanoreporter can be capable of selectively binding another molecule that comprises, for instance, a biotin moiety that is capable of selectively binding, for instance, an avidin moiety of the substrate.

*Immobilization of Two Portions of an Extended or Oriented Nanoreporter*

In certain embodiments, the present invention provides methods for selective immobilization of a first portion and a second portion of a nanoreporter that is in an extended or oriented state. Significantly, according to these methods of the invention, the nanoreporter need not be immobilized prior to application of a force capable of extending or orienting the nanoreporter.

In these methods, the nanoreporter is extended or oriented, or both, by a force capable of extending or orienting the nanoreporter. Such forces are described in detail in the sections above. In particular embodiments, the force is a force capable of extending or orienting the nanoreporter while maintaining the nanoreporter in one location, i.e. a force capable of extending or orienting without substantially moving the nanoreporter. Exemplary forces include oscillating electromagnetic fields and oscillating hydrodynamic fields. In a particular embodiment, the force is an oscillating electrical field. Exemplary techniques for extending or orienting a nanoreporter in an oscillating electric field are described in Asbury et al., 2002, Electrophoresis 23(16):2658-66; Kabata et al., 1993, Science 262(5139):1561-3; and Asbury and van den Engh, 1998, Biophys J. 74:1024-30, the contents of which are hereby incorporated by reference in their entirety.

In the methods, the nanoreporter is immobilized at a first portion and at a second portion while extended or oriented. Both the first portion and the second portion can be immobilized non-selectively, both can be immobilized selectively, or one can be immobilized selectively and the other non-selectively. Techniques for immobilization of the first portion and second portion are described in detail in the sections above.

*Substrate for Immobilization*

In the methods of the invention, the substrate for immobilization can be any substrate capable of selectively binding the nanoreporter apparent to those of skill in the art. Further, in certain aspects, the present invention provides compositions comprising a selectively immobilized nanoreporter in an extended state. The compositions comprise a substrate, as described herein, having immobilized thereto a nanoreporter in an extended state. The nanoreporter can be, of course, immobilized according to a method of the invention.

The only requirement of the substrate is that it be capable of selectively binding the second portion of the nanoreporter as described above. Thus, the substrate can be a filter or a membrane, such as a nitrocellulose or nylon, glass, a polymer such as polyacrylamide, a gel such as agarose, dextran, cellulose, polystyrene, latex, or any other material known to those of skill in the art to which capture compounds can be immobilized. The substrate can be composed of a porous material such as acrylic, styrene methyl methacrylate copolymer and ethylene/acrylic acid.

The substrate can take on any form so long as the form does not prevent selective immobilization of the second portion of the nanoreporter. For instance, the substrate can have the form of a disk, slab, strip, bead, submicron particle, coated magnetic bead, gel pad, microtiter well, slide, membrane, frit or other form known to those of skill in the art. The substrate is optionally disposed within a housing, such as a chromatography column, spin column, syringe barrel, pipette, pipette tip, 96 or 384 well plate, microchannel, capillary, etc., that aids the flow of liquid over or through the substrate.

The nanoreporter can be immobilized on a single substrate or on a plurality of substrates. For instance, in certain embodiments, the first and second portions of nanoreporter are immobilized on the same substrate, as recognized by those of skill in the art. In certain embodiments, the first portion of the nanoreporter can be immobilized on a first substrate while the second portion of the nanoreporter can be immobilized on a second substrate, distinct from the first.

The substrate can be prepared according to any method apparent to those of skill in the art. For a review of the myriad techniques that can be used to activate exemplary substrates of the invention with a sufficient density of reactive groups, see, the Wiley Encyclopedia of Packaging Technology, 2d Ed., Brody & Marsh, Ed., "Surface Treatment," pp. 867 874, John Wiley & Sons (1997), and the references cited therein. Chemical methods suitable for generating amino groups on silicon oxide substrates are described in Atkinson & Smith, "Solid Phase Synthesis of Oligodeoxyribonucleotides by the Phosphite Triester Method," In: Oligonucleotide Synthesis: A Practical Approach, M J Gait, Ed., 1984, IRL Press, Oxford, particularly at pp. 45 49 (and the references cited therein); chemical methods suitable for generating hydroxyl groups on silicon oxide substrates are described in Pease et al., 1994, Proc. Natl. Acad. Sci. USA 91:5022 5026 (and the references cited therein); chemical methods for generating functional groups on polymers such as polystyrene, polyamides and grafted polystyrenes are described in Lloyd Williams et al., 1997, Chemical Approaches to the Synthesis of Peptides and Proteins, Chapter 2, CRC Press, Boca Raton, Fla. (and the references cited therein).

Exemplary useful substrates include surfaces coated with streptavidin, e.g. Accelr8 TB0200. Further useful substrates include surfaces coated with N-hydroxysuccinamide that are capable of reacting with a portion of a nanoreporter that comprises an amine. One such surface is OptArray-DNA (Accelr8). Additional useful surfaces are coated with aldehyde (e.g. Nexterion Slide AL, Schott) and surfaces coated with epoxy (e.g. Nexterion Slide E, Schott). Another useful surface is a biotinylated BSA coated surface useful for selective immobilization of a portion of a nanoreporter that comprises avidin or streptavidin.

*Methods of Using Selectively Immobilized, Extended or Oriented Nanoreporters*

In certain embodiments, the selectively immobilized, elongated nanoreporters can be used to create macromolecular barcodes for the purposes of separation and sequential detection of labels. These labels spaced along the molecule provide a unique code that can be read when the nanoreporter is extended and immobilized. Extension and selective immobilization can facilitate the decoding of the macromolecular barcode.

The selectively immobilized, elongated nanoreporters can further be used for can be used in any context where detection or imaging of a nanoreporter might be useful. They can be used for diagnostic, prognostic therapeutic and screening purposes. For instance, they can be applied to the analysis of biomolecular samples obtained or derived from a patient so as to determine whether a diseased cell type is present in the sample and/or to stage the disease. They can be used to diagnose pathogen infections, for example infections by intracellular bacteria and viruses, by determining the presence and/or quantity of markers of bacterium or virus, respectively, in the sample. The compositions and methods of the invention can be used to quantitate target molecules whose abundance is indicative of a biological state or disease condition, for example, blood markers that are upregulated or downregulated as a result of a disease state. In addition, the compositions and methods of the invention can be used to provide prognostic information that assists in determining a course of treatment for a patient.

Detection of Tag-Based Nanoreporters

The tag-based nanoreporters of the present invention are detected by any means available in the art that is capable of detecting the specific signals on a given nanoreporter. Where the nanoreporter is fluorescently labeled, suitable consideration of appropriate excitation sources may be investigated. Possible sources may include but are not limited to arc lamp, xenon lamp, lasers, light emitting diodes or some combination thereof. The appropriate excitation source is used in conjunction with an appropriate optical detection system, for example an inverted fluorescent microscope, an epi-fluorescent microscope or a confocal microscope. Preferably, a microscope is used that can allow for detection with enough spatial resolution to determine the sequence of the spots on the nanoreporter.

*Microscope and Objective Lens Selection*

The major consideration regarding the microscope objective lens is with the optical resolution, which is determined by its numerical aperture (NA). Generally, the larger the NA, the better the optical resolution. The required NA is preferably at least 1.07 based on the relationship of $\delta=0.61\lambda/NA$ ($\delta$=optical resolution and $\lambda$=wavelength). The amount of light that is collected by an objective is determined by $NA^4/Mag^2$ (Mag=magnification of the objective). Therefore, in order to collect as much light as possible, objectives with high NA and low magnifications should be selected.

*CCD Camera Selection and Image Capture Techniques.*

When selecting a CCD camera, the first consideration is the pixel size, which partially determines the final resolution of the imaging system. Optimally the optical resolution should not be compromised by the CCD camera. For example, if the optical resolution is 210-300 nm, which corresponds to 12.6-18 $\mu$m on a CCD chip after a 60x magnification, in order to resolve and maintain the optical resolution there should be at least two pixels to sample each spot. Or the pixel size of the CCD chip should be at most 6.3-9 $\mu$m.

The second consideration is detection sensitivity which can be determined by many factors that include but are not limited to pixel size, quantum efficiency, readout noise and dark noise. To achieve high sensitivity, select a qualitative camera with big pixel size (which can give big collection area), high quantum efficiency and low noise. An exemplary camera with these criteria is the Orca-Ag camera from Hamamatsu Inc. The chip size is 1344x1024 pixels; when using the 60x objective, the field of view is 144x110 $\mu m^2$.

Computer Systems

The invention provides computer systems that may be used to computerize nanoreporter image collection, nanoreporter identification and/or decoding of the nanoreporter code. Specifically, the invention provides various computer systems comprising a processor and a memory coupled to the processor and encoding one or more programs. The computer systems can be connected to the microscopes employed in imaging the nanoreporter, allowing imaging, identification and decoding the nanoreporter, as well as storing the nanoreporter image and associated information, by a single apparatus. The one or more programs encoded by the memory cause the processor to perform the methods of the invention.

In still other embodiments, the invention provides computer program products for use in conjunction with a computer system (e.g., one of the above-described computer systems of the invention) having a processor and a memory connected to the processor. The computer program products of the invention comprise a computer readable storage medium having a computer program mechanism encoded or embedded thereon. The computer program mechanism can be loaded into the memory of the computer and cause the processor to execute the steps of the methods of the invention.

The methods described in the previous subsections can preferably be implemented by use of the following computer systems, and according to the following methods. An exemplary computer system suitable for implementation of the methods of this invention comprises internal components and being linked to external components. The internal components of this computer system include a processor element interconnected with main memory. For example, the computer system can be an Intel Pentium-based processor of 200 MHz or greater clock rate and with 32 MB or more of main memory.

The external components include mass storage. This mass storage can be one or more hard disks which are typically packaged together with the processor and memory.

Such hard disks are typically of 1 GB or greater storage capacity. Other external components include user interface device, which can be a monitor and a keyboard, together with pointing device, which can be a "mouse", or other graphical input devices (not illustrated). Typically, the computer system is also linked to a network link, which can be part of an Ethernet link to other local computer systems, remote computer systems, or wide area communication networks, such as the Internet. This network link allows the computer system to share data and processing tasks with other computer systems.

Loaded into memory during operation of this system are several software components, which are both standard in the art and special to the instant invention. These software components collectively cause the computer system to function according to the methods of the invention. The software components are typically stored on mass storage. A first software component is an operating system, which is responsible for managing the computer system and its network interconnections. This operating system can be, for example, of the Microsoft Windows family, such as Windows 95, Windows 2000, or Windows XP, or, alternatively, a Macintosh operating system, a Linux operating system or a Unix operating system. A second software component may include common languages and functions conveniently present in the system to assist programs implementing the methods specific to this invention. Languages that can be used to program the analytic methods of the invention include, for example, C, C++, JAVA, and, less preferably, FORTRAN, PASCAL, and BASIC. Another software component of the present invention comprises the analytic methods of this invention as programmed in a procedural language or symbolic package.

In an exemplary implementation, to practice the methods of the present invention, a nanoreporter code (i.e., a correlation between the order and nature of spots on a nanoreporter and the identity of a target molecule to which such a nanoreporter binds) is first loaded in the computer system. Next the user causes execution of analysis software which performs the steps of determining the presence and, optionally, quantity of nanoreporters with a given nanoreporter code.

The analytical systems of the invention also include computer program products that contain one or more of the above-described software components such that the software components may be loaded into the memory of a computer system. Specifically, a computer program product of the invention includes a computer readable storage medium having one or more computer program mechanisms embedded or encoded thereon in a computer readable format. The computer program mechanisms encoded, e.g., one or more of the analytical software components described above which can be loaded into the memory of a computer system and cause the processor of the computer system to execute the analytical methods of the present invention.

The computer program mechanisms or mechanisms are preferably stored or encoded on a computer readable storage medium. Exemplary computer readable storage media are discussed above and include, but are not limited to: a hard drive, which may be, e.g., an external or an internal hard drive of a computer system of the invention, or a removable hard drive; a floppy disk; a CD-ROM; or a tape such as a DAT tape. Other computer readable storage media will also be apparent to those skilled in the art that can be used in the computer program mechanisms of the present invention

The present invention also provides databases useful for practicing the methods of the present invention. The databases may include reference nanoreporter codes for a large variety of target molecules. Preferably, such a database will be in an electronic form that can be loaded into a computer system. Such electronic forms include databases loaded into the main memory of a computer system used to implement the methods of this invention, or in the main memory of other computers linked by network connection, or embedded or encoded on mass storage media, or on removable storage media such as a CD-ROM or floppy disk.

Alternative systems and methods for implementing the methods of this invention are intended to be comprehended within the accompanying claims. In particular, the accompanying claims are intended to include the alternative program structures for implementing the methods of this invention that will be readily apparent to one of skill in the art.

Applications of Nanoreporter Technology

The compositions and methods of the invention can be used for diagnostic, prognostic therapeutic and screening purposes. The present invention provides the advantage that many different target molecules can be analyzed at one time from a single biomolecular sample using the methods of the invention. This allows, for example, for several diagnostic tests to be performed on one sample

*Diagnostic/Prognostic Methods*

The present methods can be applied to the analysis of biomolecular samples obtained or derived from a patient so as to determine whether a diseased cell type is present in the sample and/or to stage the disease.

For example, a blood sample can be assayed according to any of the methods described herein to determine the presence and/or quantity of markers of a cancerous cell type in the sample, thereby diagnosing or staging the cancer.

Alternatively, the methods described herein can be used to diagnose pathogen infections, for example infections by intracellular bacteria and viruses, by determining the presence and/or quantity of markers of bacterium or virus, respectively, in the sample.

Thus, the target molecules detected using the compositions and methods of the invention can be either patient markers (such as a cancer marker) or markers of infection with a foreign agent, such as bacterial or viral markers.

Because of the quantitative nature of nanoreporters, the compositions and methods of the invention can be used to quantitate target molecules whose abundance is indicative of a biological state or disease condition, for example, blood markers that are upregulated or downregulated as a result of a disease state.

In addition, the compositions and methods of the invention can be used to provide prognostic information that assists in determining a course of treatment for a patient. For example, the amount of a particular marker for a tumor can be accurately quantified from even a small sample from a patient. For certain diseases like breast cancer, overexpression of certain genes, such as Her2-neu, indicate a more aggressive course of treatment will be needed.

*Analysis of Pathology Samples*

RNA extracted from formaldehyde- or paraformaldehyde-fixed paraffin-embedded tissue samples is typically poor in quality (fragmented) and low in yield. This makes gene expression analysis of low-expressing genes in histology samples or archival pathology tissues extremely difficult and often completely infeasible. The nanoreporter technology can fill this unmet need by allowing the analysis of very small quantities of low-quality total RNA.

To use nanoreporter technology in such an application, total RNA can be extracted from formaldehyde- or paraformaldehyde-fixed paraffin-embedded tissue samples (or similar) using commercially available kits such as RecoverAll Total Nucleic Acid Isolation Kit (Ambion) following manufacturer's protocols. RNA in such samples is frequently degraded to small fragments (200 to 500 nucleotides in length), and many paraffin-embedded histology samples only yield tens of nanograms of total RNA. Small amounts (5 to 100 ng) of this fragmented total RNA can be used directly as target material in a nanoreporter hybridization following the assay conditions described herein.

*Screening Methods*

The methods of the present invention can be used, inter alia, for determining the effect of a perturbation, including chemical compounds, mutations, temperature changes, growth hormones, growth factors, disease, or a change in culture conditions, on various target molecules, thereby identifying target molecules whose presence, absence or levels are indicative of a particular biological states. In a preferred embodiment, the present invention is used to elucidate and discover components and pathways of disease states. For example, the comparison of quantities of target molecules present in a disease tissue with "normal" tissue allows the elucidation of important target molecules involved in the disease, thereby identifying targets for the discovery/screening of new drug candidates that can be used to treat disease.

Kits

The invention further provides kits comprising one or more components of the invention. The kits can contain pre-labeled reporter or capture probes, or unlabeled reporter or capture probes with one or more components for labeling the nanoreporters. The kits also contain probes that contain target-specific sequences and tag sequences that bind to the reporter or capture probes.

The kit can include other reagents as well, for example, buffers for performing hybridization reactions, linkers, restriction endonucleases, and DNA ligases.

The kit also will include instructions for using the components of the kit, and/or for making and/or using the labeled nanoreporters.

EXAMPLES

Example 1. Development of 35-base tag sequences

35-base tag sequences for the reporter probes and reporter oligos were developed from "alien" sequence created by the External RNA Control Consortium (ERCC) at The National Institute of Standards and Technology. Starting with ERCC sequence, stretches of 35 bases were selected based on the following criteria:

    **A.** Tm of 78-82°C

    **B.** No more than 3 G's or 3 C's in a row

    **C.** No more than 7 bases of homology in an inverted repeat

    **D.** No more than 9 bases of homology in a direct repeat

    **E.** G/C content of 30-70%

    **F.** BLAST alignments against non-redundant NCBI nucleic acid sequence database and all nanoreporter system components with an 11 nucleotide stringency cut-off for alignment and an overall identity or complementarity of no greater than 85% (to minimize cross-hybridization issues)

**G.** the absence of EcoRI, PstI and HindIII restriction endonuclease recognition sites

For cloning purposes the selected sequences were modified such that the final base of each tag was a G. Tags were then re-screened against the above criteria. Following cloning, cloned tags were sequenced, and any single-base changes were re-screened against the above criteria before being accepted as a final tag. All tags were tested functionally for interactions with selected capture probe and capture oligo tags.

Example 2. Development of 25-base tag sequences

25-base tag sequences for capture probes and capture oligos were developed in a similar manner to the 35-base tag sequences developed in Example 1, with the exception that the G/C content was increased to allow 80%, the final base was not changed to a G, and the tags were not screened for restriction sites. Instead of being cloned, the capture probe/capture oligo tags are synthesized directly as part of the capture probe molecule or capture oligo.

The tags were cloned into existing nanoreporter backbone plasmids using standard restriction endonuclease and ligation-based cloning techniques. Each tag was assigned to a unique backbone plasmid which is used to generate a unique barcode, so that there is a 1:1 correspondence between each barcode and its associated tag. In the tag-based system conventional nanoreporter synthesis protocols are used to generate a single-stranded DNA backbone which contains both the standard label attachment regions and an intrinsic, single-stranded tag sequence which serves as a hybridization probe, in the place of the ligated gene-specific probe oligo used in the standard nanoreporter system.

Example 3. Comparison of Negative Control Between Standard and Tag-based Nanoreporter Systems

In this example, the counts generated for an internal negative control were compared between the standard non-tag-based and tag-based nanoreporter systems. In the standard non-tag-based assay, a pool of 100 target-specific reporter probes was mixed with the appropriate target-specific capture probes. In the tag-based assay, a pool of 96 reporters with tag-based probes (e.g., reporter probe and reporter oligo) was mixed with the single universal capture probe necessary for the tag-based assay.

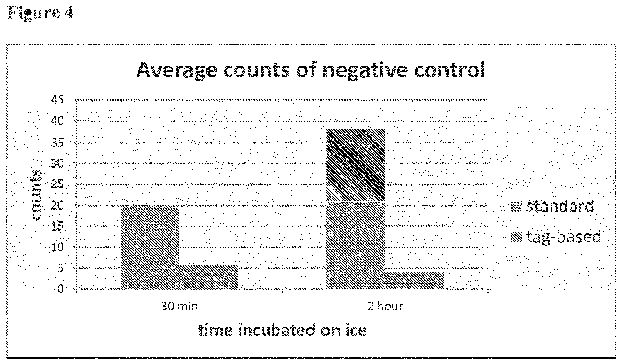

Mixes were incubated on ice for 30 minutes or for 2 hours. The pre-incubated mixes were subsequently used in hybridization reactions with the required additional assay components. The results in **Figure 4** show that the background signal generated during the assay set-up is significantly lower in the tag-based assay than the standard assay, and that it remains stable over time in the tag-based assay, while it increases with time in the standard assay.

**Example 4**. Tag-Based Assay Flexibility.

This example demonstrates the evaluation of one set of genes in a gene-expression assay, and based on this initial data, measuring some of the same genes as well as some different genes in a subsequent experiment. Using a non-tag-based nanoreporter system, a new gene-specific nanoreporter pool would be required for the subsequent experiment at a substantial cost, and with a typical manufacturing time of 4 weeks.

Utilizing the tag-based nanoreporter system of the present invention, however, it is possible to perform the subsequent experiment using the original pool of reporters mixed with a new set of gene-specific oligonucleotide probe pairs. The oligonucleotide probes are inexpensive, and can be quickly and easily obtained.

To demonstrate the flexibility of the system, a multiplex measurement of 216 targets was performed in two different experiments using identical target samples and reagents, with the exception of a pool of oligonucleotide probe pairs to 192 distinct genes that differed in each experiment. In both cases these oligonucleotide probes were attached to tags 1-192, allowing them to be detected by the same nanoreporter reagent. Oligonucleotide probes to 24 common genes were attached to tags 193-216 in both experiments, to mimic the situation described above, in which some targets are retained from one experiment to the next. These common genes also serve to demonstrate the reproducibility and robustness of the assay.

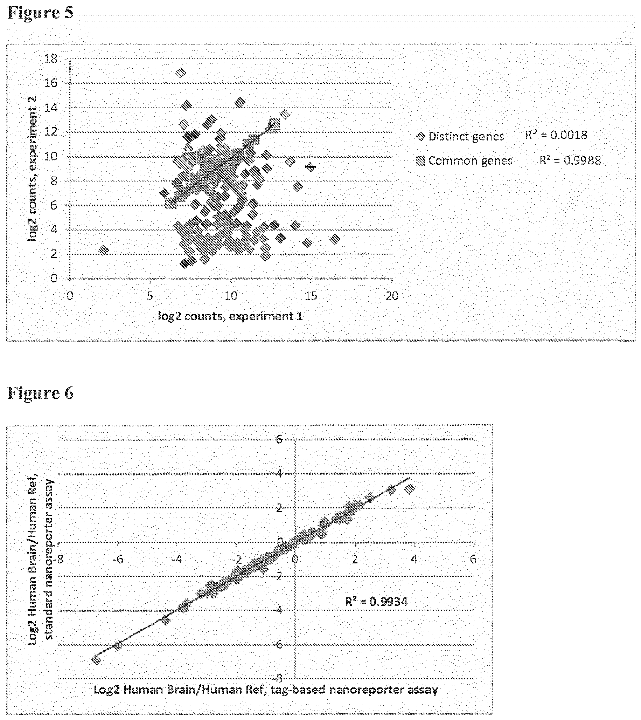

The scatter plot in **Figure 5** shows the gene expression results from two experiments using the same target sample and identical core reagents, including a common set of 24 gene-specific oligonucleotide probes (red squares). Each experiment also contains a distinct pool of an additional 192 gene-specific oligonucleotide probes (blue squares); in both experiments, these distinct probes are associated with tags 1-192. The high correlation of the common genes between the two experiments demonstrates the reproducibility of the assay, while the lack of correlation between the distinct genes demonstrates the novel data that is obtained in each experiment for a unique set of genes using only a new pool of oligonucleotides. Data is log2-transformed for visualization purposes.

The data in **Figure 5** demonstrates that the counts obtained for the common genes remain consistent in both experiments ($R^2$ of >0.99), while the counts associated with the other tags are completely independent in the two

experiments ($R^2$ of 0.0018), due to the fact that they are detecting different genes. The ease of generating distinctive data on two different sets of genes using the same set of core reagents is unique to the tag-based nanoreporter system.

**Example 5.** Comparison of gene expression data from standard and tag-based nanoreporter systems.

190 genes found in NanoString's Human Inflammation Panel were measured in two total RNA samples, Human Reference RNA and Human Brain RNA, using both the standard gene-specific nanoreporter assay and a tag-based nanoreporter assay with 190 gene-specific oligo probes to the same set of genes. For each gene, the fold-change in expression between the two sample types was calculated. The correlation ($R^2$ value) of the log fold-change values between standard and tag-based assays for all genes expressed above background was determined. All assays were performed in triplicate, and average values were used in the calculations.

**Figure 6** provides a correlation of measurements between standard and tag-based nanoreporter assays. The expression levels of 190 genes were measured with each assay type in two RNA samples, Human Reference and Human Brain. The fold-change of expression between the two samples was determined for all genes detected above background. A comparison of the data from the two assay types, shown in the scatter plot, yields an $R^2$ of >0.99, indicating that the measurements made by the tag-based nanoreporter assay are highly correlative with the measurements made by the standard nanoreporter assay.

**Example 6**. Reproducibility of technical replicates in the tag-based nanoreporter assay

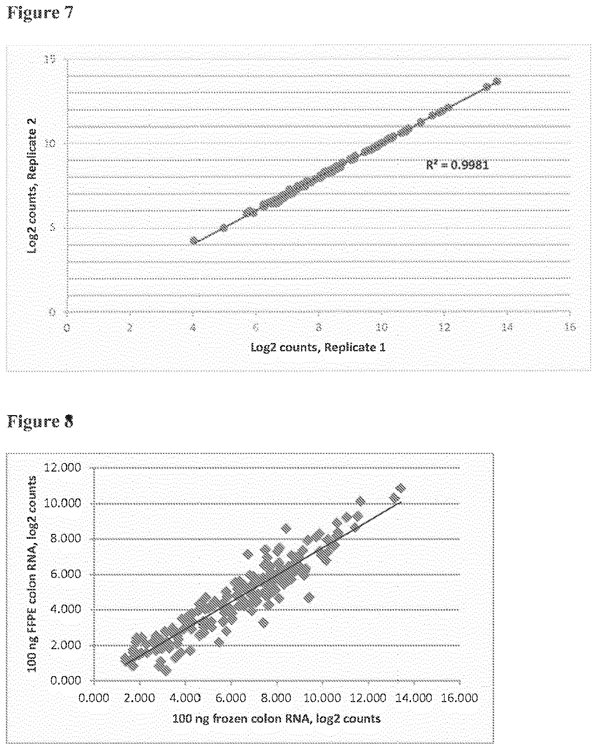

The tag-based nanoreporter system of the present invention provides extremely reproducible results in technical replicates. In this experiment, 96 gene-specific oligonucleotide probe pairs were combined with a 96-plex pool of tag-based reporters and a universal capture probe, and hybridized to 100 ng of Human Reference RNA in 12 separate assays. The assays were set up independently, and the resulting counts were normalized to internal positive controls. The average $R^2$ value of each replicate compared to Replicate 1 was >0.99. The comparison of Replicate 1 to Replicate 2 is shown in **Figure 7**. The data in **Figure 7** shows high correlation of technical replicates in this assay system.

**Example 7**. Correlation of measurements in different RNA sample types.

The tag-based nanoreporter system of the present invention was used to measure the correlations between counts obtained from different RNA preparation types of the same samples. All assays were run in triplicate with multiplexed probes to 192 genes, and the averaged, normalized counts as well as the log2-converted counts were analyzed.

For the formalin-fixed, paraffin-embedded (FFPE) tissue experiments, RNAs purified from human colon and human kidney FFPE samples were compared to RNAs purified from matched frozen tissue samples. For the lysate experiments, pellets of normal human dermal fibroblast cells (NHDF), and human umbilical vein endothelial cells (HUVEC) were lysed in RLT buffer (a guanidinium-based lysis buffer manufactured by Qiagen), and these lysates were used directly in the assay; RNAs purified from the same cell samples served as the controls.

**Table 3** shows a 90% correlation between the measurements obtained from frozen tissue and those obtained from FFPE tissue. RNA purified from FFPE tissue is highly degraded relative to RNA from frozen tissue; a 90% correlation is robust for these sample types, and is the same as what is seen when measuring these RNAs with other assay systems.

In addition, **Table 3** shows an almost perfect correlation between the data generated with whole cell lysates compared to those generated with purified RNA. The correlations in Table 2 indicate that the tag-based nanoreporter assay system works consistently on different types of RNA preparations.

**Table 3**

| Samples | $R^2$ value, normalized counts |
| --- | --- |
| FFPE colon vs. frozen colon | 0.90 |
| FFPE kidney vs. frozen kidney | 0.90 |
| NHDF lysate vs. purified RNA | 1.0 |
| HUVEC lysate vs. purified RNA | 1.0 |

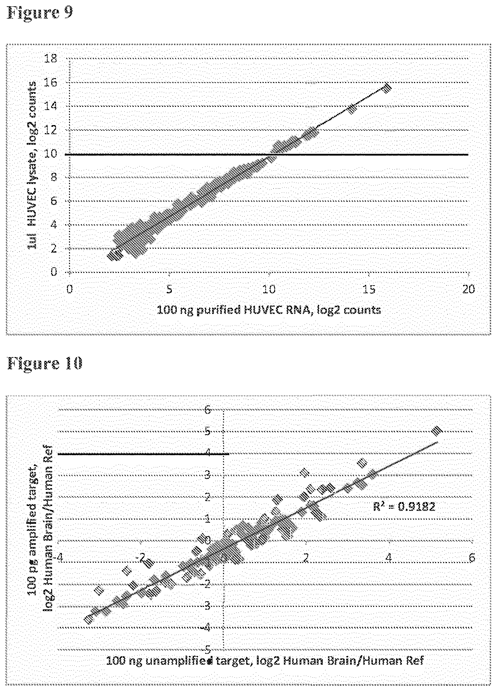

A more detailed representation of the data from one FFPE experiment and one lysate experiment can be found in **Figures 8 and 9**. **Figure 8** shows the comparison of gene expression data for 192 genes from RNA purified from a colon tissue sample which has either been frozen (x-axis) or formalin-fixed and paraffin-embedded (FFPE; y-axis), demonstrating good correlation of the results obtained by the tag-based nanoreporter assay on these two types of sample preparation. The data are shown as log2 counts for visualization purposes.

**Figure 9** shows the comparison of gene expression data for 192 genes from RNA purified from HUVEC cells (x-axis), or unpurified HUVEC cell lysate (y-axis), demonstrating good correlation of the results obtained by the tag-based nanoreporter assay on these two types of sample preparation. The data are shown as log2 counts for visualization purposes.

These results indicate that the tag-based nanoreporter system can be used to obtain consistent results on nucleic acid samples that have been prepared in different ways.

**Example 8.** Performance of tag-based assay in amplified samples.

The tag-based nanoreporter system was used to measure gene expression in low abundance samples that had been amplified by RT-PCR, thereby converting the RNA into DNA and amplifying the number of copies of each target. The fold-change in gene expression between a pair of RNA samples, Human Reference and Human Brain, which had been amplified from a starting amount of 100pg, was compared with the fold-change between 100ng of unamplified RNA from the same pair of samples. A primer pool containing primer pairs for 174 genes was used for the amplification. 96 of these genes were detected above background in all four samples (Human Reference and Human Brain, amplified and unamplified), and were used in the final fold-change analysis.

**Figure 10** shows the fold-change of expression between the two samples determined for the 96 genes detected above background. A comparison of the log2 data from the two assay types, shown in the scatter plot, yields an $R^2$ of >0.91, indicating that the measurements made by the tag-based assay are consistent between unamplified and amplified samples. Discrepancies in measurements between the samples are due to variability introduced by the amplification and not the tag-based nanoreporter hybridization assay itself, as unamplified replicate measurements typically have an $R^2$ value of >0.99 (see Example 6, Figure 7).

The data in **Figure 10** show a robust correlation between the tag-based nanoreporter measurements on unamplified RNA and on RNA that has been amplified by RT-PCR, indicating that this assay system can be used on amplified RNA samples.

**Example 9**. Performance of tag-based assay in determination of copy number variation (CNV) in genomic DNA.

The tag-based nanoreporter system was used to measure 96 genomic targets, including 27 genes (81 probes at 3 probes per gene), 10 invariant regions, 3 regions on the X chromosome and 2 regions on the Y chromosome. The assay was run with a sample input of 300 ng of genomic DNA from 1 diploid reference cell line, nine diploid cell lines, and two cell lines with trisomies for chromosome 13 and 18, respectively, as well as with genomic DNA purified from 12 normal (non-tumor) FFPE samples, presumed to be diploid for the selected genes. The sex of the samples was known, allowing inference of the correct copy number for X and Y-linked regions in each sample. The same samples were also assayed using the standard nanoreporter system.

Copy number for each probe was calculated relative to the diploid reference DNA for each experimental DNA sample. Percent accuracy was assessed relative to the known values for each region in each sample.

**Table 4** shows the percent accuracy of the copy number calls for 96 probes hybridized to genomic DNA from 12 tissue samples and 12 cell lines using both the tag-based nanoreporter assay and the standard assay. Both assay types give 100% accurate calls on the cell line DNAs, and an average of >95% accurate calls on the lower quality DNAs purified from FFPE tissue samples. The results indicate that the tag-based nanoreporter system is highly accurate for measuring copy number variation in genomic DNA from different sources, and the tag-based nanoreporter system has equivalent performance to the standard nanoreporter system for CNV assays.

**Table 4**

| Sample | Sample Type | Copy Number, tag-based assay % Accuracy | Copy Number, standard assay % Accuracy |
|---|---|---|---|
| VXV4ONCX | FFPE | 86% | 93% |
| QKIB ONJQ | FFPE | 100% | 100% |
| 96P VANYO | FFPE | 93% | 97% |
| NW9 2EEBJ | FFPE | 86% | 90% |
| VNVU1 NC6 | FFPE | 100% | 100% |
| E721 | FFPE | 97% | 97% |

(continued)

| Sample | Sample Type | Copy Number, tag-based assay % Accuracy | Copy Number, standard assay % Accuracy |
|---|---|---|---|
| WU2SZAKG | FFPE | 97% | 100% |
| B508310 | FFPE | 97% | 100% |
| ZBDNMN4L | FFPE | 100% | 100% |
| NIEV2 N91 | FFPE | 100% | 100% |
| B65M FNAX | FFPE | 100% | 100% |
| FPG4 NNT3 | FFPE | 93% | 90% |
| NA01359 | Cell Line | 100% | 100% |
| NA03330 | Cell Line | 100% | 100% |
| NA07055 | Cell Line | 100% | 100% |
| NA19003 | Cell Line | 100% | 100% |
| NA18488 | Cell Line | 100% | 100% |
| NA18517 | Cell Line | 100% | 100% |
| NA10851 | Cell Line | 100% | 100% |
| NA07022 | Cell Line | 100% | 100% |
| NA18862 | Cell Line | 100% | 100% |
| NA18524 | Cell Line | 100% | 100% |
| NA18521 | Cell Line | 1000% | 100% |
| NA10860 | Cell Line | 100% | 100% |
| **Avg. Copy Number Accuracy** | **FFPE** | **96%** | **97%** |
| | **Cell Line** | **100%** | **100%** |

The invention can be further defined with reference to the following embodiments or clauses:

1. A composition comprising a first probe and a second probe,

a. said first probe comprising

i. a first region that comprising a first target-specific sequence; and
ii. a second region that does not overlap with the first region and does not bind to the target molecule;

b. said second probe comprising

i. a first region that binds to the second region of said first probe;
ii. a first label attachment region which is hybridized to a first RNA molecule, wherein the first RNA molecule is attached to one or more label monomers that emit light constituting a first signal; and
iii. a second label attachment region, which is non-overlapping to the first label attachment region, and which is hybridized to a second RNA molecule, wherein the second RNA molecule is attached to one or more label monomers that emit light constituting a second signal, and wherein the label attachment regions do not overlap with the first region of the second probe.

2. A composition comprising a first probe and a second probe,

a. said first probe comprising

i. a first region comprising a first target-specific sequence; and
ii. a second region that does not overlap with the first region and does not bind to the target molecule;

b. said second probe comprising

i. a first region that binds to the second region of said first probe; and
ii. a second region that does not overlap with the first region and comprises at least one affinity moiety.

3. The composition of 2, wherein the second probe further comprises at least a first label attachment region which is hybridized to a first RNA molecule, wherein the first RNA molecule is attached to one or more label monomers that emit light constituting a first signal.

4. The composition of clause 1 or 2, wherein the second region of the first probe or the first region of the second probe comprises any one of SEQ ID NOs 1-1345 or a complement thereof.

5. The composition of clause 1 or 2, wherein a plurality of first RNA molecules are hybridized to the first label attachment region, wherein the first RNA molecules are attached to said one or more label monomers the emit light constituting said first signal; and wherein a plurality of second RNA molecules are hybridized to the second label attachment region, wherein the second RNA molecules are attached to one or more label monomers that emit light constituting a second signal.

6. The composition of clause 1 or 2, wherein the first signal and the second signal are spatially or spectrally distinguishable.

7. The composition of clause 1 or 2, wherein the first and second label attachment regions are predetermined nucleotide sequences.

8. The composition of clause 1 or 2, wherein the first and second probes are nucleic acid molecules.

9. A composition pair comprising a first composition and a second composition, wherein the first composition comprises a first probe and a second probe,

a. said first probe comprising

i. a first region comprising a first target-specific sequence; and
ii. a second region that does not overlap with the first region and does not bind to the target molecule;

b. said second probe comprising

i. a first region that binds to the second region of said first probe; and
ii. a second region comprising at least one affinity moiety, wherein the first region does not overlap with the second region;

and wherein the second composition comprises a third probe and a fourth probe,
c. said third probe comprising

i. a first region comprising a second target-specific sequence; and
ii. a second region that does not bind to the target molecule, wherein the first region and the second region do not overlap;

d. said fourth probe comprising

i. a first region that binds to the second region of said third probe;
ii. a first label attachment region which is hybridized to a first RNA molecule, wherein the first RNA molecule is attached to one or more label monomers that emit light constituting a first signal; and
iii. a second label attachment region, which is non-overlapping to the first label attachment region, and which is hybridized to a second RNA molecule, wherein the second RNA molecule is attached to one or more label monomers that emit light constituting a second signal,

wherein the first target-specific sequence and the second target-specific sequence bind to different regions of the same target molecule, and

wherein the first and second probes of the first composition cannot bind to the third or fourth probe of the second composition, and

wherein when said composition pair is bound to its target molecule, the identity of the first and second signals and their locations relative to each other constitute at least part of a code that identifies the target molecule.

10. The composition of 9, wherein the second probe further comprises at least a first label attachment region which is hybridized to a first RNA molecule, wherein the first RNA molecule is attached to one or more label monomers that emit light constituting a first signal.

11. The composition of clause 9, wherein the second region of the first probe or the first region of the second probe comprises any one of SEQ ID NOs: 1-1345, or a complement thereof; where the second region of the third probe or the first region of the fourth probe comprises any one of SEQ ID NOs: 1-1345, or a complement thereof; and wherein the second region of the first probe and the second region of the third probe are not the same sequence.

12. The composition pair of clause 9, wherein a plurality of first RNA molecules are hybridized to the first label attachment region, wherein the first RNA molecules are attached to said one or more label monomers the emit light constituting said first signal; and wherein a plurality of second RNA molecules are hybridized to the second label attachment region, wherein the second RNA molecules are attached to one or more label monomers that emit light constituting a second signal.

13. The composition pair of clause 9, wherein the first signal and the second signal are spatially or spectrally distinguishable.

14. The composition pair of clause 9, wherein the first and second label attachment regions are predetermined nucleotide sequences.

15. The composition pair of clause 9, wherein when the composition is bound to its target molecule, the code comprises the identity of the first and second signals and their locations relative to each other.

16. The composition pair of clause 9, wherein the code comprises the identity of the first and second signals, and the size of the spot resulting from at least one of said signals.

17. The composition pair of clause 9, wherein the first, second, third and fourth probes are nucleic acid molecules.

18. A composition comprising:

a. a first region comprising a target-specific sequence; and
b. a second non-overlapping region comprising any one of SEQ ID NOs: 1-1345, or a complement thereof.

19. A method of detecting a target molecule in a biomolecular sample comprising:

a. contacting said sample with the composition pair according to clause 9 under conditions that allow (i) binding of the first target-specific sequence and the second target-specific sequence to the target molecule, (ii) binding of the first probe to the second probe; and (iii) binding of the third probe to the fourth probe; and
b. detecting the code that identifies the target molecule.

20. The method of clause 19, further comprising quantitating the amount of said target molecule in said biomolecular sample.

21. A method of detecting a plurality of target molecules in a biomolecular sample comprising:

a. contacting said sample with a population of composition pairs according to clause 9 under conditions that allow (i) binding of the first target-specific sequence and the second target-specific sequence of each composition to their respective target molecule, wherein each composition in said population when bound to its respective target molecule is associated with a distinguishable code; (ii) binding of the first probe to the second probe; and (iii) binding of the third probe to the fourth probe; and

b. detecting the codes that identify the plurality of target molecules.

22. The method of clause 21, further comprising quantitating the amount of each of said plurality of target molecules in said biomolecular sample.

23. The method of clause 22, wherein the fourth probe is different for each target molecule in said biomolecular sample.

24. The method of clause 22, wherein the second probe is the same for all target molecules in said biomolecular sample.

25. A method of manufacturing the second probe of clause 1, comprising introducing the sequence of the first region adjacent to the sequence of the second region in an expression plasmid, and transcribing the first and second region to produce the second probe.

26. A method of manufacturing the second probe of clause 2, comprising introducing the sequence of the first region adjacent to the sequence of the second region in an expression plasmid, and transcribing the first and second region to produce the second probe.

27. An expression plasmid of clause 24 or 25, wherein the sequence of the first region comprises any one of SEQ ID NOs: 1-1345, or a complement thereof.

**Claims**

1. A method of detecting a plurality of target molecules in a sample comprising:

   contacting the sample with a composition pair comprising a first composition and a second composition, wherein the first composition comprises a first probe and a second probe,

   a) wherein the first probe comprises;

      i) a first region comprising a first target molecule-specific sequence; and
      ii) a second region that does not overlap with the first region and does not bind to the target molecule;

   b) wherein the second probe comprises;

      i) a first region that binds to the second region of the first probe; and
      ii) a second region comprising at least one affinity moiety, wherein the first region does not overlap with the second region; and wherein the second composition comprises a third probe and a fourth probe,

   c) wherein the third probe comprises

      i) a first region comprising a second target molecule-specific sequence; and
      ii) a second region that does not bind to the target molecule, wherein the first region and the second region do not overlap;

   d) wherein the fourth probe comprises

      i) a first region that binds to the second region of the third probe;
      ii) a first label attachment region which is hybridized to at least one first RNA molecule, wherein each first RNA molecule is attached to one or more label monomers that emit light constituting a first signal; and
      iii) a second label attachment region which is non-overlapping to the first label attachment region, and which is hybridized to at least one second RNA molecule, wherein each second RNA molecule is attached to one or more label monomers that emit light constituting a second signal,

   wherein the first signal and the second signal are spatially or spectrally distinguishable, wherein the first target molecule-specific sequence and the second target molecule-specific sequence bind to different regions of the same target molecule,

wherein the first and second probes of the first composition cannot bind to the third or fourth probe of the second composition,
wherein the fourth probe binds to a single third probe; and
wherein when said composition pair is bound to its target molecule, the identity of the first and second signal and their locations relative to each other constitute at least part of a code that identifies the target molecule.

2. The method of claim 1, wherein the target molecule is obtained from a biomolecular sample.

3. The method of claim 1, wherein the target molecule is a nucleic acid.

4. The method of claim 1, wherein the sample is in an aqueous solution.

5. The method of claim 1, wherein the second region of the first probe or the first region of the second probe comprises any one of SEQ ID NOs: 1-1345, or a complement thereof; where the second region of the third probe or the first region of the fourth probe comprises any one of SEQ ID NOs: 1-1345, or a complement thereof; and wherein the second region of the first probe and the second region of the third probe are not the same sequence.

6. The method of claim 1, wherein a plurality of first RNA molecules are hybridized to the first label attachment region, wherein the first RNA molecules are attached to said one or more label monomers the emit light constituting said first signal; and wherein a plurality of second RNA molecules are hybridized to the second label attachment region, wherein the second RNA molecules are attached to one or more label monomers that emit light constituting a second signal.

7. The method of claim 1, wherein the first signal and the second signal are spatially or spectrally distinguishable.

8. The method of claim 1, wherein the first and second label attachment regions are predetermined nucleotide sequences.

9. The method of claim 1, wherein when the composition is bound to its target molecule, the code comprises the identity of the first and second signals and their locations relative to each other.

10. The method of claim 1, wherein the code comprises the identity of the first and second signals, and the size of the spot resulting from at least one of said signals.

11. The method of claim 1, wherein the probes are nucleic acid molecules.

12. The method of claim 1, further comprising quantitating the amount of said target molecule in said biomolecular sample.

13. The method of claim 1, wherein the fourth probe is different for each target molecule in said biomolecular sample.

14. The method of claim 1, wherein the second probe is the same for all target molecules in said biomolecular sample.

Figure 1

Gene-specific Reporter
Probe

Gene-specific Capture
Probe

B B B

Nucleic acid
target

Figure 2

Reporter Probe      Capture Probe

tag      tag      B B B

Oligo A      Oligo B

Nucleic acid
target

Figure 3

Reporter Probe            Labeled Capture Probe

tag                  tag

B B B

Oligo A                    Oligo B

Nucleic acid
target

**Figure 4**

Average counts of negative control

**Figure 5**

**Figure 6**

Figure 7

Figure 8

Figure 9

Figure 10

Europäisches Patentamt
European Patent Office
Office européen des brevets

# EUROPEAN SEARCH REPORT

**Application Number**

EP 18 17 6103

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2007/076128 A2 (NANOSTRING TECHNOLOGIES INC [US]; INST SYSTEMS BIOLOGY [US]; GEISS GAR) 5 July 2007 (2007-07-05) * claims 1,2, 5, 42, 45 * | 1-14 | INV. C12Q1/68 C12Q1/6876 C12P19/34 C12Q1/6816 |
| Y | WO 2012/054795 A1 (ADVANCED CELL DIAGNOSTICS INC [US]; WU XINGYONG [US]; WANG HUEI-YU FAY) 26 April 2012 (2012-04-26) * claim 1; Fig. 1; par. 72 * | 1-14 | |
| Y | WO 2007/001986 A2 (LUO YULING [US]; CHEN SHIPING [US]) 4 January 2007 (2007-01-04) * Fig. 2 and 3; par. 200 and 201 * | 1-14 | |

**TECHNICAL FIELDS SEARCHED (IPC)**

C12Q

The present search report has been drawn up for all claims

| Place of search | Date of completion of the search | Examiner |
|---|---|---|
| Munich | 11 October 2018 | Leber, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 17 6103

This annex lists the patent family members relating to the patent documents cited in the above-mentioned European search report.
The members are as contained in the European Patent Office EDP file on
The European Patent Office is in no way liable for these particulars which are merely given for the purpose of information.

11-10-2018

| Patent document cited in search report | Publication date | Patent family member(s) | | Publication date |
|---|---|---|---|---|
| WO 2007076128 A2 | 05-07-2007 | AT | 525482 T | 15-10-2011 |
| | | AU | 2006330830 A1 | 05-07-2007 |
| | | CA | 2640385 A1 | 05-07-2007 |
| | | EP | 1963531 A2 | 03-09-2008 |
| | | ES | 2374788 T3 | 22-02-2012 |
| | | JP | 5537034 B2 | 02-07-2014 |
| | | JP | 2009521230 A | 04-06-2009 |
| | | US | 2010015607 A1 | 21-01-2010 |
| | | US | 2013230851 A1 | 05-09-2013 |
| | | US | 2016265025 A1 | 15-09-2016 |
| | | US | 2018135099 A1 | 17-05-2018 |
| | | WO | 2007076128 A2 | 05-07-2007 |
| | | WO | 2007076129 A2 | 05-07-2007 |
| WO 2012054795 A1 | 26-04-2012 | CN | 103429755 A | 04-12-2013 |
| | | CN | 104849472 A | 19-08-2015 |
| | | CN | 107365847 A | 21-11-2017 |
| | | EP | 2630260 A1 | 28-08-2013 |
| | | EP | 3034625 A1 | 22-06-2016 |
| | | ES | 2562821 T3 | 08-03-2016 |
| | | ES | 2648564 T3 | 04-01-2018 |
| | | US | 2012100540 A1 | 26-04-2012 |
| | | US | 2014249040 A1 | 04-09-2014 |
| | | US | 2016201117 A1 | 14-07-2016 |
| | | WO | 2012054795 A1 | 26-04-2012 |
| WO 2007001986 A2 | 04-01-2007 | CA | 2612577 A1 | 04-01-2007 |
| | | CN | 101495650 A | 29-07-2009 |
| | | CN | 104673903 A | 03-06-2015 |
| | | CN | 104673905 A | 03-06-2015 |
| | | DK | 1910572 T3 | 14-03-2016 |
| | | DK | 2500439 T3 | 10-11-2014 |
| | | EP | 1910572 A2 | 16-04-2008 |
| | | EP | 1913158 A2 | 23-04-2008 |
| | | EP | 2460893 A1 | 06-06-2012 |
| | | EP | 2500439 A1 | 19-09-2012 |
| | | EP | 2711434 A1 | 26-03-2014 |
| | | EP | 3042963 A1 | 13-07-2016 |
| | | ES | 2434915 T3 | 18-12-2013 |
| | | ES | 2511218 T3 | 22-10-2014 |
| | | ES | 2565670 T3 | 06-04-2016 |
| | | US | 2007015188 A1 | 18-01-2007 |
| | | US | 2008038725 A1 | 14-02-2008 |
| | | US | 2011059442 A1 | 10-03-2011 |
| | | US | 2011059866 A1 | 10-03-2011 |
| | | US | 2016186245 A1 | 30-06-2016 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 18 17 6103

11-10-2018

| Patent document cited in search report | Publication date | Patent family member(s) | | Publication date |
|---|---|---|---|---|
| | | WO | 2007001986 A2 | 04-01-2007 |
| | | WO | 2007002006 A2 | 04-01-2007 |

EPO FORM P0459

For more details about this annex : see Official Journal of the European Patent Office, No. 12/82

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61834926 A
- US 20100015607 A
- US 20100047924 A
- US 5293050 A
- US 5354707 A, Chapple Sokol
- US 5496934 A
- US 5202247 A
- US 5137819 A

- US 6548255 B
- US 6344319 B
- US 6303296 B
- US 6265153 B
- US 6225055 B
- US 6054327 A
- US 5840862 A

**Non-patent literature cited in the description**

- **ZUKER.** *Nucleic Acids Res.,* 2003, vol. 31 (13), 3406-15
- **MATHEWS et al.** *J. Mol. Biol.,* 1999, vol. 288, 911-940
- **HYRUP et al.** *Bioorganic & Medicinal Chemistry,* 1996, vol. 4 (1), 5-23
- **PERRY-O'KEEFE et al.** *Proc. Natl. Acad. Sci. USA,* 1996, vol. 93, 14670-675
- Metal-chelate affinity chromatography. **PETTY et al.** Current Protocols in Molecular Biology. Greene Publish. Assoc. & Wiley Interscience, 1996, vol. 2
- **SMITH.** *Methods Mol. Cell. Bio.,* 1993, vol. 4, 220-229
- **GUAN et al.** *Gene,* 1987, vol. 67, 21-30
- **TOMME et al.** *Protein Eng.,* 1994, vol. 7, 117-123
- **HENEGARIU et al.** *Biotechniques,* 2001, vol. 31, 246-250
- **YOKOTA et al.** *Nuc. Acids Res.,* 1997, vol. 25, 1064-1070
- **MATSUURA et al.** *Nuc. Acids Res.,* 2001, vol. 29, E79
- **MATSUURA et al.** *J Biomol Struct Dyn.,* 2002, vol. 20 (3), 429-36
- **FERREE ; BLANCH.** *Biophys J.,* 2003, vol. 85 (4), 2539-46
- **STIGTER ; BUSTAMANTE.** *Biophys J. 1998,* 1998, vol. 75 (3), 1197-210
- **MATSUURA et al.** *Nucleic Acids Res.,* 2001, vol. 29 (16
- **FERREE ; BLANCH.** *Biophys J.,* 2004, vol. 87 (1), 468-75
- **BENSIMON et al.** *Science,* 1994, vol. 265, 2096-2098
- **HENEGARIU et al.** *BioTechniques,* 2001, vol. 31, 246-250

- **KRAUS et al.** *Human Genetics,* 1997, vol. 99, 374-380
- **MICHALET et al.** *Science,* 1997, vol. 277, 1518-1523
- **YOKOTA et al.** *Nucleic Acids Res.,* 1997, vol. 25 (5), 1064-70
- **OTOBE et al.** *Nucleic Acids Research,* 2001, vol. 29, 109
- **ZIMMERMAN ; COX.** *Nucleic Acids Res.,* 1994, vol. 22 (3), 492-7
- **ASHKIN et al.** *Optics Letters,* 1986, vol. 11, 288-290
- **ASHKIN et al.** *Science,* 1987, vol. 235, 1517-1520
- **ASHKIN et al.** *Nature,* vol. 330, 769-771
- **PERKINS et al.** *Science,* 1994, vol. 264, 822-826
- **SIMMONS et al.** *Biophysical Journal,* 1996, vol. 70, 1813-1822
- **BLOCK et al.** *Nature,* 1990, vol. 348, 348-352
- **GRIER.** *Nature,* 2003, vol. 424, 810-816
- **ASBURY et al.** *Electrophoresis,* 2002, vol. 23 (16), 2658-66
- **KABATA et al.** *Science,* 1993, vol. 262 (5139), 1561-3
- **ASBURY ; VAN DEN ENGH.** *Biophys J.,* 1998, vol. 74, 1024-30
- Surface Treatment. Wiley Encyclopedia of Packaging Technology. John Wiley & Sons, 1997, 867-874
- Solid Phase Synthesis of Oligodeoxyribonucleotides by the Phosphite Triester Method. **ATKINSON ; SMITH.** Oligonucleotide Synthesis: A Practical Approach. IRL Press, 1984, 45-49
- **PEASE et al.** *Proc. Natl. Acad. Sci. USA,* 1994, vol. 91, 5022-5026

- **LLOYD WILLIAMS et al.** Chemical Approaches to the Synthesis of Peptides and Proteins. CRC Press, 1997